# EUROPÄISCHE PATENTANMELDUNG

(11) **EP 3 889 835 A2**
(43) Veröffentlichungstag der Anmeldung: **06.10.2021**
(21) Anmeldenummer: 21166686.2
(22) Anmeldetag: 01.04.2021
(51) Int. Cl.: G06K 9/00

(54) **AUTHENTIFIZIERUNGSSYSTEM AUF BASIS VON FREMDBILDERN UND BEKANNTBILDERN UND ENTSPRECHENDEN HIRNAKTIVITÄTSSIGNALEN**

(30) Priorität: 02.04.2020 DE 102020109285
(71) Anmelder: Bundesdruckerei GmbH, 10969 Berlin (DE)
(72) Erfinder: FISCHER, Jörg, 10317 Berlin (DE); GRAUPNER, Hendrik, 14050 Berlin (DE); Dr. PAESCHKE, Manfred, 16348 Wandlitz (DE); TIETKE, Markus, 12439 Berlin (DE); TRÖLENBERG, Stefan, 15749 Mittenwalde, OT Ragow (DE)
(74) Vertreter: Richardt Patentanwälte PartG mbB

(57) **Zusammenfassung**

Die Erfindung betrifft ein computerimplementiertes Verfahren zur Authentifizierung einer Person (224). Das Verfahren umfasst:
- Empfang (102) von Daten (223) der Person;
- Auswertung (104) der Daten, um die Person mittels eines ersten Authentifizierungsverfahrens zu authentifizieren;
- Ermittlung (104) von Bekanntbildern (504) und Fremdbildern (208, 212, 210);
- Anzeige (108) zumindest eines Bekanntbilds und zumindest eines Fremdbilds;
- während die mehreren Bilder der Person angezeigt werden, Erfassung (110) von ersten (708, 712, 902) und zweiten (704, 706, 710, 904) Hirnaktivitätssignalen, die in der einen Person durch die Betrachtung der Bilder jeweils ausgelöst werden, durch einen kontaktlosen Sensor (218) zur Sensierung von Hirnaktivitäten - im Folgenden als KLHA-Sensor (218) bezeichnet, wobei erste Hirnaktivitätssignale erfasst werden, während der Person ein Bekanntbild (304, 404, 504) angezeigt wird, und wobei zweite Hirnaktivitätssignale erfasst werden, während der Person ein Fremdbild angezeigt wird;
- Durchführung (112) eines zweiten Authentifizierungsverfahrens durch die Authentifizierungssoftware, wobei das zweite Authentifizierungsverfahren umfasst eine Analyse der erfassten ersten und zweiten Hirnaktivitätssignale;
- Behandlung (114) der Person durch die Authentifizierungssoftware als authentifiziert nur dann, wenn diese sich sowohl im ersten als auch im zweiten Authentifizierungsverfahren erfolgreich authentifiziert hat.

## Beschreibung

### Gebiet

Die Erfindung betrifft ein Verfahren zur Authentifizierung einer Person.

### Hintergrund

Im Stand der Technik sind verschiedene Authentifizierungsmethoden basieren auf verschiedenen Daten eines Nutzers bekannt. Insbesondere biometrische Authentifizierungsverfahren wie z.B. Gesichtserkennungsverfahren kommen zunehmend zum Einsatz, z.B. an Grenzkontrollen, an den Eingangstoren von Firmengeländen und vielen anderen Anwendungsszenarien. Gesichtserkennungsverfahren und verwandte biometrische Verfahren haben den Vorteil, dass sie sehr schnell, oftmals rein optisch und damit kontaktfrei und dabei recht zuverlässig sind. Diese Verfahren sind somit insb. für die Authentifizierung einer großen Anzahl an Personen in kurzer Zeit geeignet.

Allerdings geht die zunehmende Verbreitung automatischer Gesichtserkennungsverfahren und verwandter Authentifizierungsverfahren einher mit einer zunehmenden Verfügbarkeit geeigneter Umgehungs- und Täuschungsverfahren. Beispielsweise besteht ein technischer Ansatz, ein auf Gesichtserkennung-basierendes Authentifizierungssystem zu täuschen darin, eine Gesichtsmaske z.B. auf Silikonbasis anfertigen zu lassen, die dem Gesicht einer "vertrauenswürdigen" Person (z.B. dem Gesicht eines Mitarbeiters einer Firma, die mit Gesichtserkennung ihr Firmengelände vor unberechtigtem Zutritt schützt) täuschend ähnlich sieht. Sowohl automatische Gesichtserkennungsverfahren als auch menschliches Wachpersonal lassen sich durch solche Masken täuschen.

Authentifizierungsverfahren, die darauf beruhen, dass eine Person sich durch einen Ausweis mit integriertem Gesichtsbild authentisiert, können dadurch unterwandert werden, dass beim Antrag auf Ausstellung eines Ausweises ein gemorphtes Bild bereitgestellt wird, das eine mittels einer Grafiksoftware errechneten Kombination eines "echten" Gesichtsbilds dieser Person und einer anderen Person, die dieser zumindest etwas ähnlich sieht, darstellt. Ein solcher Ausweis mit "gemorphtem" Bild kann ggf. nicht nur der Person, für die er ausgestellt wurde, sondern zusätzlich auch der anderen, geringfügig ähnlichen Person, Zutritt zu einem geschützten Bereich verschaffen.

### Zusammenfassung

Der Erfindung liegt die Aufgabe zugrunde, ein verbessertes Authentifizierungsverfahren vorzuschlagen, sowie ein entsprechendes Authentifizierungssystem.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung ein computerimplementiertes Verfahren zur Authentifizierung einer Person als Vertrauensperson. Das Verfahren umfasst:
- Empfang von Daten der Person durch eine Authentifizierungssoftware;
- Auswertung der empfangenen Daten durch die Authentifizierungssoftware, um die Person als die Vertrauensperson mittels eines ersten Authentifizierungsverfahrens zu authentifizieren;
- Ermittlung von einem oder mehreren Bekanntbildern und einem oder mehreren Fremdbildern durch die Authentifizierungssoftware, wobei ein Bekanntbild ein Bild ist mit einem Bildmotiv, das der Person bekannt ist, sofern es sich bei der Person um die Vertrauensperson handelt, wobei ein Fremdbild ein Bild ist mit einem Bildmotiv, das der Person nicht bekannt ist oder signifikant weniger bekannt ist als ein Bekanntbild;
- Anzeige mehrerer Bilder auf einer Anzeige, wobei die angezeigten Bilder zumindest eines ermittelten Bekanntbilder und zumindest eines der ermittelten Fremdbilder enthalten;
- während die mehreren Bilder der Person angezeigt werden, Erfassung von ersten und zweiten Hirnaktivitätssignalen, die in der einen Person durch die Betrachtung der Bilder jeweils ausgelöst werden, durch einen kontaktlosen Sensor zur Erfassung von Hirnaktivitäten - im Folgenden als KLHA-Sensor bezeichnet, wobei erste Hirnaktivitätssignale erfasst werden, während der Person ein Bekanntbild angezeigt wird, und wobei zweite Hirnaktivitätssignale erfasst werden, während der Person ein Fremdbild angezeigt wird;
- Durchführung eines zweiten Authentifizierungsverfahrens durch die Authentifizierungssoftware, um die Person als die Vertrauensperson zu authentifizieren, wobei das zweite Authentifizierungsverfahren umfasst eine Analyse der erfassten ersten und zweiten Hirnaktivitätssignale;
- Behandlung der Person durch die Authentifizierungssoftware als authentifiziert nur dann, wenn diese sich sowohl im ersten als auch im zweiten Authentifizierungsverfahren erfolgreich authentifiziert hat.

Bei der Vertrauensperson kann es sich z.B. um eine bestimmte Person handeln, die sich bei der Authentifizierungssoftware vorab registriert hat und die als vertrauenswürdig gilt zumindest im Hinblick auf bestimmte ausgewählte Funktionen. Die Authentifizierung kann, muss aber nicht individuell für eine bestimmte Person erfolgen. In manchen Ausführungsformen reicht es aus, wenn die Person im Zuge der Authentifizierung nachweist, Mitglied einer als vertrauenswürdig eingestuften Personengruppe zu sein, um sich erfolgreich als "Vertrauensperson" authentifiziert zu haben.

Dieses Verfahren kann aus mehreren Gründen vorteilhaft sein: zum einen können Ausführungsformen das Problem überwinden, dass manche biometrischen Daten gestohlen werden können und die gestohlenen Daten es Unberechtigten ermöglichen, sich mit diesen erfolgreich zu authentifizieren. Personen hinterlassen bei fast jeder alltäglichen Aktivität Fingerabdrücke und es ist für Angreifer entsprechend einfach, den Fingerabdruck einer Person von Gegenständen, Türgriffen oder ähnlichen zu erfassen und zur Herstellung gefälschter Fingerabdruck-Replika nutzen. Das Problem, dass ein z.B. rein biometrisches Authentifizierungsverfahren möglicherweise nicht hinreichend sicher ist kann z.B. dadurch überwunden werden, dass zusätzlich noch das zweite, auf der Analyse der Hirnaktivitätssignale basierende Authentifizierungsverfahren durchgeführt wird. Die ersten und zweiten Hirnaktivitätssignale, die gemäß Ausführungsformen der Erfindung zur Authentifizierung einer Person dienen, entstehen jedoch in Antwort auf das Anzeigen bzw. das Betrachten der Bekanntbilder und Fremdbilder. Das Erfassen von Hirnaktivitätssignalen im Alltag der Person wird einen Angreifer nicht in die Lage versetzen, sich durch das Ausstrahlen von heimlich im Alltagsleben einer Person erfassten Hirnaktivitätssignalen dieser einen Person zu authentifizieren, denn die Hirnaktivitätssignale, die entstehen, wenn eine Person fernsieht, sich im Raum bewegt, mit anderen Leuten spricht oder isst unterscheiden sich von den ersten und zweiten Hirnaktivitätssignale, die entstehen, wenn dieser einen Person Bekanntbilder und Fremdbilder gezeigt werden.

Sogar wenn es einem Angreifer gelänge, die ersten und zweiten Hirnaktivitätssignale der Person zu messen, auf deren Basis sich die eine Person erfolgreich gegenüber der Authentifizierungssoftware authentifiziert hat, besteht eine erhebliche technische Schwierigkeit darin, diese Signale über eine technische Sendeeinheit so auszustrahlen, dass sie auf den Sensor wirken wie die echten Hirnaktivitätssignale einer Person in räumlicher Nähe des Sensors.

Und sogar falls der Angreifer eine solche Sendeeinheit erfolgreich hergestellt und in die Räumlichkeiten mit der Anzeige eingeschleust haben sollte, würde dies allein dem Angreifer noch nicht ermöglichen, sich gegenüber der Authentifizierungssoftware zu authentifizieren, denn die Art der gefälschten Hirnaktivitätssignale, die von der Sendeeinheit des Angreifers ausgesendet wird, müsste mit der Art der Gesichtsbilder, die dem Angreifer gezeigt wird, synchronisiert werden. Falls die Reihenfolge der angezeigten Gesichtsbilder abweicht von der Reihenfolge der Anzeige beim unberechtigten Aufzeichnen der Hirnsignale durch den Angreifer wird der Authentifizierungsversuch des Angreifers fehlschlagen. Gleiches gilt, falls der Zeitpunkt des Beginns des Sendens der Hirnsignale nicht zeitgleich ist zu dem Beginn des Anzeigens der Gesichtsbilder. Außerdem würde auch das Gehirn des "Angreifers" beim Anblick der Gesichtsbilder Hirnaktivitätssignale aussenden, die sich mit den Signalen der technischen Sendeeinheit des Angreifers überlagern würden, sodass allein schon deshalb sichergestellt ist, dass das bei dem Sensor ankommende und erfasste Signal nicht identisch ist zu den ersten und zweiten Hirnaktivitätssignalen, die zu einer erfolgreichen Authentifizierung führen würden.

Ein weiterer Vorteil besteht darin, dass das Verfahren sicher ist bezüglich eines Authentifizierungsversuchs eines unberechtigten Dritten, der eine Gesichtsmaske trägt, die aussieht wie das Gesicht einer berechtigten Person, oder der Kontaktlinsen trägt, deren Irismuster aussieht wie das Irismuster einer berechtigten Person, oder die ein sonstiges Replika eines biometrischen Merkmals einer berechtigten Person mit sich führt.

Beispielsweise ist es denkbar, dass eine unberechtigte Person eine Gesichtsmaske trägt, sodass die Maske dem Gesicht der unberechtigten Person ein Aussehen verleiht, das so ähnlich zu dem Gesicht einer berechtigten Person ist, dass weder ein menschlicher Kontrolleur noch eine Gesichtserkennungssoftware die Maske als solche erkennen kann und die unberechtigte Person für die berechtigte Person hält. Auf Basis der Maske kann die unberechtigte Person also möglicherweise das erste Authentifizierungsverfahren überlisten und sich in diesem erfolgreich authentifizieren. Wird dieser eigentlich unberechtigten Person mit der Maske allerdings im Zuge der zweiten Authentifizierung ein Bekanntbild der eigentlich berechtigten Person angezeigt (also z.B. das Gesichtsbilder der berechtigten Person oder ein Bild eines Haustieres oder sonstigen Lebewesens oder Dinges gezeigt, dass der berechtigten Person bekannt ist aber das für die unberechtigte Person ein Fremdbild darstellt), dann wird der Sensor anstatt der zu erwartenden ersten Hirnaktivitätssignale, die bei Anblick eines Bekanntbilds entstehen, zweite Hirnaktivitätssignale, die beim Anblick eines Fremdbilds entstehen, erfassen. Die entsprechenden Bekanntbilder und Fremdbilder können zum Beispiel in einer Mitarbeiterdatenbank hinterlegt sein, wobei zumindest die Bekanntbilder einzelnen berechtigten Mitarbeitern zugewiesen sind. Ein Betrüger kann zwar mithilfe der Maske und eines gefälschten Personalausweises vorübergehend das Aussehen einer eigentlich berechtigten Person annehmen, er kann aber nicht verhindern, dass seine eigenen Hirnsignale beim Betrachten eines Fremdbilds, das ein Motiv beinhaltet, welches zur der berechtigten Person oder dem berechtigten Personenkreis bekannt ist, zu erkennen geben, dass er bzw. sie eine andere Person ist als die, als welche er bzw. sie sich durch Verwendung der Maske vorgibt zu sein.

Ausführungsformen des erfinderischen Authentifizierungsverfahrens können weiterhin den Vorteil haben, dass sie Schutz vor einer Authentifizierung einer unberechtigten Person auf Basis eines gemorphten Bildes auf einem Ausweis, z.B. einem Reisepass oder Personalausweises, bieten. Mittlerweile gibt es Softwareprogramme, die biometrische Fotos von zwei Personen "morphen", also miteinander auf elektronischem Wege zu einem einzigen "gemorphten" Bild kombinieren können. Im Ergebnis zeigt das gemorphte Bild biometrische Merkmale zweier Personen, sodass die Gefahr besteht, dass eine berechtigte Person im Zuge der Ausweiserstellung ein gemorphtes Bild abgibt, sodass sich auf Basis des neuen Ausweises mit dem gemorphten Bild neben der eigentlich berechtigten Person noch eine weitere Person mit diesem Ausweis authentifizieren kann. Die Anmelderin hat jedoch beobachtet, dass eine Person, deren Gesichtsbild für die Erstellung eines gemorphten Bildes verwendet wurde, anders als z.B. Gesichtserkennungssoftware und menschliche Ausweiskontrolleure, sehr wohl erkennt, dass dieses Bild erheblich von einem echten eigenen Bild (Bekanntbild) abweicht. Die Hirnaktivitätssignale einer Person, die ein gemorphtes Bild ihres eigenen Gesichtsbilds betrachtet, sind also den Hirnaktivitätssignalen, die beim Betrachten eines Fremdbildes (z.B. Gesichtsbild einer für den Betrachter fremden Person) entstehen, recht ähnlich, sodass die Authentifizierungssoftware gemäß Ausführungsformen der Erfindung einen Authentifizierungsversuch auf Basis eines Ausweises mit gemorphtem Gesichtsbild erkennen kann, indem Hirnaktivitätssignale beim Betrachten des auf dem Ausweis befindlichen Dokuments analysiert werden. Sind die Hirnaktivitätssignale denjenigen ähnlich, die beim Betrachten von Fremdbildern gemessen werden, scheitert der Authentifizierungsversuch im Rahmen des zweiten Authentifizierungsverfahrens und eine Authentifizierung wird verweigert.

Ein weiterer Vorteil besteht darin, dass die Erfassung der Hirnaktivitätssignale kontaktlos erfolgt. Das Authentifizierungsverfahren gemäß Ausführungsformen der Erfindung ist somit besonders geeignet zur schnellen Authentifizierung einer großen Anzahl von Personen, zum Beispiel im Kontext von Passagierkontrollen an Flughäfen, Personenkontrollen an Grenzübergängen oder Personenkontrollen im Eingangsbereich von Firmengeländen. Viele biometrische Authentifizierungsverfahren erfordern einen physischen Kontakt des sich authentifizierenden Person mit dem Sensor, zum Beispiel zur Erfassung von Fingerabdrücken. Dies erfordert Zeit und reduziert die Nutzbarkeit mancher biometrische Authentifizierungsverfahren in Anwendungsszenarien in welchen mit hohem Personenaufkommen zu rechnen ist. Demgegenüber ermöglicht eine kontaktlose Erfassung der Hirnaktivitätssignale eine sehr schnelle Erfassung der biometrischen Merkmale einer Person.

Gegenüber anderen aus dem Stand der Technik bekannten Verfahren können Ausführungsformen der Erfindung mehrere Vorteile aufweisen: Das Verfahren ist noch sicher gegenüber einem Angreifer, der sich fälschlicherweise als eine andere Person zu authentifizieren versucht, denn das Verfahren beruht nicht notwendigerweise auf dem Vergleich eines beobachteten Merkmals mit dem in einer Datenbank gespeicherten Merkmal, sondern auf dem direkten Vergleich von Hirnaktivitätssignalen, die in Reaktion auf Selbst- bzw. Fremdbilder erzeugt wurden. Das Verfahren benötigt außerdem nicht die Speicherung von Merkmalen einzelner Personen in einer Datenbank, wodurch die Privatsphäre der zu authentifizierenden Person geschützt wird. Hierdurch kann das Verfahren auch in Situationen eingesetzt werden bei denen eine Speicherung personenbezogener, biometrischer Daten unerwünscht ist. Durch die Löschung der Daten kann außerdem die Privatsphäre der sich authentifizierenden Person verbessert werden ("improved privacy").

Bei dem ersten Authentifizierungsverfahren kann es sich um ein biometrisches Authentifizierungsverfahren handeln.

Nach Ausführungsformen besteht die Anzeige aus einer digitalen Anzeige oder umfasst eine digitale Anzeige. Die Bekanntbilder und/oder Fremdbilder sind als digitale Bilder ausgebildet. Die digitale Anzeige ist dazu ausgebildet, die mehreren Gesichtsbilder als digitale Bilder anzuzeigen. Beispielsweise können sowohl die Fremdbilder als auch die Bekanntbilder als Gesichtsbilder von Personen ausgebildet sein, die als digitale Bilder in einer Datenbank gespeichert sind.

Die Verwendung einer digitalen Anzeige hat den Vorteil der größeren apparativen Einfachheit. Digitale Anzeigen in Form von z.B. LCD-, LED- oder OLED-Bildschirmen gibt es in allen möglichen Größen und Formen und zu günstigen Preisen. Oftmals sind bestehende, aktuell schon für Authentifizierungszwecke verwendete Terminals ohnehin schon mit einer digitalen Anzeige ausgestattet.

Nach Ausführungsformen der Erfindung behandelt das zweite Authentifizierungsverfahren die Person als erfolgreich authentifiziert, wenn die ersten Hirnaktivitätssignale sich signifikant unterscheiden von den zweiten Hirnaktivitätssignalen.

Nach Ausführungsformen der Erfindung behandelt das zweite Authentifizierungsverfahren die eine Person als erfolgreich authentifiziert, wenn die ersten Hirnaktivitätssignale einander ähnlich sind, wenn die zweiten Hirnaktivitätssignale einander ähnlich sind, wenn die ersten Hirnaktivitätssignale unähnlich zu den zweiten Hirnaktivitätssignalen sind und wenn die Sequenz voneinander ähnlichen ersten Hirnaktivitätssignalen und einander ähnlichen zweiten Hirnaktivitätssignalen zu der Sequenz der angezeigten Bekanntbildern und Fremdbildern korrespondiert.

Ausführungsformen der Erfindung können den Vorteil haben, dass das Ergebnis der Analyse der ersten und zweiten Hirnaktivitätssignale im Zuge des zweiten Authentifizierungsverfahrens in Abhängigkeit der relativen Beschaffenheit der ersten Hirnsignale gegenüber den zweiten Hirnsignalen erfolgt und somit nicht notwendigerweise das Erfassen, Speichern und Auswerten von absoluten, für eine bestimmte Person spezifischen, Referenzsignalen für die ersten und zweiten Hirnaktivitätssignale erfordert. Die Problematik des Abhandenkommens solcher personenbezogenen biometrischen Referenzdaten kann somit vermieden werden. Zudem können Ausführungsformen der Erfindung den Vorteil haben, dass es nicht erforderlich ist, dass alle Personen beim Betrachten von Bekanntbildern im Wesentlichen identische erste Hirnaktivitätssignale erzeugen und dass alle Personen beim Betrachten von Fremdbildern im Wesentlichen identische zweite Hirnaktivitätssignale erzeugen. Vielmehr kann es ausreichend sein, festzustellen, dass das Betrachten mehrerer Bekanntbilder in reproduzierbarer Weise das Erzeugen von ersten Hirnaktivitätssignalen bewirkt, die zueinander signifikant ähnlich sind, und auch das Betrachten mehrerer Fremdbilder in reproduzierbarer Weise das Erzeugen von zweiten Hirnaktivitätssignalen bewirkt, die zueinander signifikant ähnlich sind, wohingegen jedes der ersten Hirnaktivitätssignale sich signifikant von jedem der zweiten Hirnaktivitätssignale unterscheidet.

Gemäß einer Ausführungsform der Erfindung enthalten die angezeigten Bilder mehrere ermittelte Bekanntbilder und mehrere ermittelte Fremdbilder. Das zweite Authentifizierungsverfahren behandelt die Person als erfolgreich authentifiziert, wenn die ersten Hirnaktivitätssignale sich signifikant und in einer über eine Mindestanzahl and ersten und zweiten Hirnaktivitätssignalen hinweg reproduzierbaren Weise unterscheiden von den zweiten Hirnaktivitätssignalen.

Ausführungsformen der Erfindung können den Vorteil haben, dass das Anzeigen mehrerer Bekanntbilder und mehrerer Fremdbilder es der Authentifizierungssoftware erlaubt, zu prüfen, ob sich die ersten Hirnaktivitätssignale in reproduzierbarer Weise von den zweiten Hirnaktivitätssignalen unterscheiden. Würde nur ein einziges erstes Hirnaktivitätssignal erfasst und mit einem einzigen zweiten Hirnaktivitätssignal verglichen, bestünde ein erhöhtes Risiko, dass das zweite Authentifizierungsverfahren aufgrund messtechnischer Varianzen ein falsch positives oder falsch negatives Ergebnis liefert.

Nach Ausführungsformen ist die Authentifizierungssoftware dazu konfiguriert, im Zuge des zweiten Authentifizierungsverfahrens die Variabilität verschiedener erster Hirnaktivitätssignale und/oder die Variabilität verschiedener zweiter Hirnaktivitätssignale zu bestimmen. Überschreitet die Variabilität der ersten und/oder zweiten Hirnaktivitätssignalen einen vorbestimmten Grenzwert, bricht die Authentifizierungssoftware das zweite Authentifizierungsverfahren ab, wobei vorzugsweise eine Fehlermeldung ausgegeben wird. Die Person hat sich somit im zweiten Authentifizierungsverfahrens nicht authentifiziert. Dies kann vorteilhaft sein, falls die Erfassung von Hirnaktivitätssignalen aufgrund verschiedener Störsignale oder sonstige ungünstige Umstände nur in einer Qualität gelingt, die eine verlässliche Authentifizierung nicht zulassen. In der Regel würde eine starke Störung der vom Sensor erfassten Hirnaktivitätssignale dazu führen, dass der Authentifizierungsversuch der Person im zweiten Authentifizierungsverfahren abgelehnt wird und die Person als "nicht berechtigt" gilt. In manchen Fällen kann dies mit negativen Konsequenzen für die Person verbunden sein, wenn zum Beispiel die Authentifizierungssoftware den Account der Person nach einer gewissen Anzahl an fehlgeschlagenen Authentifizierungsversuchen sperrt. Nach Ausführungsformen der Erfindung führt ein Abbruch des zweiten Authentifizierungsverfahrens aufgrund hoher Signalvarianz nicht zu negativen Konsequenzen für die sich authentifizierende Person, da der Grund des Scheiterns der Authentifizierung in der hohen Signalvarianz begründet ist.

Gemäß einer Ausführungsform der Erfindung sind die empfangenen Daten der Person biometrische Daten der Person. Das erste Authentifizierungsverfahren ist ein biometrisches Authentifizierungsverfahren, insbesondere ein Verfahren der automatischen Gesichtserkennung und/oder ein Authentifizierungsverfahren basierend auf Fingerabdruckdaten oder Irisbildern.

Gemäß Ausführungsformen handelt es sich bei dem ersten Authentifizierungsverfahren um ein nicht-biometrisches Authentifizierungsverfahren, z.B. ein passwortbasiertes und/oder ID-Dokument-basiertes Authentifizierungsverfahren.

Ausführungsformen der Erfindung können den Vorteil haben, dass Nachteile, die mit klassischen biometrischen Authentifizierungsverfahren verbunden sind, vermieden werden können und gleichzeitig die Vorteile biometrischer Verfahren genutzt werden können. Beispielsweise können klassische biometrische Authentifizierungsverfahren den Nachteil haben, dass biometrische Authentifizierungsdaten nicht einfach zurückgesetzt werden können (wie das beispielsweise bei Passwörtern problemlos möglich ist). Biometrische Daten können gestohlen werden. Auf der Basis von Fingerabdrücken, die auf Gegenständen wie Türklinken oder Trinkgläsern vielfach hinterlassen werden, können Folien bzw. Kunststoffreplika erzeugt werden, die den auf Gegenständen hinterlassenen Fingerabdruck akkurat widergeben und von unberechtigten Dritten dazu verwendet werden können, sich erfolgreich zu authentifizieren. Mittlerweile ist bekannt, dass noch nicht einmal ein physischer Fingerabdruck verfügbar sein muss, um diesen ein gefälschtes Fingerabdruckreplika zu erstellen. Ein hochauflösendes Bild des Fingerabdrucks, das irgendwo online gespeichert ist, reicht für die Erstellung dieses Replikas aus. Wenn biometrische Daten kompromittiert werden, können die Folgen weitreichend sein, da man sie nicht zurücksetzen kann. Ein Fingerabdruck oder Blutgefäßmuster lässt sich nur bedingt oder gar nicht ändern. Wenn ein Kennwort kompromittiert wird, kann es dagegen leicht geändert werden. Biometrische Daten haben aber den Vorteil, dass sie an sich charakteristisch für die Person sind und von der Person automatisch immer mitgeführt werden, ohne dass hierfür ein Passwort erinnert oder ein ID-Token mitgeführt werden muss. Ausführungsformen der Erfindung nutzen die Hirnaktivitätssignale zur Authentifizierung, wobei allerdings nicht auf einmalig erfasste, statische biometrische Merkmale einer Person zurückgegriffen wird, sondern ein dynamisch erzeugtes Challenge-Response Verfahren mit biometrischen Hirnwellendaten zur Authentifizierung verwendet wird. Falls die in einer Datenbank hinterlegten Bekanntbilder einer Person entwendet werden, sodass unberechtigte Dritte diese durch häufiges Betrachten womöglich zu "Bekanntbildern" machen, können die Bilder einfach ersetzt werden.

Die Verwendung von ID-Dokumenten wie zum Beispiel Reisepässen, Personalausweisen oder Mitarbeiterausweisen zur Authentifizierung im ersten Authentifizierungsverfahren kann vorteilhaft sein, da diese Dokumente oftmals weitere Sicherheitsmerkmale und/oder personenbezogene Daten beinhalten, die eine zusätzliche manuelle Eingabe von einer Nutzer-ID, z.B. eines Namens, im Zuge des ersten Authentifizierungsverfahrens überflüssig machen, da diese Daten in dem oder auf dem Dokument enthalten sind und automatisch optisch oder elektronisch erfasst werden können. Zudem kann die Sicherheit weiter erhöht werden, indem ein ID-Dokument verwendet wird, das ein oder mehrere weitere Sicherheitsmerkmale enthält wie zum Beispiel Hologramme, Prüfzahlen, kryptographische Schlüssel oder dergleichen, und diese im Zuge des ersten Authentifizierungsverfahren zusätzlich ausgewertet werden.

Gemäß einer Ausführungsform der Erfindung ist der KLHA-Sensor integriert und/oder operativ gekoppelt an ein Terminal.

Gemäß einer weiteren Ausführungsform der Erfindung ist die Authentifizierungssoftware integriert und/oder operativ gekoppelt an das Terminal.

Gemäß einer Ausführungsform der Erfindung erzeugt die Authentifizierungssoftware für jeden Authentifizierungsvorgang der Person mit dem zweiten Authentifizierungsverfahren eine zufällige Reihung der mehreren für die Person ermittelten Bilder. Die Anzeige der mehreren Bilder erfolgt derart, dass die chronologischen Sequenz der angezeigten Bilder dieser zufälligen Reihung entspricht. Die Analyse der erfassten ersten und zweiten Hirnaktivitätssignale erfolgt unter Berücksichtigung der chronologischen Sequenz.

Beispielsweise kann die Authentifizierungssoftware dazu konfiguriert sein, jeder Person, die sich authentifizieren möchte, eine Sequenz aus sechs Gesichtsbildern anzuzeigen. Die sechs Gesichtsbilder können zum Beispiel immer eine festgelegte Zahl an Bekanntbildern und Fremdbildern umfassen, zum Beispiel immer zwei Bekanntbilder und 4 Fremdbilder. Es ist aber auch möglich, dass der relative Anteil von Fremdbildern und Bekanntbildern bei jedem Authentifizierungsverfahren ebenfalls zufällig neu gewählt wird, solange nur sichergestellt ist, dass die mehreren angezeigten Bilder mindestens ein Bekanntbild, vorzugsweise mehrere Bekanntbilder, und mindestens ein Fremdbild, vorzugsweise mehrere Fremdbilder, beinhalten.

Nach Ausführungsformen dient die zufällige Reihung als Challenge im Sinne eines Challenge-Response-Verfahrens. Die Analyse der erfassten Hirnaktivitätssignale umfasst eine Prüfung, ob die zeitliche Reihenfolge der ersten und zweiten Hirnaktivitätssignale die in der Challenge codierte Reihenfolge von Bekanntbildern und Fremdbildern widergibt.

Ausführungsformen der Erfindung können den Vorteil haben, dass hierdurch ein besonders wirksamer Schutz gegen Replay-Angriffen erreicht werden kann. Außerdem können Nachteile, die mit klassischen biometrischen Authentifizierungsverfahren verbunden sind, vermieden und gleichzeitig die Vorteile biometrischer Verfahren genutzt werden können. Wie oben bereits erläutert können klassische biometrische Authentifizierungsdaten nicht einfach zurückgesetzt werden, was ein Problem darstellt, sollten diese gestohlen werden. Ausführungsformen der Erfindung nutzen dagegen eine spezifische Sequenz von Hirnaktivitätssignalen zur Authentifizierung, wobei nicht einmalig erfasste, statische biometrische Merkmale einer Person verwendet werden, sondern ein dynamisch erzeugtes Challenge-Response Verfahren bei dem die Authentifizierungssoftware dynamisch und für jeden Authentifizierungsversuch individuell (z.B. mittels eines Zufallsgenerators) die Sequenz der angezeigten Bekanntbilder und Fremdbilder bestimmt. Falls die in einer Datenbank hinterlegten Bekanntbilder einer Person entwendet werden, sodass unberechtigte Dritte diese durch häufiges Betrachten womöglich zu "Bekanntbildern" machen, können die Bilder einfach ersetzt werden. Die Authentifizierung basiert auf einer Analyse des Wechsels bestimmter Hirnaktivitätssignale, die sich für "Bekanntbilder" und "Fremdbilder" signifikant unterscheiden, wobei es hierfür nicht zwingend notwendig ist, dass ein für eine Person "charakteristisches" Hirnwellenreferenzsignal einmalig erfasst und als Referenz gespeichert wird.

Nach Ausführungsformen behandelt die Authentifizierungssoftware die Person im zweiten Authentifizierungsverfahren nur dann als erfolgreich authentifiziert, wenn neben einer signifikanten Unterschiedlichkeit der ersten und zweiten Hirnaktivitätssignale folgende weitere Kriterien zutreffen:
- alle ersten Hirnaktivitätssignale, die je während des Anzeigens eines der Bekanntbilder erfasst werden, sind identisch oder hinreichend ähnlich zueinander;
- alle zweiten Hirnaktivitätssignale, die je während des Anzeigens eines der Fremdbilder erfasst werden, sind identisch oder hinreichend ähnlich zueinander.

Ein Vorteil des Authentifizierungsverfahrens gemäß Ausführungsformen der Erfindung kann darin bestehen, dass es nicht notwendig ist, dass die ersten Hirnaktivitätssignale bei verschiedenen Personen identische oder nahezu identische Eigenschaften aufweisen. Die Anmelderin hat beobachtet, dass auch dann, wenn die durch Betrachtung von Bekanntbildern ausgelösten ersten Hirnaktivitätssignale von Person zu Person deutlich unterschiedlich sind, sie dann doch zumindest sehr deutlich unterschiedlich sind von den zweiten Hirnaktivitätssignalen, die beim Betrachten von Fremdbildern erzeugt werden. Unter der Voraussetzung dass der zu authentifizieren Person eine hinreichend große Anzahl von Fremdbildern und Bekanntbildern gezeigt wird, kann eine Analyse der zeitlichen Abfolge der Eigenschaften der Hirnaktivitätssignale während der Anzeigezeit für eine korrekte Authentifizierungsentscheidung ausreichend sein. Beispielsweise können der zu authentifizierenden Person mindestens zwei, vorzugsweise mindestens 3 Bekanntbilder und mindestens zwei, vorzugsweise mindestens 5 Fremdbilder angezeigt werden. Die Hirnaktivitätssignale der Person werden während der Anzeige der verschiedenen Bekanntbilder und Fremdbilder kontinuierlich erfasst und mit der bekannten zeitlichen Sequenz der Anzeige der Fremdbilder und Bekanntbilder verglichen. Stellt sich heraus, dass die ersten Hirnaktivitätssignale, die erfasst wurden, während die eine Person die Bekanntbilder betrachtete, alle identische oder sehr ähnliche Eigenschaften haben, und dass die zweiten Hirnaktivitätssignale, die erfasst wurden, während die eine Person die Fremdbilder betrachtete, alle identische oder sehr ähnliche Eigenschaften haben, wobei die Eigenschaften der ersten und zweiten Hirnaktivitätssignale signifikant unterschiedlich sind, gilt die eine Person gemäß Ausführungsformen der Erfindung als im zweiten Authentifizierungsverfahren erfolgreich authentifiziert.

Gemäß manchen Ausführungsformen haben die ersten Hirnaktivitätssignale Eigenschaften, die bei allen Menschen gleich oder sehr ähnlich sind, und auch die zweiten Hirnaktivitätssignale haben Eigenschaften, die bei allen Menschen gleich oder sehr ähnlich sind, wobei die ersten und zweiten Hirnaktivitätssignale der allen Menschen signifikant unterschiedlich sind. In diesem Fall kann die Analyse der erfassten ersten und zweiten Hirnaktivitätssignale im Zuge des zweiten Authentifizierungsverfahrens auch einen Vergleich der erfassten ersten und zweiten Hirnaktivitätssignale mit gespeicherten ersten und zweiten Hirnaktivitäts-Referenzsignalen umfassen.

Die von dem KLHA-Sensor erfassten Hirnaktivitätssignale können zum Beispiel drahtgebunden (zum Beispiel per Ethernet-Kabel) oder über eine drahtlose Schnittstelle (zum Beispiel Bluetooth) an die Authentifizierungssoftware übertragen werden.

Die Authentifizierungssoftware kann dazu ausgebildet sein, die Hirnaktivitätssignale, die von dem KLHA-Sensor empfangen werden, zu korrelieren mit weiteren Messdaten. Beispielsweise kann eine Kamera die Position und/oder Orientierung des Kopfes der einen Person im Raum erkennen und an die Authentifizierungssoftware weiterleiten. Änderungen der Position und/Orientierung des Kopfes können also mit Signaländerungen korreliert werden. Dies kann hilfreich sein, denn nach Ausführungsformen der Erfindung verwendet die Authentifizierungssoftware diese weiteren Messdaten und die erfassten Korrelationen, um Störsignale, die durch Bewegung des Kopfes im Raum während der Authentifizierung erzeugt werden, herauszufiltern, sodass nur oder vorwiegend diejenigen Messsignale übrig bleiben, die ursächlich auf die Betrachtung von Fremdbildern und Bekanntbildern bzw. auf die Beobachtung eines Bildwechsels zurückzuführen sind. Auch Vibrationen des Sensors, die darauf beruhen, dass Vibrationen, die z.B. durch Geh- und Dreh-Bewegungen der Person im Raum während des Authentifizierungsprozesses erzeugt werden und durch mechanische Kopplung an den Sensor übertragen werden, können bei manchen Sensortypen automatisch erfasst werden, z.B. mittels des als KLHA Sensor verwendeten elektrischen Sensors oder eines weiteren elektrischen, gyroskopischen oder optischen Sensors. Zusätzlich oder alternativ dazu kann die Kamera eine Serie von Gesichtsbildern der sich authentifizierenden Person über einen Zeitraum erfassen und diese Gesichtsbilder an die Authentifizierungssoftware weiterleiten. Durch Analyse der zeitlichen Serie der Gesichtsbilder kann die Authentifizierungssoftware beispielsweise erkennen, ob und wann die eine Person mit den Augen blinzelt und/oder ob und wann die Person eine deutliche Emotion (Freude, Angst, Schreck, Unsicherheit) erlebt, die zum Beispiel anhand des Gesichtsausdrucks erkennbar ist. Hirnaktivitätssignale, die mit solchen Emotionen einhergehen, können die durch die Betrachtung der Bekanntbilder und Fremdbilder erzeugten Hirnaktivitätssignale überlagern und zu Fehlern führen. Auch die mit starken Emotionen einhergehenden Hirnaktivitätssignale werden gemäß Ausführungsformen der Erfindung von der Authentifizierungssoftware automatisch als Störsignale erkannt und herausgefiltert.

Nach Ausführungsformen beinhaltet die Ermittlung der ein oder mehreren Bekanntbilder für die Person eine Erfassung von einem oder mehreren Gesichtsbildern der Person durch eine Kamera.

Dies kann vorteilhaft sein, da dieser Schritt in mehreren existierenden auf Gesichtserkennung beruhenden Authentifizierungsverfahren ohnehin enthalten ist. Nach Ausführungsformen der Erfindung ist das erste Authentifizierungsverfahren ein automatisches Gesichtserkennungsverfahren und die Ermittlung der ein oder mehreren Bekanntbilder erfolgt als Teil des ersten Authentifizierungsverfahrens. Dies kann zum einen das gesamte Authentifizierungsverfahren beschleunigen, da kein zusätzlicher Bilderfassungsschritt erforderlich ist. Zum Anderen kann dies den Vorteil haben, dass eine Erfassung und Speicherung von Bekanntbildern speziell nur für das zweite Authentifizierungsverfahren überflüssig ist. Die Erfassung und Speicherung von Bekanntbildern auf eine Weise, dass die Bekanntbilder spezifisch einer berechtigten Person oder Personengruppe zugeordnet sind, kann aufwändig sein, sodass die Verwendung von Gesichtsbilddatenbanken oder davon abgeleiteten Gesichtsbildprofilen, wie sie derzeit schon für etablierte Authentifizierungsverfahren auf Basis automatischer Gesichtserkennung verwendet werden, kann die Etablierung des Authentifizierungsverfahrens erleichtern und beschleunigen. Die Erzeugung von Fremdbildern stellt dagegen in der Regel einen geringeren technischen Aufwand dar. Beispielsweise können Bilder von immobilen Gegenständen, Tierarten, Fahrzeugen oder Gebäuden verwendet werden. Die Gesichtsbilder eines "Betrügers" mit einer Maske sind Bekanntbilder in dem Sinne, dass das in der Maske repräsentierte und von der Kamera erfasste Gesichtsbild der Person, dessen Gesicht die Maske nachbildet, also der Vertrauensperson, bekannt sind. Der sich authentifizierenden Person sind die "Maskengesichtsbilder" jedoch nicht bekannt, zumindest sind sie nicht so vertraut, wie dies bei den eigenen Gesichtsbildern der Fall wäre. Die Authentifizierungssoftware wird also während des Anzeigens von Bildern der Gesichtsmaske "erste Hirnaktivitätssignale" erwarten, die sich deutlich von denen unterscheiden, die beim Anzeigen von Fremdbildern erfasst werden. Die tatsächlich gemessenen Signale entsprechen dieser Erwartung jedoch nicht, da die Maskenbilder für die sich authentifizierende Person letztlich ähnlich fremd sind wie die Fremdbilder. Aufgrund der Diskrepanz zwischen den erwarteten und tatsächlich gemessenen Signalähnlichkeiten wird die Authentifizierungssoftware eine Authentifizierung der Person als die Vertrauensperson zurückweisen.

Zusätzlich oder alternativ zu den oben genannten Optionen zur Ermittlung von Bekanntbildern beinhaltet nach Ausführungsformen die Ermittlung der ein oder mehreren Bekanntbilder für die Person eine Erfassung von einem oder mehreren Bildern eines auf einem Identitätsdokument der Person abgebildeten Gesichtsbilds der Person durch eine Kamera. Beispielsweise kann das zweite Authentifizierungsverfahren vorsehen, dass die Person zur Authentifizierung ein ID-Dokument, z.B. einen Personalausweis, einen Reisepass oder einen Mitarbeiterausweis, auf dem ein Gesichtsbild einer Person abgebildet ist, an einer bestimmten Position unter die Kamera eines Terminals hält. Die Kamera erfasst das auf dem Ausweis dargestellte Gesicht als digitales Bekanntbild und zeigt dieses - ggf. mehrfach in einem zufälligen Wechsel mit Fremdbildern - über die Anzeige an. Für den Fall, dass es sich bei dem Gesichtsbild auf dem Ausweis um ein gemorphtes Bild handelt, das teilweise eine andere Person zeigt als die, die den Ausweis vor die Kamera hält, wird die Person, die sich aktuell versucht zu authentifizieren, beim Betrachten dieser "Bekanntbilder" Hirnaktivitätssignale aussenden, die den ebenfalls gezeigten Fremdbildern ähnlich sind. Somit wird auf die "Challenge" in Form der Bildsequenzen nicht mit einer passenden "Response" in Form von zu dieser Sequenz passenden ersten und zweiten Hirnaktivitätssignalen gemessen.

Zusätzlich oder alternativ dazu beinhaltet nach Ausführungsformen die Ermittlung der ein oder mehreren Bekanntbilder für die Person eine Identifikation der Bekanntbilder und/oder Fremdbilder in einer Bilddatenbank.

Zusätzlich oder alternativ dazu beinhaltet nach Ausführungsformen der Erfindung die Ermittlung der ein oder mehreren Fremdbilder für die Person eine Identifikation der Bekanntbilder und/oder der Fremdbilder in einer Bilddatenbank.

Beispielsweise kann die Bilddatenbank Bekanntbilder enthalten, die Gesichtsbilder von Personen sind, die der Authentifizierungssoftware individuell oder als Mitglied einer Gruppe bekannt sind (also z.B. registriert sind) und als vertrauenswürdig gelten. Beispielsweise kann es sich bei den Gesichtsbildern um Bilder handeln, die in einer sicheren Umgebung erfasst wurden, z.B. innerhalb der Räumlichkeiten einer Behörde oder einer Firma, die das Authentifizierungsverfahren durchführt. Dies kann Morphingangriffe verhindern. Zusätzlich oder alternativ dazu kann es sich bei den Motiven der Bekanntbilder aber auch um physische Objekte jeglicher Art handeln, die nur einer bestimmten, berechtigten Person oder Personengruppe bekannt sind, der bzw. denen die Bekanntbilder in der Datenbank auch zugewiesen sind, wohingegen diese Motive für alle oder die allermeisten anderen Personen unbekannt oder zumindest deutlich weniger bekannt sind. Beispielsweise können die Mitarbeiter einer Firma verschiedenen Arbeitsgruppen oder Abteilungen angehören, die selektiv je nach Gruppen- bzw. Abteilungszugehörigkeit an unterschiedlichen Standorten und/oder in unterschiedlichen Gebäuden oder Räumen arbeiten. Bilder, die Objekte innerhalb von Gebäuden oder Räumen zeigen, in welchem nur eine bestimmte Personengruppe arbeitet, sind Bekanntbilder für die Personen dieser Gruppe, und gleichzeitig Fremdbilder für Personen, die diese Gebäude und Räume nicht betreten (vorausgesetzt, die gezeigten Objekte sind nicht in beiden Gebäuden vorhanden). Gemäß einer weiteren Ausführungsform kann es sich bei den einer Person zugeordneten Bekanntbildern auch um Bilder von Familienangehörigen, Haustieren, Einrichtungsgegenständen der von der Person bewohnten Wohnung oder von Fahrzeugen der Person handeln.

Beispielsweise kann es sich bei der Bilddatenbank um eine Datenbank mit Gesichtsbildern von Mitarbeitern einer Firma oder sonstigen Organisation handeln. Beispielsweise kann die Person, die sich authentifizieren möchte, im Zuge des ersten Authentifizierungsverfahrens ihren Namen eingeben oder die Authentifizierungssoftware erkennt auf andere Weise, zum Beispiel durch automatische Gesichtserkennung, welche aus einer Vielzahl von registrierten Personen (zum Beispiel Mitarbeitern) sich eben authentifizieren möchte. Auf Basis dieser Information durchsucht die Authentifizierungssoftware die Bilddatenbank und ermittelt ein oder mehrere Bekanntbilder, die in der Datenbank verknüpft mit dem eingegebenen Namen bzw. einem automatisch erkannten Identifikator dieser Person gespeichert sind. Bei den Bekanntbildern kann es sich z.B. um Gesichtsbilder dieser registrierten Person handeln. Im Zuge des Authentifizierungsprozesses kann es z.B. darum gehen, dass sich eine Person als die Person ("Vertrauensperson") ausweist, als welche sie sich vormals bei der Authentifizierungssoftware registriert hatte.

In manchen Ausführungsformen können die Bekanntbilder anstelle dieser Gesichtsbilder oder zusätzlich zu diesen Gesichtsbildern der registrierten Person Bilder beinhalten, von welchen der Betreiber der **Authentifizierungssoftware** weiß oder verlässlich annehmen kann, dass das auf den Bekanntbildern abgebildete Motiv der Vertrauensperson bekannt ist, die die sich authentifizierte Person vorgibt zu sein (explizit durch Eingabe entsprechender Nutzer-IDs oder Ausweisdokumente, implizit durch das Positionieren des Gesichts im Erfassungsbereich der Kamera). Bei dieser bestimmten Person kann es sich z.B. um eine Person handeln, die bei der Authentifizierungssoftware individuell oder als Mitglied einer Personengruppe registriert ist und deren digitaler Repräsentanz (Nutzerprofil der Vertrauensperson) als vertrauenswürdig gilt. Der digitalen Repräsentanz können ein oder mehrere Bekanntbilder zugeordnet sein. Die Zuordnung kann z.B. über einen sekundären Schlüssel einer relationalen Datenbank realisiert sein, welche Nutzerprofile registrierter Personen oder Personengruppen je mit einem oder mehreren digitalen Bekanntbildern verknüpft. Die Zuordnung kann auch dynamisch während des ersten Authentifizierungsverfahren ermittelt bzw. etabliert werden, wenn die Authentifizierungssoftware anhand eines von einer Kamera aktuell erfasstes Gesichtsbilds einer Person das dieser Person gehörende Nutzerprofil ermittelt. Das im ersten Authentifizierungsverfahren erfasste Gesichtsbild wird also im ersten Schritt einem Nutzerprofil zugeordnet, z.B. dem Nutzerprofil der Vertrauensperson, die die sich authentifizierende Person vorgibt zu sein. Dann im zweiten Authentifizierungsverfahren wird das im ersten Authentifizierungsverfahren erfasste Gesichtsbild als ein dieser Vertrauensperson zugeordnetes Bekanntbild behandelt.

Gemäß Ausführungsformen ermittelt die Authentifizierungssoftware ein oder mehrere Fremdbilder für die sich aktuell authentifizierende Person. Die Fremdbilder können z.B. Gesichter von anderen Personen abbilden, die nicht die Person darstellen, die sich eben authentifizieren möchte. Bei den anderen Personen kann es sich um andere Mitarbeiter bzw. Mitglieder der Organisation handeln, die sich im Prinzip mittels dieser Bilder authentifizieren könnten. In diesem Fall kann ein bestimmtes Gesichtsbild sowohl als Bekanntbild als auch als Fremdbild von der Authentifizierungssoftware verwendet werden je nachdem, welche Person sich gerade authentifizieren möchte: ist ein Gesichtsbild in der Bilddatenbank oder aufgrund einer dynamischen Gesichtserkennung einem Nutzerprofil einer bestimmten Person zugeordnet, wird dieses Bild von der Authentifizierungssoftware als ein Bekanntbild für diese Person verwendet. Wenn keine derartige Zuordnung vorhanden ist, kann das Bild als Fremdbild für die sich authentifizierende Person verwendet werden.

In anderen Ausführungsformen zeigen die in der Datenbank gespeicherten Fremdbilder Personen, die nicht Mitglied der besagten Firma bzw. Organisation sind, die also niemals bei einem Authentifizierungsverfahren für diese Organisation als Bekanntbild dienen können, da sie nicht das Gesicht einer registrierten Person bzw. eines registrierten Organisationsmitglieds abbilden. Die Verwendung von Gesichtsbildern von Nicht-registrierten Personen als Fremdbilder hat den Vorteil, dass sichergestellt ist, dass das in einem Fremdbild dargestellte Gesicht der sich authentifizieren Person sicherlich nicht bekannt ist, wohingegen im Hinblick auf Gesichtsbilder von Mitarbeitern im Einzelfall ein Wiedererkennungseffekt in den Hirnaktivitätssignalen erkennbar sein kann, wenn es sich zum Beispiel um einen Kollegen handelt, dem der Mitarbeiter täglich begegnet. Die Verwendung von Gesichtsbildern Organisations-externer Personen als Fremdbilder kann den Vorteil haben, dass automatisch eine recht große Menge unterschiedlicher Gesichtsbilder verfügbar ist, z.B. in entsprechenden kommerziellen Bilddatenbanken.

Gemäß manchen Ausführungsformen kann es sich bei den Fremdbildern aber auch um Bilder handeln, die physische Objekte jeglicher Art (Personen, Tiere, Gebäude, Landschaften, Fahrzeuge, Gegenstände, etc.) darstellen, von welchen der Betreiber der Authentifizierungssoftware mit hinreichend hoher Wahrscheinlichkeit davon ausgehen kann, dass diese physischen Objekte keine Person, die als vertrauenswürdige Person bei der Authentifizierungssoftware registriert sind und ein entsprechendes Nutzerprofil haben, jemals gesehen hat oder zumindest nicht regelmäßig über einen längeren Zeitraum (von z.B. mehreren Wochen, mehreren Monaten oder gar mehreren Jahren) gesehen hat. Auch die Bekanntbilder können Bilder sein, die die entsprechenden physischen Objekte abbilden, wobei hier der Betreiber der Authentifizierungssoftware mit hinreichend hoher Wahrscheinlichkeit davon ausgehen kann, dass diese physischen Objekte der vertrauenswürdigen und registrierten Person, der das Bekanntbild zugeordnet ist, regelmäßig über einen längeren Zeitraum (von z.B. mehreren Wochen, mehreren Monaten oder gar mehreren Jahren) gesehen hat.

Vorzugsweise ist der Typ des Motives, das in den Bekanntbildern und den Fremdbildern der Person im Zuge des zweiten Authentifizierungsverfahren gezeigt wird, identisch, lediglich der Bekanntheitsgrad des in den Bekanntbildern und Fremdbildern jeweils gezeigten Motivs für den sich authentifizierenden Nutzer ist unterschiedlich. Beispielsweise werden in ersten Ausführungsformen der Erfindung Fremdbilder verwendet, die Gesichter fremder Personen zeigen, und es werden entsprechend Bekanntbilder verwendet, die Gesichtsbilder sind und die dem Nutzerprofil der Person, die sich im ersten Authentifizierungsverfahren authentifiziert hat, zugewiesen sind. Beispielsweise werden in zweiten Ausführungsformen der Erfindung Fremdbilder verwendet, die ausgefallene Gegenstände wie z.B. Vasen oder Kunstwerke zeigen, die allgemein unbekannt sind und sich auch nicht in den Arbeitsräumlichkeiten befinden, in denen die Person, die sich im ersten Authentifizierungsverfahren authentifiziert hat, regelmäßig arbeitet, und es werden entsprechende Bekanntbilder von Gegenständen des gleichen Typs (Vasen, Kunstwerke, etc.) verwendet, die sich ausschließlich in den Arbeitsräumlichkeiten befinden, in denen die besagte Person regelmäßig arbeitet. Beispielsweise werden in dritten Ausführungsformen der Erfindung Fremdbilder verwendet, die Haustiere wie z.B. Hunde oder Katzen zeigen, die allgemein unbekannt sind (z.B. Bilder entsprechender Tiere aus anderen Ländern oder weit entfernter Orte), und es werden entsprechende Bekanntbilder von physischen Objekten des gleichen Typs (Hunde, Katzen, etc.) verwendet, die ein Haustier der Person abbilden, die sich im ersten Authentifizierungsverfahren erfolgreich authentifiziert hat.

Die Verwendung typgleicher Bildmotive bei Bekanntbildern und Fremdbildern kann den Vorteil haben, dass Unterschiede der ersten und zweiten Hirnaktivitätssignale ausschließlich auf den unterschiedlichen Bekanntheitsgrad zurückzuführen sind und nicht auf die Unterschiedlichkeit der angezeigten Bildmotive (Gesicht, Haus, Kunstgegenstand, Tier, etc.).

### Elektrische(r) Sensor(en)

Nach Ausführungsformen der Erfindung ist der KLHA -Sensor ein elektrischer Sensor.

Insbesondere kann dieser Sensor dazu ausgebildet sein, Ladungsänderungen und/oder Ladungsumverteilungen in der Nähe des Sensors zu messen. Der Sensor kann z.B. als ein Sensor ausgebildet sein, der einen Instrumentenverstärker oder Differenzverstärker mit hoher Gleichtaktunterdrückung beinhaltet, wobei der Verstärker mit Messelektroden gekoppelt wird. Beispielsweise kann der elektrische Sensor in verschiedene Gegenstände oder Geräte integriert sein, die sich in der Nähe des Kopfes der zu authentifizierenden Person befinden. Beispielsweise kann der elektrische Sensor in einen Stuhl oder Sessel, insbesondere in die Kopfstütze dieses Stuhls bzw. Sessels, integriert sein. Es ist auch möglich, dass die Person sich im Zuge des Authentifizierungsprozesses in eine begehbare Box begibt und der elektrische Sensor in, unter oder auf einer Seitenwand oder Decke dieser Box integriert ist. Die Person kann also während des Authentifizierungsprozesses ihren Kopf zumindest innerhalb eines gewissen Radius frei bewegen und drehen, und es ist nicht erforderlich, dass die Person Elektroden an bestimmten Bereichen des Kopfes platziert, damit Hirnaktivitätssignale erfasst werden können.

Der elektrische Sensor kann zum Beispiel einen Hochpassfilter beinhalten oder an diesen gekoppelt sein der Signalkomponenten geringer Frequenz zurückhält. Beispielsweise kann der Hochpassfilter Hirnaktivitätssignale mit einer Frequenz von mindestens einem Herz ungehindert passieren lassen, aber Signale mit einer Frequenz von unter einem Herz abschwächen oder komplett herausfiltern. Hierdurch kann Hintergrundrauschen reduziert werden.

Der Verstärker kann es ermöglichen, Hirnaktivitätssignale mit einer Amplitude ab 10 Nanovolt zu erfassen. Ein elektrischer Sensor mit einer derartigen Signalverstärkung ist um ein Vielfaches sensitiver als konventionelle, kontaktbehaftete EEG Technologie, die in der Regel Hirnaktivitätssignale erst ab einer Amplitude von mindestens einigen Mikrovolt erkennen kann.

Gemäß Ausführungsformen der Erfindung beinhaltet die Authentifizierungssoftware ein Modul zur Datenvorverarbeitung , das dazu ausgebildet ist, Störsignale zu erkennen und durch automatische Filterung zu minimieren, sodass die ersten und zweiten Hirnaktivitätssignale als störungsminimierte, "entrauschte" erste und zweite Hirnaktivitätssignale bereitgestellt und von einem anderen Modul der Authentifizierungssoftware weiterverarbeitet werden. Die Störsignale können z.B. durch Vibrationen des KLHA-Sensors, durch den Herzschlag der einen Person, durch Ändern der Position und/oder Ausrichtung des Kopfes der einen Person im Raum und/oder durch Blinzelbewegungen oder starke Emotionen der einen Person hervorgerufen werden.

Beispiele für geeignete Hochpassfilter und Signalverstärker sind in der internationalen Patentanmeldung WO 2017/189748 beschrieben.

Im Stand der Technik sind verschiedene Sensoren zur kontaktlosen Erfassung von Hirnaktivitätssignalen, insbesondere elektrische Sensoren, bekannt, die allerdings nicht zur Authentifizierung von Personen verwendet wurden.

Beispielsweise beschreibt die internationale Patentanmeldung WO 2017/189748 verschiedene Typen geeigneter Sensoren. Der elektrische Sensor kann beispielsweise in Form einer kontaktlosen Elektrode, zum Beispiel einer trockenen Elektrode, die kein Kontaktgel benötigt und die nicht in physischen Kontakt mit der einen Person ist, ausgebildet sein. Derartige Sensoren werden auch als "Biopotentialsensoren" bezeichnet. Die Größe und Form dieser Elektrode kann je nach Anwendungsszenario sehr unterschiedlich ausgebildet sein. Beispielsweise kann die Elektrode die Form mehrerer konzentrischer Ringe aufweisen, oder die Form mehrerer parallel zueinander angeordnete Stäbe. Vorzugsweise ist der elektrische Sensor als Array mehrerer Elektroden ausgebildet, sodass Hirnaktivitätssignale von mehreren Bereichen des Kopfes unterschiedliche Bereiche der Elektrode in unterschiedlicher Stärke erreichen. Dies erlaubt es dem elektrischen Sensor, zusätzliche Informationen bezüglich der Hirnregion, die ein Hirnaktivitätssignale erzeugt, zu erfassen. Beispielsweise kann dieser Array 5-6 Einzelelektroden beinhalten (eine im Vergleich zu 30-60 Kontaktelektroden bei einem konventionellen EEG immer noch geringe Zahl). Durch die Verwendung von elektrischen Sensoren mit mehreren Einzelelektroden bzw. einer räumlichen Ausdehnung von vorzugsweise mehr als 5 cm in zwei Dimensionen kann auch die Abhängigkeit der Signalstärke vom Abstand und Ausrichtung des Kopfes der zu authentifizierenden Person erfasst werden. Dies ermöglicht es zum Beispiel festzustellen, ob die zu authentifizierenden Person auch tatsächlich auf die Anzeigevorrichtung blickt, auf welcher aktuell die Fremdbilder bzw. Bekanntbilder angezeigt werden (und nicht zum Beispiel auf ein heimlich mitgebrachtes Selbst-Foto, dessen Anblick es einem Maskenträger ermöglichen würde, Hirnaktivitätssignale, die typisch für die Betrachtung eines Bekanntbilds sind, zu erzeugen, obwohl das offiziell gezeigte Bekanntbild (Spiegelbild) nur die Maske zeigt.

Die Doktorarbeit "Kapazitive Elektroden zur Messung bioelektrischer Signale" von Martin Oehler, 2009, Institut für Elektrische Messtechnik und Grundlagen der Elektrotechnik, und dort insbesondere das Kapitel 3.2, beschreibt weitere Beispiele für elektrische Sensoren, die zur kontaktlosen Erfassung von Hirnstromsignalen (und Herzschlagsignalen) geeignet sind, sowie Beispiele dafür, wie die Verstärkung und der Hochpassfilter ausgebildet sein können. Ein elektrischer Sensor kann z.B. eine oder mehrere Elektroden umfassen und folgende Elemente beinhalten: eine oder mehrere Elektroden, wobei die Elektrodenfläche, die nach außen hin isoliert sein kann, das eigentliche Signal erfasst. Die Elektrode(n) ist/sind mit dem zweiten wichtigen Teil, der Elektronik, verbunden, welche das Signal hochimpedant aufnimmt und verstärkt. Das dritte Element des elektrischen Sensors ist die Abschirmung, auch "Filter" genannt, die sowohl aktiv als auch passiv ausgeführt sein kann.

Die Funktion der kapazitiven Elektrode basiert gemäß einer Ausführungsform der Erfindung auf einer extrem hochohmigen Messung des kapazitiv eingekoppelten Hirnaktivitätssignals ("Biosignals"). Die Elektrodenfläche und der Elektrodenkörper bilden einen Koppelkondensator. Die hochohmige Messung des Eingangssignals resultiert aus der Eingangscharakteristik, die sich aus dem Koppelkondensator und dem Eingangswiderstand der Schaltung ergibt. Dieser Eingang verhält sich wie ein RC-Hochpass für das eingekoppelte Signal.

Gemäß Ausführungsformen der Erfindung wird das Authentifizierungsverfahren innerhalb eines Authentifizierungssystems durchgeführt, wobei innerhalb des Authentifizierungssystems der zumindest eine KLHA-Sensor in räumlicher Nähe zum Kopf der zu authentifizierenden Person angebracht ist.

Unter der räumlichen Nähe wird hier vorzugsweise ein Abstand von weniger als 100 cm, in manchen Ausführungsformen weniger als 70cm, in manchen Ausführungsformen von weniger als 30 cm, verstanden. Diese Abstände sind insbesondere beim elektrischen Sensor vorteilhaft, beim optischen Sensor kann der die räumliche Nähe definierende Abstand auch größer sein, z.B. mehrere Meter, sofern gewährleistet ist, dass die räumliche Auflösung des optischen Sensors eine bildanalysegestützte Erkennung von Hirnaktivitätssignalen wie z.B. Pupillendurchmesseränderungen zulässt.

Die von dem elektrischen Sensor erfassten Hirnaktivitätssignale können auch als Hirnstromsignale bezeichnet werden. Sie werden direkt durch elektrochemische Aktivitäten in den Zellen des Gehirns verursacht und geben Aufschluss über bewusste und/oder unbewusste Denkprozesse und Aktivitäten des Gehirns zum Zeitpunkt der Erfassung dieser Signale durch den Sensor.

Bei den von einem elektrischen Sensor erfassten Hirnaktivitätssignalen kann es sich insbesondere um Signale im Frequenzbereich von (von 0,1 bis zu 40 Hz) handeln.

Nach Ausführungsformen ist der elektrische Sensor dazu ausgebildet, die ersten und/oder zweiten Hirnaktivitätssignale durch Messung von Ladungsänderungen bzw. Ladungsumverteilungen in der Nähe des Sensors zu erfassen. Unter der Nähe wird hier vorzugsweise ein Bereich verstanden, der durch einen Abstand von dem elektrischen Sensor von weniger als 100 cm gekennzeichnet ist. Gemäß Ausführungsformen der Erfindung umfasst die räumliche Nähe einen Bereich mit einem Abstand von 1 cm bis 100 cm von dem Sensor, in manchen Ausführungsformen von 1 cm bis 70 cm vom Sensor, in anderen Ausführungsformen von 1 cm bis 60 cm. In manchen Fällen wird der Sensor so positioniert, dass er sich in einem Abstand von 15-60 cm vom Kopf der Person entfernt befindet. Die exakte Positionierung kann vom Sensortyp abhängen und/oder davon, wie sich die Person während des zweiten Authentifizierungsverfahrens bewegen oder positionieren soll, um auf die Anzeige zu blicken, und wieviel Bewegungsfreiheit dem Kopf der Person während des Authentifizierungsprozesses eingeräumt werden soll.

Aus dem erfassten Signal können die Amplitude und/oder der zeitliche Amplitudenverlauf der ersten und/oder zweiten Hirnaktivitätssignale ermittelt werden.

Nach Ausführungsformen der Erfindung ist der elektrische Sensor dazu ausgebildet, die Amplitude der ersten und/oder zweiten Hirnaktivitätssignale und/oder die Änderung der Amplitude während eines Zeitintervalls zu erfassen.

Gemäß einer ersten, einfachen Ausführungsform umfasst die Analyse der erfassten ersten und zweiten Hirnaktivitätssignale eine Feststellung, ob die Amplitude der ersten Hirnaktivitätssignale, also der vom elektrischen Sensor gemessenen Höhe der Ladungsänderungen und/oder Ladungsumverteilungen in der Nähe des Sensors, beim Betrachten eines Bekanntbildes, höher ist als die Amplitude der zweiten Hirnaktivitätssignale, also der vom elektrischen Sensor beim Betrachten eines Fremdbildes gemessenen Höhe der Ladungsänderungen und/oder Ladungsumverteilungen in der Nähe des Sensors. Ist dies der Fall, entspricht das gemessene Muster der ersten und zweiten Hirnaktivitätssignale dem Erwarteten und die Person wird als erfolgreich authentifiziert betrachtet.

Gemäß einer zweiten, komplexeren Ausführungsform umfasst die Analyse der erfassten Hirnaktivitätssignale einen Vergleich von Amplitudenverlaufsprofilen der ersten Hirnaktivitätssignale und von Amplitudenverlaufsprofilen der zweiten Hirnaktivitätssignale untereinander und/oder mit Amplitudenverlaufsprofilen von ersten und zweiten Referenz-Hirnaktivitätssignalen, um festzustellen, ob sich die ersten Hirnaktivitätssignale signifikant von den zweiten Hirnaktivitätssignalen unterscheiden.

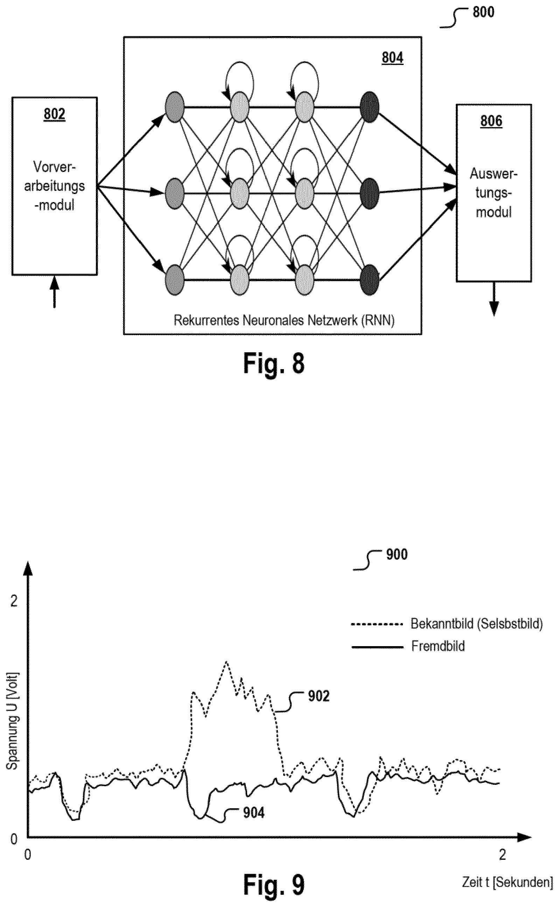

Nach Ausführungsformen sind die ersten Hirnaktivitätssignale identisch oder ähnlich zu den Hirnaktivitätssignalen gemäß Kurve 902 in Figur 9.

Der Ausdruck "identisch oder ähnlich" kann insbesondere bedeuten, "identisch im Rahmen der jeweiligen Messungenauigkeit des Sensors oder so ähnlich, dass ein vordefiniertes Mindestähnlichkeitskriterium erfüllt ist". Bei dem vordefinierten Mindestähnlichkeitskriterium kann es sich z.B. einen vordefinierten Mindestwert für ein Ähnlichkeitsmaß (Kurvenabstand, Integral der Differenzfläche der Hirnaktivitätssignalprofile über einen Zeitraum, etc.) handeln.

Zusätzlich oder alternativ dazu sind die zweiten Hirnaktivitätssignale identisch oder ähnlich zu den Hirnaktivitätssignalen gemäß Kurve 904 in Figur 9. Das bedeutet, dass bei vielen Menschen innerhalb eines Zeitraums von ca. 100 bis 2000 ms, insbesondere von ca. 300 - 1500 ms, insbesondere von 500 bis 1000 ms nach dem Beginn des Betrachtens eines Bekanntbildes, insbesondere eines Bildes, das das "eigene" Gesicht des Betrachters darstellt ("Selbstbild"), eine deutliche Verstärkung des Signals (Amplitudenzuwachs um ca. 100% relativ zum Ausgangszustand) beobachtbar ist, wohingegen beim Betrachten eines Fremdbildes dieser Anstieg ausbleibt oder sich die Signalstärke im Zeitraum von ca. 500-700 ms nach dem Beginn des Betrachtens des Fremdbildes sogar verringert.

Nach Ausführungsformen umfasst die Analyse der erfassten Hirnaktivitätssignale einen Vergleich von Amplitude und/oder Amplitudenverlauf der ersten Hirnaktivitätssignale und zweiten Hirnaktivitätssignale untereinander und/oder mit der Amplitude und/oder dem Amplitudenverlauf ersten und zweiten Referenz-Hirnaktivitätssignale gegeneinander, um festzustellen, ob sich die ersten Hirnaktivitätssignale signifikant von den zweiten Hirnaktivitätssignalen unterscheiden.

Gemäß bevorzugten Ausführungsformen umfasst die Analyse lediglich einen Vergleich der Amplituden und zeitlichen Amplitudenprofile der ersten Hirnaktivitätssignalen und zweiten Hirnaktivitätssignalen jeweils untereinander und mit dem anderen Hirnaktivitätssignaltyp unter Berücksichtigung der bekannten zeitlichen Abfolge der Anzeige der ein oder mehreren Bekanntbilder und der ein oder mehreren Fremdbilder. Hirnaktivitätssignale, die während der Anzeige eines Bekanntbilds erfasst werden, werden als erste Hirnaktivitätssignale erfasst. Hirnaktivitätssignale, die während der Anzeige eines Fremdbilds erfasst werden, werden als zweite Hirnaktivitätssignale erfasst. Wenn die Person wirklich die Person ist, die auf dem angezeigten Bekanntbild abgebildet ist, ist also zu erwarten, dass alle erfassten ersten Hirnaktivitätssignale identische oder sehr ähnliche Eigenschaften (Amplitude, Amplitudenverlauf über die Zeit, ggf. weitere Eigenschaften des Signals wie z.B. Frequenz) haben. Ebenso ist zu erwarten, dass die erfassten zweiten Hirnaktivitätssignale identische oder zumindest ähnliche Eigenschaften haben, wobei sich allerdings die Eigenschaften der ersten und der zweiten Hirnaktivitätssignale signifikant unterscheiden. Trägt die zu authentifizierenden Person dagegen eine Maske oder ist das als "Bekanntbild" angezeigte Bild ein "gemorphtes" Bild, dann reagiert das Hirn der zu authentifizierenden Person so, als ob ein Fremdbild angezeigt würde. In diesem Fall haben die ersten Hirnaktivitätssignale identische o. ä. Eigenschaften wie die zweiten Hirnaktivitätssignale. In diesem Fall behandelt die Authentifizierungssoftware die Authentifizierung der einen Person als gescheitert.

Nach Ausführungsformen umfasst die Analyse der erfassten Hirnaktivitätssignale einen Vergleich von Frequenz und Amplitudenverlauf der ersten Hirnaktivitätssignale und zweiten Hirnaktivitätssignale untereinander und/oder mit ersten und zweiten Referenz-Hirnaktivitässignalen, um festzustellen, ob sich die ersten Hirnaktivitätssignale signifikant von den zweiten Hirnaktivitätssignalen unterscheiden. Beispielsweise kann die Authentifizierungssoftware dazu konfiguriert sein, einen Ähnlichkeitsscore für ein bestimmtes erstes (oder zweites) Hirnaktivitätssignal in Bezug auf ein erstes (oder zweites) Referenz-Hirnaktivitätssignal berechnen, z.B. anhand geometrischer Abstände entsprechender Hirnaktivitätssignalkurven in einem Kurvendiagramm. Ist der berechnete Ähnlichkeitsscore, der z.B. als inverser Abstandsscore der verglichenen Signalprofile oder Signale berechnet werden kann, unterhalb eines vordefinierten Mindestschwellenwerts gelten die verglichenen Signale als signifikant unterschiedlich.

### Optischer Sensor

Nach Ausführungsformen der Erfindung ist der KLHA-Sensor ein optischer Sensor, insbesondere eine IR-Kamera oder IR-Videokamera oder eine Kamera oder Videokamera für Licht im sichtbaren Wellenlängenbereich.

Nach Ausführungsformen der Erfindung ist der KLHA-Sensor dazu ausgebildet, Bilder oder Bildsequenzen des Gesichts oder von Teilen des Gesichts der einen Person zu erfassen, die die ersten und/oder zweiten Hirnaktivitätssignale enthalten, wobei die Analyse der ersten und zweiten Hirnaktivitätssignale durch die Authentifizierungssoftware eine automatische Bildanalyse der Bilder oder Bildsequenzen umfasst. Die Bildanalyse umfasst eine rechnerische Bestimmung eines Pupillendurchmessers der einen Person. Zusätzlich oder alternativ dazu umfasst die Bildanalyse eine rechnerische Bestimmung eines Pupillendurchmesseränderungsprofils (also eine Änderung des Durchmessers über die Zeit) der einen Person.

Falls das erste Authentifizierungsverfahren eine Kamera zur Erfassung biometrischer Merkmale verwendet, kann beispielsweise dieselbe oder eine andere Kamera auch zur Erfassung der Hirnaktivitätssignale auf optischem Wege verwendet werden.

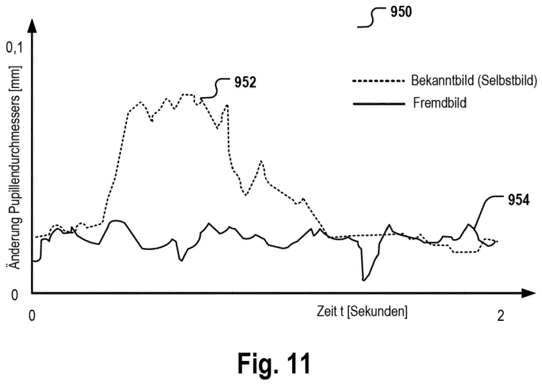

Nach Ausführungsformen der Erfindung sind die ersten Hirnaktivitätssignale identisch oder ähnlich zu den Hirnaktivitätssignalen gemäß Kurve 952 in Figur 11. Zusätzlich oder alternativ dazu sind die zweiten Hirnaktivitätssignale identisch oder ähnlich sind zu den Hirnaktivitätssignalen gemäß Kurve 954 in Figur 11.

Nach Ausführungsformen der Erfindung umfasst das Verfahren Machine-Learning Verfahrensschritte, z.B. um im Zuge einer Trainingsphase einen personenspezifischen oder generischen Klassifikator zu erzeugen, der dazu ausgebildet ist, die Unterschiede der ersten und/oder zweiten Hirnaktivitätssignale zu lernen (z.B. während der Registrierung eines Nutzers), und/oder im Zuge einer Test- bzw. Anwendungsphase des trainierten Klassifikators zur Unterscheidung der ersten und/oder zweiten Hirnaktivitätssignale während eines Authentifizierungsprozesses.

Nach Ausführungsformen der Erfindung umfasst die Analyse der erfassten Hirnaktivitätssignale:
- Bildverarbeitung zur Erkennung eines Pupillendurchmessers; und/oder
- Korrelation von Eigenschaften der ersten und zweiten Hirnaktivitätssignale mit den Zeiten, an welchen Bekanntbilder und Fremdbilder angezeigt wurden.

Nach Ausführungsformen ist der KLHA-Sensor dazu ausgebildet, Bilder und/oder Bildsequenzen des Gesichts oder von Teilen des Gesichts der einen Person zu erfassen. Aus den Bildern, die beim Betrachten von Fremdbildern und Bekanntbildern erfasst wurden, können absolute Pupillendurchmesser und damit auch Unterschiede im Pupillendurchmesser bei der Betrachtung von Bildern unterschiedlichen Typs ermittelt werden. Aus den Bildsequenzen wird der zeitliche Verlauf des Pupillendurchmessers beim Betrachten eines bestimmten Bildes ermittelt. Aus dem ermittelten Signal können die Amplitude (Pupillendurchmesser) und/oder der zeitliche Amplitudenverlauf der ersten und/oder zweiten Hirnaktivitätssignale ermittelt werden. Ein "Amplitudenverlauf" oder "Amplitudenverlauf über die Zeit" wird hier auch als "Profil" oder "Amplitudenprofil" bezeichnet. Die Amplitude kann sich z.B. auf Hirnaktivitätssignalintensitäten beziehen, die in den als Rohdaten erfassten Signalen vorkommen. In anderen Ausführungsformen bezieht sich die Amplitude auf Signale, die von den Rohdaten rechnerisch abgeleitet wurden, z.B. auf Amplituden von Signalen innerhalb eines bestimmten Frequenzbereichs. Die Beschränkung auf ausgewählte Frequenzbereiche kann den Vorteil haben, dass Störsignale reduziert werden.

Gemäß Ausführungsformen umfasst das Verfahren eine automatische Bildanalyse der Bilder oder Bildsequenzen, die als die Hirnaktivitätssignale erfasst wurden, um einen Pupillendurchmesser der einen in dem Bild abgebildeten Person zu bestimmen. Die Bestimmung des Pupillendurchmessers kann durch die Authentifizierungssoftware oder durch ein anderes Softwareprogramm durchgeführt werden.

Gemäß einer ersten, einfachen Ausführungsform umfasst die Analyse der erfassten ersten und zweiten Hirnaktivitätssignale eine Feststellung, ob die Amplitude der ersten Hirnaktivitätssignale, also der Pupillendurchmesser beim Betrachten eines Bekanntbildes, höher ist als die Amplitude der zweiten Hirnaktivitätssignale, also der Pupillendurchmesser beim Betrachten eines Fremdbildes. Ist dies der Fall, entspricht das gemessene Muster der ersten und zweiten Hirnaktivitätssignale dem Erwarteten und die Person wird als erfolgreich authentifiziert betrachtet.

Gemäß einer zweiten, komplexeren Ausführungsform umfasst die Analyse der erfassten Hirnaktivitätssignale einen Vergleich von Amplitudenverlaufsprofilen der ersten Hirnaktivitätssignale und von Amplitudenverlaufsprofilen der zweiten Hirnaktivitätssignale untereinander und/oder mit Amplitudenverlaufsprofilen von ersten und zweiten Referenz-Hirnaktivitätssignalen, um festzustellen, ob sich die ersten Hirnaktivitätssignale signifikant von den zweiten Hirnaktivitätssignalen unterscheiden. Nach Ausführungsformen sind die ersten Hirnaktivitätssignale identisch oder ähnlich zu den Hirnaktivitätssignalen gemäß Kurve 952 in Figur 11. Nach Ausführungsformen sind die die zweiten Hirnaktivitätssignale identisch oder ähnlich zu den Hirnaktivitätssignalen gemäß Kurve 954 in Figur 11. Das bedeutet, dass bei vielen Menschen innerhalb der ersten 1500 ms nach dem Beginn des Betrachtens eines Bekanntbildes, z.B. eines "Selbstbildes", eine deutliche Vergrößerung des Pupillendurchmessers (um ca. 0,05 mm - 0,50 mm) beobachtbar ist, die beim Betrachten eines Fremdbildes mit dem gleichen Motivtyp ausbleibt.

Nach Ausführungsformen umfasst die Analyse der erfassten Hirnaktivitätssignale die Durchführung eines Bildverarbeitungsprozesses. Dabei werden unter Zuhilfenahme von Verfahren des Machine-Learnings und Deep-Learning-Algorithmen trainiert, die selbstständig Unterschiede bezüglich des Pupillendurchmessers während der Betrachtung des Fremd- und Bekanntbilder detektieren und so eine Klassifizierung vornehmen können.

Beispielsweise kann es sich bei dem optischen Sensor um eine Kamera oder Videokamera handeln, die im Infrarotbereich oder im Wellenlängenbereich des sichtbaren Lichtes ein oder mehrere Bilder des Gesichts oder von Teilen des Gesichts der zu authentifizierenden Person aufnimmt und an die Authentifizierungssoftware weiterleitet. Die Authentifizierungssoftware führt eine Bildanalyse durch, beispielsweise um einzelne Komponenten der Augenpartie des Gesichtes zu erkennen, insbesondere Iris und/oder Pupille von einem oder beiden Augen der zu authentifizierenden Person. Durch Analyse der Position und/oder Größe der Pupille einer Person in mehreren über die Zeit aufgenommenen Bildern kann außerdem ein Pupillendurchmesseränderungsprofil über ein Zeitintervall (von z.B. 0-4 Sekunden Dauer nach dem Beginn des Anzeigens eines Bekanntbilds oder Fremdbilds, insb. von 0-4 Sekunden Dauer) von einem oder beiden Augen dieser Person durch Bildanalyse ermittelt werden.

Die Anmelderin hat festgestellt, dass der Pupillendurchmesser und die Änderung des Pupillendurchmessers über die Zeit Ausdruck von Hirnaktivitäten sind, und insbesondere auch signifikant unterschiedlich sind in Abhängigkeit davon, ob einer Person ein Bekanntbild oder ein Fremdbild angezeigt wird. Somit ist es möglich, durch automatisches Erfassen und Analysieren von Bildern der Augenpartie von Personen während eines Zeitraums, innerhalb welchem der Person ein oder mehrere Bekanntbilder und ein oder mehrere Fremdbilder gezeigt werden, zu erkennen, ob sich der oder die Pupillendurchmesser, die während des Betrachtens eines Bekanntbildes gemessen wurden, signifikant von dem oder den Pupillendurchmesser(n), die während des Betrachtens eines Fremdbilds gemessen werden, unterscheiden, wohingegen die entsprechenden Signale, die beim Betrachtens des gleichen Bildtyps (Bekanntbild oder Fremdbild) gemessen werden, identisch oder sehr ähnlich sind. Somit eignen sich die besagten biometrische Merkmale "Pupillendurchmesser" bzw. "Pupillendurchmesseränderungsprofil" dazu, die Person auf Basis des Authentifizierungsverfahrens gemäß Ausführungsformen der Erfindung zu authentifizieren.

Die oben genannten optisch erfassten Hirnaktivitätssignale geben zumindest indirekt Aufschluss über Hirnaktivitäten, denn auch wenn bei diesen Merkmalen die Hirnaktivität nicht direkt in Form von Hirnstromsignalen gemessen wird, so werden doch physiologische Parameter erfasst, die auf direkte und bewusst nicht steuerbare Weise von den Hirnaktivitäten abhängen, die durch das Betrachten von Bekanntbildern und Fremdbildern ausgelöst werden. Hierbei ist hervorzuheben, dass nicht jegliche Art physiologischer Parameter zur Authentifizierung der Person geeignet ist, denn wenn diese physiologische Parameter nicht einerseits zumindest bei der einen Person bei Bildern des gleichen Typs (entweder Bekanntbild oder Fremdbild) reproduzierbar identische oder sehr ähnliche Eigenschaften aufweist und gleichzeitig die Signale beim Betrachten von Bildern unterschiedlichen Typs signifikant unterschiedliche Eigenschaften aufweisen, sind sie nicht geeignet, als Basis für das Authentifizierungsverfahren Verwendung zu finden.

Die Anmelderin hat beobachtet, dass eine deutliche Vergrößerung des Pupillendurchmessers innerhalb der ersten 2000 ms, vorzugsweise innerhalb der ersten 1000 ms nach dem Anzeigen eines Bildes (z.B. eine Vergrößerung von mindestens 8%, vorzugsweise mindestens 15%), ein Indiz dafür ist, dass der Betrachter ein Bekanntbild, insbesondere ein Selbstbild, sieht. Bleibt diese Pupillenweitung aus, handelt es sich um ein Fremdbild.

Gemäß einer Ausführungsform wird eine Bestimmung der Augenlidöffnung mehrfach pro angezeigtem Bild gemessen und dadurch ein oder mehrere Blinzelbewegungen der einen Person erfasst. Vorzugsweise dienen die erfassten Blinzelbewegungen dazu, ein erfasstes Hirnaktivitätssignal zu korrigieren. Dies kann vorteilhaft sein, da das Blinzeln in manchen Fällen auch eine Störung anderer Hirnaktivitätssignale wie z.B. der von einem elektrischen Sensor erfassten Hirnstromsignale bewirkt. Durch eine entsprechende Korrektur wird die Akkuratheit der Vorhersage erhöht.

Gemäß einer Ausführungsform werden die Fremdbilder so gewählt oder deren Helligkeit so normalisiert, dass diese einen ähnlichen Helligkeitsgrad aufweisen wie das eine Bekanntbild, oder wie ein mittlerer Helligkeitsgrad mehrerer Bekanntbilder oder wie der Helligkeitsgrad des zuletzt gezeigten Bekanntbildes. Beispielswiese kann ein ähnlicher Helligkeitsgrad bedeuten, dass der Mittelwert aller Pixelintensitäten des Fremdbildes nicht mehr als 25%, vorzugweise nicht mehr als 10% unter oder über dem Mittelwert aller Pixelintensitäten des Bekanntbildes oder der vorgenannten Bekanntbilder liegt.

Dies kann den Vorteil haben, dass Änderungen des Pupillendurchmessers, die auf die Adaptation an unterschiedliche Helligkeitsverhältnisse und nicht auf die Fremdbild/Bekanntbild-Rezeption zurückzuführen sind und die als Störsignale zu betrachten sind, reduziert oder vermieden werden.

Nach einer Ausführungsform wird die Bildanalyse von einem neuronalen Netz oder einer anderen Machine-Learning- Software (zum Beispiel Supportvektormaschine) durchgeführt. Die Machine-Learning- Software kann beispielsweise in einer Trainingsphase gelernt haben, bestimmte Gesichtsregionen bzw. Augenregionen wie zum Beispiel Iris und/oder Pupille automatisch zu erkennen und optional auch Größe und/oder Position von Pupille (und/oder ggf. für Signalkorrekturzwecke auch der Augenlidöffnung) zu bestimmen. Das Ergebnis der Bildanalyse besteht entsprechend aus ein oder mehreren numerischen Werten, die die Pupillengröße und/oder ein Pupillengrößenänderungsprofil über ein Zeitintervall spezifizieren. Zumindest ein oder mehrere dieser numerischen Werte werden als Eingabe an ein weiteres Softwaremodul der Authentifizierungssoftware weitergegeben. Das weitere Modul ist dazu ausgebildet, derartige numerische Werte, die jeweils beim Betrachten von Bekanntbildern erfasst wurden, als erste Hirnaktivitätssignale zu verarbeiten, und entsprechende numerische Werte, die jeweils beim Betrachten von Fremdbildern erfasst wurden, als zweite Hirnaktivitätssignale zu verarbeiten. Im nächsten Schritt ermittelt die Authentifizierungssoftware die Ähnlichkeit der ersten Hirnaktivitätssignale untereinander (also die Ähnlichkeit mehrerer erster Hirnaktivitätssignale die beim Betrachten mehrerer Bekanntbilder erfasst werden untereinander), die Ähnlichkeit der zweiten Hirnaktivitätssignale untereinander (also die Ähnlichkeit mehrerer zweiten Hirnaktivitätssignale die beim Betrachten mehrerer Fremdbilder erfasst werden untereinander) sowie die Ähnlichkeit der ersten Hirnaktivitätssignale und der zweiten Hirnaktivitätssignale. Für den letztgenannten Vergleich können verschiedene Verfahren verwendet werden, beispielsweise Clusteranalysen oder ein Mehrschrittverfahren, in welchem ein erster Mittelwert aus allen ersten Hirnaktivitätssignalen berechnet wird, ein zweiter Mittelwert aus allen zweiten Hirnaktivitätssignalen berechnet wird, und sodann die Ähnlichkeit des ersten und zweiten Mittelwerts berechnet wird. Durch Vergleich der berechneten Ähnlichkeiten bzw. Abweichungen mit vordefinierten Grenzwerten kann die Authentifizierungssoftware automatisch feststellen, ob die ersten und zweiten Hirnaktivitätssignale signifikant voneinander unterschieden sind.

Nach Ausführungsformen umfasst die Analyse der erfassten Hirnaktivitätssignale die Ausführung eines Machine-Learning-Programms.

Beispielsweise kann ein "supervised learning" Ansatz dazu verwendet werden, zeitliche Profile der Signalamplituden (z.B. Spannung eines elektrischen Feldes im Falle von elektrischen Sensoren, Pupillendurchmesser im Falle von optischen Sensoren), die von einer Person je beim Betrachten von Fremdbildern oder Bekanntbildern erzeugt werden, zu analysieren und zu lernen, auf Basis dieser Verläufe den Typ des betrachteten Bildes automatisch zu erkennen bzw. vorherzusagen. Beispielsweise kann es sich bei dem Machine-Learning-Programm um ein neuronales Netzwerk oder eine Supportvektormaschine handeln. Insbesondere kann das neuronale Netz als ein rekurrentes neuronales Netzwerk ausgebildet sein. Das neuronale Netz lernt während des Trainings auf Grundlage von zeitlichen Verläufen der Signalamplitude (z.B. Durchmesser Pupille in Micrometer), wie die Signalamplituden bzw. Signalamplitudenprofile bei der Betrachtung von Bekanntbildern und Fremdbildern jeweils aussehen und/oder wie diese sich bei der Betrachtung von Bekanntbildern und Fremdbildern unterscheiden.

Gemäß Ausführungsformen wird das vom elektrischen Sensor erfasste Rohsignal als erstes oder zweites Hirnaktivitätssignal verwendet und ausgewertet. Bei dem Rohsignal kann es sich um die Gesamtheit der innerhalb eines oder mehrerer Frequenzbereiche erfassten Hirnaktivitätssignale handeln oder um eine oder mehrere diskrete Frequenzen. Gemäß anderer Ausführungsformen wird das vom elektrischen Sensor erfasste Rohsignal zunächst vorverarbeitet, z.B. normalisiert, entrauscht, und/oder gefiltert, um die Signalqualität und/oder Vergleichbarkeit des Signals zu erhöhen, und dieses vorverarbeitete Hirnaktivitätssignal wird als erstes oder zweites Hirnaktivitätssignal verwendet und ausgewertet.

Eine über die Bestimmung des Pupillendurchmessers hinausgehende Verarbeitung von Bilddaten ist nicht notwendig, da keine Objektklassifizierung stattfindet. Das Training kann daher auf Grundlage von annotierten Daten (z.B. Pupillendurchmessern oder Pupillendurchmesseränderungsprofilen, die mit "bei Bekanntbildbetrachtung" oder "bei Fremdbildbetrachtung" annotiert sind, performant durchgeführt werden.

Die für das Training verwendeten annotierten Trainingsdaten können z.B. anhand eines Protokolls aufgezeichnet und annotiert werden, damit das Modell trainiert und verifiziert werden kann.

Nach Ausführungsformen umfasst die Analyse der erfassten Hirnaktivitätssignale die Erkennung einer Korrelation von signifikant unterschiedlichen Hirnaktivitätssignalen zu Bildtypwechselzeitpunkten, wobei an einem Bildtypwechselzeitpunkt ein in der Anzeige gezeigtes Bekanntbild durch ein Fremdbild ersetzt wird oder umgekehrt.

Nach Ausführungsformen beinhaltet das Verfahren einen Schritt der Freigabe einer Funktion eines Authentifizierungssystem an die Person nur dann, wenn diese sich sowohl im ersten als auch im zweiten Authentifizierungsverfahren erfolgreich authentifiziert hat.

### Authentifizierungssystem

In einem weiteren Aspekt betrifft die Erfindung ein Authentifizierungssystem zur Authentifizierung einer Person als Vertrauensperson. Das Authentifizierungssystem umfasst eine Schnittstelle zum Empfang von Daten der Person; eine Authentifizierungssoftware; eine Anzeige; einen kontaktlosen Sensor zur Sensierung von Hirnaktivitäten - im Folgenden als KLHA-Sensor bezeichnet. Das Authentifizierungssystem ist konfiguriert zum:
- Empfang der Daten der Person durch die Authentifizierungssoftware über die Schnittstelle; bei den empfangenen Daten kann es sich z.B. um biometrische oder andere personenbezogene Daten dieser Person oder um personengruppenbezogene Daten einer Gruppe, der diese Person angehört, handeln, insbesondere Gesichtsbild, Fingerabdruck, Bewegungsprofil, Irisbild, ID-Tokendaten, etc.;
- Auswertung der empfangenen Daten durch die Authentifizierungssoftware, um die Person mittels eines ersten Authentifizierungsverfahrens als Vertrauensperson zu authentifizieren;
- Ermittlung von einem oder mehreren Bekanntbildern und einem oder mehreren Fremdbildern durch die Authentifizierungssoftware, wobei ein Bekanntbild ein digitales Bild ist mit einem Bildmotiv, das der Person bekannt ist, sofern es sich bei der Person um die Vertrauensperson handelt, wobei ein Fremdbild ein digitales Bild ist mit einem Bildmotiv, das der Person nicht bekannt ist oder signifikant weniger bekannt ist als ein Bekanntbild; Beispielsweise kann davon ausgegangen werden, dass einem Betrüger, der eine Maske trägt, eine Gesichtsbildaufnahme dieser Maske deutlich weniger bekannt ist als ein Bild seines wahren Gesichts; Der Bekanntheitsgrad einer Maske ist für deren Träger also deutlich geringer als der des eigenen tatsächlichen Gesichts;
- Anzeige mehrerer Bilder auf der Anzeige, wobei die angezeigten Bilder zumindest eines der ermittelten Bekanntbilder und zumindest eines der ermittelten Fremdbilder enthalten;
- während die mehreren Bilder der Person angezeigt werden, Erfassung von ersten und zweiten Hirnaktivitätssignalen, die in der einen Person durch die Betrachtung der Bilder jeweils ausgelöst werden, durch den KLHA-Sensor, wobei erste Hirnaktivitätssignale erfasst werden, während der Person ein Bekanntbild angezeigt wird, und wobei zweite Hirnaktivitätssignale erfasst werden, während der Person ein Fremdbild angezeigt wird;
- Durchführung eines zweiten Authentifizierungsverfahrens durch die Authentifizierungssoftware, um die Person als die Vertrauensperson zu authentifizieren, wobei das zweite Authentifizierungsverfahren eine Analyse der erfassten ersten und zweiten Hirnaktivitätssignale umfasst;
- Behandlung der Person durch die Authentifizierungssoftware als authentifiziert als die Vertrauensperson nur dann, wenn diese sich sowohl im ersten als auch im zweiten Authentifizierungsverfahren erfolgreich authentifiziert hat.

Nach Ausführungsformen beinhaltet das Authentifizierungssystem eine Funktion, die für authentifizierte Vertrauenspersonen freigegeben wird. Das Authentifizierungssystem ist konfiguriert zur Freigabe der Funktion für die Person durch die Authentifizierungssoftware nur dann, wenn diese sich sowohl im ersten als auch im zweiten Authentifizierungsverfahren erfolgreich als die Vertrauensperson authentifiziert hat.

Beispielsweise kann es sich bei dem Authentifizierungssystem um ein Zugriffskontrollsystem auf eine Softwarefunktion oder Hardwarefunktion handeln. Eine Person kann z.B. eine sicherheitskritische Hardware oder Softwarefunktion, die z.B. einen von einem Roboter oder Roboterarm ausgeführten Arbeitsschritt in einem industriellen Herstellungsprozess steuert, nur nach erfolgreicher Authentifizierung der Person und Freigabe dieser Steuerfunktion durch die Authentifizierungssystem bedienen und zur Ausführung dieses Arbeitsschrittes veranlassen.

Gemäß eines weiteren Beispiels kann es sich bei dem Authentifizierungssystem um ein Zutrittskontrollsystem oder Zufahrtskontrollsystem. Die Funktion kann darin bestehen, automatisch eine Tür, ein Tor, eine Schranke oder sonstige Zutrittsbeschränkung oder Zufahrtsbeschränkung in einen anderen zustand zu versetzen, der es der erfolgreich authentifizierten Person erlaubt, einen bestimmten Raum zu betreten oder zu befahren.

Nach Ausführungsformen werden die Bilder oder zumindest die Bekanntbilder nur dann ermittelt, wenn die Person sich im ersten Authentifizierungsverfahren erfolgreich authentifiziert hat.

Nach Ausführungsformen ist der KLHA-Sensor ein elektrischer Sensor zur Erfassung eines Hirnsignals ohne direkten Kontakt des Kopfes mit dem elektrischen Sensor.

Nach anderen Ausführungsformen ist der KLHA-Sensor ein optischer Sensor.

Nach Ausführungsformen ist die Schnittstelle eine Kamera, die positioniert ist zum Erfassen eines Gesichtsbildes oder Irisbildes der Person.

Nach anderen Ausführungsformen ist die Schnittstelle ein Fingerabdrucksensor.

Nach weiteren Ausführungsformen ist die Schnittstelle ein Lesegerät zum Lesen von Daten aus einem ID-Token der Person.

Nach manchen Ausführungsformen umfasst das Authentifizierungssystem ein Terminal. Die Anzeigevorrichtung ist Bestandteil des Terminals. Das Terminal insbesondere ein Flughafenterminal, ein Grenzkontrollterminal, ein Terminal zur Kontrolle des Zutritts zu einem geschützten geographischen Bereich ist.

Nach Ausführungsformen ist das Authentifizierungssystem dazu konfiguriert, ein Verfahren gemäß einer der hier beschriebenen Ausführungsformen und Beispiele auszuführen.

Nach Ausführungsformen ist der KLHA-Sensor ein elektrischer Sensor zur Erfassung eines Hirnsignals ohne direkten Kontakt des Kopfes mit dem elektrischen Sensor. Insbesondere kann der elektrische Sensor als isolierte kapazitive Elektrode ausgebildet sein, wobei der elektrische Sensor vorzugsweise einen Signalverstärker umfasst.

Nach anderen Ausführungsformen ist der KLHA-Sensor ein optischer Sensor, z.B. eine IR-Kamera oder IR-Videokamera.

Nach Ausführungsformen umfasst das Authentifizierungssystem ein Terminal, das die Anzeige beinhaltet. Bei dem Terminal kann es sich insbesondere um ein Flughafenterminal, ein Grenzkontrollterminal, und/oder ein Terminal zur Kontrolle des Zutritts zu einem geschützten geographischen Bereich handeln.

Gemäß einer Ausführungsform wird, z.B. im Zuge der Registrierung einer Person bei der authentifizierenden Instanz, durch Messung von Hirnaktivitätssignalen während der Betrachtung mehrerer Bekanntbilder und Fremdbilder ein Trainingsdatensatz erzeugt und ein personen-individueller Klassifikator auf diesen Bildern trainiert. Der trainierte Klassifikator ist in der Lage, Hirnaktivitätssignale speziell dieser Person in solche zu klassifizieren, die das Resultat der Betrachtung eines Bekanntbildes sind und in solche, die das Resultat einer Betrachtung eines Fremdbildes sind. Der personenspezifisch trainierte Klassifikator kann z.B. als Bestandteil des Nutzerprofils dieser Person gespeichert werden.

In einer alternativen Ausführungsform wird ein vereinfachter Registrierungsprozess (Enrollment) und ein vereinfachter Authentisierungsprozess durchgeführt. In dieser Ausführungsform wird nicht ein individuell auf den Hirnaktivitätssignalen einer bestimmten Person trainierter Klassifikator verwendet, um festzustellen, ob die Eigenschaften der erfassten Hirnaktivitätssignale zu den Eigenschaften der Hirnaktivitätssignale passen, wie diese angesichts der Sequenz an angezeigten Bekannt- und Fremdbildern zu erwarten wären. Vielmehr wird ein generischer Klassifikator, der nicht individuell für eine bestimmte Person angelernt wurde, sondern auf der Basis von Hirnaktivitätssignalen einer Vielzahl von Personen erstellt wurde, verwendet. In dem Fall müsste während der Registrierung lediglich ein verifiziertes Bild, z.B. das elektronische Passbild vom neuen Personalausweis, an das System übermittelt werden.

Mehrere Ausführungsformen der Erfindung wurden unter Bezugnahme auf eine Behörde als Beispiel für eine authentifizierende Instanz beschrieben. Anstelle einer Behörde kann jedoch auch eine private Organisation, z.B. ein Unternehmen, ein Verein oder eine sonstige Entität, gegenüber welcher sich ein Nutzer authentifizieren möchte, fungieren.

In einer alternativen Ausführungsform wird die Analyse ausschließlich auf Basis der ersten und zweiten Hirnaktivitätssignale und auf Basis der Anzeigesequenz der Bekanntbilder und Fremdbilder und optional zusätzlich auf Basis typisierter (nicht personen-individuell erhobener) Referenzdaten durchgeführt. Dieses Verfahren kann vorteilhaft sein, weil das Verfahren keine vorausgehende Speicherung von sensiblen, biometrischen, personenbezogenen Daten beansprucht. Folglich ist das Verfahren auch in Situationen einsetzbar in denen eine Speicherung personen-bezogener, biometrischer Daten unerwünscht ist. Außerdem wird hierdurch die Privatsphäre der sich authentifizierenden Personen geschützt. Gegenüber anderen biometrischen Authentifizierungsverfahren, die aus dem Stand der Technik bekannt sind, ist das Verfahren zudem noch sicherer gegenüber einem Angreifer, denn biometrische Daten, die nicht gespeichert werden müssen, können nicht gestohlen werden.

In einer alternativen Ausführungsform umfasst das Verfahren außerdem die Löschung der empfangenen Hirnaktivitätssignale nach dem zweiten Authentifizierungsvorgang. Beispielsweise kann die Löschung sämtliche ersten und zweiten Hirnaktivitätssignale und ggf. von diesen erstellten Kopien umfassen und z.B. von der Authentifizierungssoftware durchgeführt werden. Diese Ausführungsform der Erfindung kann vorteilhaft sein, weil dadurch die Privatsphäre der zu authentifizierenden Person geschützt wird. Hierdurch kann das Verfahren auch in Situationen eingesetzt werden bei denen eine Speicherung personenbezogener, biometrischer Daten unerwünscht ist. Außerdem ist das Verfahren gegenüber anderen biometrischen Authentifizierungsverfahren, die aus dem Stand der Technik bekannt sind, noch sicherer gegenüber einem Angreifer, denn biometrische Daten, die nicht gespeichert werden, können nicht gestohlen werden.

Unter einer **"Anzeigevorrichtung"** wird hier ein Gerät verstanden, das dazu ausgebildet ist, zeitlich veränderlichen Informationen wie Bilder oder Zeichen anzuzeigen. Insbesondere kann es sich bei der Anzeigevorrichtung um einen Bildschirm oder sonstige Form einer elektrisch angesteuerten Anzeige handeln. Es kann sich aber auch um eine Kombination eines Projektors mit einer Projektionsfläche handeln. Die Anzeigevorrichtung kann dabei ein eigenständiges Gerät oder Teil eines Gerätes sein, z.B. Teil eines Terminals.

Unter einem "**Sensor**", auch als Detektor oder (Mess-)Fühler bezeichnet, ist ein technisches Bauteil oder Gerät, das bestimmte physikalische und/oder chemische Eigenschaften (insbesondere Impedanz, elektrische Felder, elektromagnetische Felder, optische Signale, Ladungsänderungen und/oder Ladungsumverteilungen in der Umgebung, etc.) qualitativ oder quantitativ erfassen kann. Diese Größen werden mittels physikalischer oder chemischer Effekte erfasst und vom Sensor in ein weiterverarbeitbares elektrisches Signal umgeformt.

Unter einem **"optischen Sensor"** wird hier ein Sensor bezeichnet, welcher dazu ausgebildet ist, ein oder mehrere Bilder eines physischen Objekts, z.B. eines Gesichts oder Gesichtsbereichs, zu erfassen. Die Bilder können insbesondere digitale Bilder sein. Der optische Sensor kann z.B. eine Kamera oder eine Videokamera sein. Der optische Sensor kann z.B. dazu ausgebildet sein, selektiv Licht eines bestimmten Wellenlängenbereichs zu erfassen, z.B. Infrarotlicht (IR-Licht) oder Licht im für das menschliche Auge sichtbaren Wellenlängenbereich (Weißlicht, Tageslicht). Die erfassten Bilder werden chemisch oder elektronisch aufgezeichnet. Vorzugsweise handelt es sich bei den erfassten Bildern um digitale Bilder, die elektronisch gespeichert und/oder direkt an eine Authentifizierungssoftware bereitgestellt werden.

Unter einem **"elektrischen Sensor"** wird hier ein Sensor verstanden, der dazu ausgebildet ist, Ladungsänderungen und/oder Ladungsumverteilungen in seiner Umgebung zu messen, wobei die Umgebung der räumliche Sensitivitätsbereich des Sensors ist.

Unter einem **"kontaktlosen Hirnaktivitäts-Sensor (KLHA-Sensor)"** wird hier ein Sensor verstanden, welcher ein oder mehrere Signale empfangen kann, die von einem Menschen ausgesendet werden, ohne dass während des Empfangs der Signale ein Kontakt des Sensors zu dem Menschen besteht. Die empfangenen Signale geben direkt (Hirnstromsignale gemessen durch einen kontaktlosen elektrischen Sensor) oder indirekt (Pupillendurchmesser, Pupillendurchmesseränderungsprofil) Aufschluss über Hirnaktivitäten des Menschen, von dem die Signale empfangen werden, zum Zeitpunkt des Empfangs.

Unter einer "**Hirnaktivität**" wird hier die physiologische Aktivität einer Vielzahl von Neuronen des Gehirns eines Menschen verstanden. Die physiologische Aktivität korrespondiert in elektrischen Zustandsänderungen der Neuronen zur Informationsverarbeitung des Gehirns.

Unter einem "**Hirnaktivitätssignal**" wird hier ein von einem Menschen ausgestrahltes Signal verstanden, welches durch eine Hirnaktivität direkt oder indirekt bewirkt wird. Beispielsweise addieren sich die über den gesamten Kopf verteilten elektrischen Zustandsänderungen und Potentialänderungen der Neuronen gemäß ihrer spezifischen räumlichen Anordnung auf und lassen sich direkt als sogenannte "Hirnstromsignale" mittels eines elektrischen Sensors messen. Bei einem Hirnaktivitätssignal kann es sich aber auch um jegliches andere durch einen physiologischen Prozess in einem menschlichen Körper emittierte Signal handeln, welches von einer Hirnaktivität hervorgerufen wird und sich vorzugsweise bewusst nicht oder nur eingeschränkt kontrollieren lässt. Beispielsweise ist das Einstellen der Iris auf einen bestimmten Pupillendurchmesser abhängig von unbewussten neuronalen Verarbeitungsprozessen im menschlichen Gehirn, die z.B. davon abhängen, was eine Person gerade sieht, z.B. ein Bild ihrer selbst oder ein Bild einer anderen Person (Fremdbild).

Unter einem "**ersten Hirnaktivitätssignal**" wird hier ein Hirnaktivitätssignal verstanden, welches erfasst wird, während eine Person ein Bekanntbild betrachtet.

Unter einem "**zweiten Hirnaktivitätssignal**" wird hier wird hier ein Hirnaktivitätssignal verstanden, welches erfasst wird, während eine Person ein Fremdbild betrachtet

Unter einem "**Hirnstromsignal**" wird hier ein Hirnaktivitätssignal verstanden, welches sich ergibt durch Überlagerung einer Vielzahl von über den gesamten Kopf einer Person verteilten elektrischen Zustandsänderungen, insb. Potentialänderungen, der Neuronen gemäß ihrer spezifischen räumlichen Anordnung.

Unter einem "**Gesichtsbild**" wird hier ein Bild, insbesondere ein digitales Bild, verstanden, welches das Gesichts oder einen Gesichtsbereich einer Person abbildet.

Ein "**digitales Bild**" ist ein Datensatz, in dem Bildinhalte repräsentiert und gespeichert werden. Insbesondere kann es sich bei dem digitalen Bild um einen Datensatz handeln, in dem der Inhalt eines Bildes durch ganze Zahlen repräsentiert wird. Insbesondere kann es sich bei dem digitalen Bild um eine Rastergraphik handeln.

Unter einer "**Vertrauensperson**" wird hier eine Person oder Entität verstanden, die sich bei der Authentifizierungssoftware vorab registriert hat und die von der Authentifizierungssoftware als vertrauenswürdig behandelt wird zumindest im Hinblick auf bestimmte ausgewählte Funktionen. Die Entität kann eine einzelne Person aber auch eine Personengruppe sein. Die Authentifizierung als Vertrauensperson kann also, muss aber nicht individuell für eine bestimmte Person erfolgen. In manchen Ausführungsformen reicht es aus, wenn die Person im Zuge der Authentifizierung nachweist, Mitglied einer als vertrauenswürdig eingestuften Personengruppe zu sein, um sich erfolgreich als "Vertrauensperson" zu authentifizieren.

Unter einem "**Bekanntbild**" wird hier ein Bild verstanden mit einem Bildmotiv, das der Person bekannt ist, sofern es sich bei der Person um die Vertrauensperson handelt. Dies kann z.B. durch die Art, wie die Authentifizierungssoftware das Bekanntbild erfasst oder über die Quelle von welcher die Authentifizierungssoftware das Bekanntbild bezieht, sichergestellt sein: wenn eine Person sich mit einem ID-Dokument authentifizieren möchte, das das Gesichtsbild der Vertrauensperson beinhaltet, als die sich die Person authentifizieren möchte, so muss und kann die Authentifizierungssoftware davon ausgehen, dass das auf dem ID-Dokument abgebildete Gesichtsbild der sich authentifizierenden Person bekannt ist, wenn diese Person wirklich die Vertrauensperson ist, die auf dem Bild abgebildet ist.

Beispiel aktuell erfasstes Gesichtsbild als Bekanntbild der Vertrauensperson: wenn ein aktuelles Bild des Gesichts der sich authentifizierenden Person erfasst wird, so wird zunächst die Vertrauensperson bzw. deren digitale Repräsentation ermittelt, als welche sich die Person authentifizieren möchte, und die aktuell erfassten Gesichtsbilder werden dieser Vertrauensperson dynamisch als "Bekanntbilder" zugeordnet. Nun gibt es zwei Möglichkeiten: a) die Person trägt keine Maske, dann ist das Gesichtsbild der Person das eigene Gesichtsbild und der Person auch bekannt. Das Gesicht der Person auf dem erfassten Bild ist das Gesicht der Vertrauensperson, die sie vorgibt zu sein. Die Ähnlichkeitsanalyse der erfassten ersten und zweiten Hirnaktivitätssignale ergibt eine Signalsequenz erster und zweiter Hirnaktivitätssignale, die der Sequenz der gezeigten Bekannt- und Fremdbilder entspricht; und b) die Person trägt eine Gesichtsmaske, die das Gesicht einer anderen Person, nämlich der Vertrauensperson, abbildet. Das Gesicht der Person auf dem erfassten Bild ist das Gesicht der Vertrauensperson, aber nicht das Gesicht der Person, die sich aktuell versucht zu authentifizieren. Die Ähnlichkeitsanalyse der erfassten ersten und zweiten Hirnaktivitätssignale ergibt eine Sequenz ähnlicher und unähnlicher Hirnaktivitätssignale, die nicht der Sequenz der gezeigten Bekannt- und Fremdbilder entspricht, weil die Bekanntbilder der Vertrauensperson der sich authentifizierenden Person nicht bekannt sind.

Beispiel aktuell erfasstes Gesichtsbild auf einem ID-Dokument als Bekanntbild der Vertrauensperson: wenn ein auf einem ID-Dokument der sich authentifizierenden Person aufgedrucktes Gesichtsbild erfasst wird, so wird zunächst die Vertrauensperson bzw. deren digitale Repräsentation ermittelt, als welche sich die Person authentifizieren möchte, und die aktuell erfassten Gesichtsbilder werden dieser Vertrauensperson dynamisch als "Bekanntbilder" zugeordnet. Nun gibt es mehrere Möglichkeiten:
a) das ID-Dokument ist "echt" und das darauf gezeigte Gesichtsbild zeigt das Gesichtsbild der Vertrauensperson, die sich bereits bei der Authentifizierungssoftware registriert hat und die der Software daher bekannt ist. Der Person, die sich authentifizieren möchte, gehört das ID-Dokument auch, d.h., das Gesicht der Person auf dem Ausweisbild ist das Gesicht der Vertrauensperson, die die sich authentifizierende Person vorgibt zu sein. Die Ähnlichkeitsanalyse der erfassten ersten und zweiten Hirnaktivitätssignale ergibt eine Sequenz ähnlicher und unähnlicher Hirnaktivitätssignale, die der Sequenz der gezeigten Bekannt- und Fremdbilder entspricht;
b) das ID-Dokument ist echt, gehört aber eigentlich einer anderen Person (Vertrauensperson) als der Person ("Dieb"), die das Dokument aktuell verwendet, um sich damit zu authentifizieren. Der "Dieb" und die Vertrauensperson sehen sich so ähnlich, dass das Gesichtsbild auf dem Ausweis für die meisten Menschen nicht unterscheidbar ist. In diesem Fall zeigt das Ausweis-Gesichtsbild also ein Gesicht, das der Vertrauensperson bekannt ist, für den Dieb ist es aber ein Fremdbild. Die Ähnlichkeitsanalyse der erfassten ersten und zweiten Hirnaktivitätssignale ergibt eine Sequenz ähnlicher und unähnlicher Hirnaktivitätssignale, die nicht der Sequenz der gezeigten Bekannt- und Fremdbilder entspricht, weil das Bekanntbild der Vertrauensperson tatsächlich ein Fremdbild für den "Dieb" darstellt.
c) das ID-Dokument ist manipuliert und das darauf gezeigte Gesichtsbild ist gemorpht, d.h., es zeigt Teile des Gesichts der Vertrauensperson, die sich bereits bei der Authentifizierungssoftware registriert hat, und Teile des Gesichts der Person, die sich aktuell authentifizieren möchte. In diesem Fall existiert die Vertrauensperson, die das gemorphte Bild als Bekanntbild erkennen könnte, nur fiktiv, da das gemorphte Bild künstlich erzeugt wurde. Faktisch ist das gemorphte Bild für jeden Menschen ein Fremdbild. Die Ähnlichkeitsanalyse der erfassten ersten und zweiten Hirnaktivitätssignale ergibt eine Sequenz ähnlicher und unähnlicher Hirnaktivitätssignale e, die nicht der Sequenz der gezeigten Bekannt- und Fremdbilder entspricht, weil die Bekanntbilder der (fiktiven) Vertrauensperson der sich authentifizierenden Person nicht bekannt sind.

Nach manchen Ausführungsformen sind Bekanntbilder in einer Datenbank verknüpft mit den Nutzerprofilen von Nutzern verknüpft. Die jeweiligen Nutzerprofile repräsentieren Vertrauenspersonen, als welche sich Nutzer bei der Authentifizierungssoftware authentifizieren können.

Die Motive der Bekanntbilder und Fremdbilder, die von der Authentifizierungssoftware ermittelt und angezeigt werden, gehören vorzugsweise zum gleichen Typ. Ein Motivtyp kann z.B. ein Typ eines physischen Objekts sein, z.B. Gesicht, der menschliche Körper, ein Gegenstand einer bestimmten Kategorie, ein Fahrzeug, ein Wasserfahrzeug, ein Gebäude, eine Tierart, insbesondere eine Haustierart. Bei den Bekanntbildern einer Vertrauensperson kann es sich z.B. um das Arbeitsgebäude oder Haustier oder das Gesicht eines Familienmitglieds handeln und bei dem Fremdbild um ein unbekanntes Gebäude ähnlicher Größe oder Funktion, ein unbekanntes Individuum der gleichen Haustierart, ein Gesicht einer fremden Person, etc.

Vorzugsweise ist ein Bekanntbild ein Bild, von welchem bekannt ist oder der Betreiber der Authentifizierungssoftware mit hinreichender Wahrscheinlichkeit annehmen kann, dass das Motiv des Bekanntbildes nur der Vertrauensperson bekannt und keiner weiteren Person sonst, oder zumindest keiner weiteren Person, die sich mit hinreichender Wahrscheinlichkeit unberechtigterweise authentifizieren möchte. Beispielsweise können Gegenstände in einem Raum, zu dem nur ein kleiner Personenkreis Zutritt hat, durchaus allen Mitgliedern dieses Personenkreises bekannt sein und als Motiv eines Bekanntbildes verwendet werden. Alle diese Personen gelten jedoch als berechtigt bezüglich des Zutritts zu diesem Raum. Damit der Gegenstand als Motiv eines Bekanntbildes verwendet werden kann, ist nur erforderlich, dass er nicht auch in anderen Räumen vorkommt, in welchen andere Personen arbeiten, die keinen Zutritt zu dem geschützten Raum erhalten sollen.

Bekanntheit ist hier im Sinne von hoher Vertrautheit zu verstehen, die durch mehrfaches Betrachten des Motivs des Bekanntbildes über einen längeren Zeitraum entsteht, vorzugweise durch mindestens tägliches oder mindestens wöchentliches Betrachten über einen Zeitraum von mehreren Monaten oder Jahren hinweg.

Unter einem "Fremdbild" wird hier ein Bild verstanden, das ein physisches Objekt darstellt, von welchem bekannt ist oder von dem mit hinreichend hoher Wahrscheinlichkeit davon auszugehen ist, dass das dargestellte physische Objekt der sich authentifizierenden Person nicht bekannt ist oder zumindest signifikant weniger bekannt ist als der Vertrauensperson.

Beispielsweise kann es sich bei einem Fremdbild, das einer bestimmten Person gezeigt wird, um ein Bild handeln, welches das Gesicht einer anderen Person abbildet, die auf einem anderen Kontinent lebt, die vor langer Zeit gelebt hat oder von welcher aus sonstigen Gründen nicht anzunehmen ist, dass die Personen, die sich authentifizieren wollen, diese andere Person jemals gesehen haben. Das "nicht-Bekanntsein" des Motiv eines Fremdbildes ist vorzugsweise so zu verstehen, dass mit einer gewissen Mindestwahrscheinlichkeit keine Person, die sich des Authentifizierungsverfahrens voraussichtlich bedienen wird, diese andere Person jemals gesehen hat.

Unter einer **"signifikanten Ähnlichkeit"** von Datenwerten wie zum Beispiel Hirnaktivitätssignalen wird hier insbesondere eine Situation verstanden, in welchem zwei Signale als im Rahmen der Messgenauigkeit identisch oder sehr ähnlich angesehen werden. Unter Berücksichtigung der Messungenauigkeit des Sensortyps und Sensormodells können "signifikant ähnliche Signale" also Signale sein, deren Unterschiedlichkeits-Score unterhalb eines vordefinierten Grenzwerts liegt, wobei der Grenzwert so gewählt ist, dass Unterschiedlichkeits-Scores von Hirnaktivitätssignalen, die von im Wesentlichen identischen oder sehr ähnlichen Vorgängen im Kopf der Person erzeugt werden, unterhalb dieses Grenzwerts liegen.

Unter einer **"signifikanten Unähnlichkeit"** von Datenwerten wie zum Beispiel Hirnaktivitätssignalen wird hier verstanden, dass die verglichenen Daten sich so stark voneinander unterscheiden, dass es sehr unwahrscheinlich ist, dass die Unterschiede auf messtechnische oder sonstige Varianzen (Messfehler) zurückzuführen sind. Bei der messtechnischen Erfassung von Signalen (z.B. mit optischem oder elektrischem Sensor) ist es unvermeidlich, dass hierbei leichte Schwankungen der Signalstärke auftreten. Die hierdurch bedingte Varianz, auch Messfehler oder "Hintergrundrauschen" bezeichnet, hat je nach Sensortyp und Sensormodell eine unterschiedliche Größe und Beschaffenheit, sodass es nicht möglich ist, für alle denkbaren Typen und Modelle von Sensoren eine absolute Grenze vorzugeben, ab welcher Höhe Signalunterschiede noch im Rahmen der Messungenauigkeit unterschiedlich sind und wann sie dagegen "signifikant unterschiedlich" ("signifikant unähnlich") sind, also so unterschiedlich sind, dass die Unterschiede nicht mehr auf die übliche Messungenauigkeit zurückführbar sind. Dem Fachmann ist der Umgang mit Messungenauigkeiten beim Verarbeiten und Vergleichen von gemessenen Signalen jedoch bekannt. Durch Wahl geeigneter Grenzwerte, z.B. eines Mindestabstands zwischen zwei Signalamplituden oder einer Mindestfläche, die sich errechnet als Differenz der Flächen unter zwei Amplitudenprofile der beiden Signale über die Zeit, kann der Fachmann definieren, ab welcher Unterschiedlichkeitsschwelle zwei Hirnaktivitätssignale als "signifikant unterschiedlich" gelten und wann die Signale dagegen als "identisch oder ähnlich im Rahmen der jeweiligen Messgenauigkeit" gelten.

Unter Berücksichtigung der Messungenauigkeit des Sensortyps und Sensormodells können "signifikant unähnliche Signale" oder "Signale, die signifikant unterschiedlich sind" also Signale sein, deren Unterschiedlichkeits-Score über einem vordefinierten Grenzwert liegt, wobei der Grenzwert so gewählt ist, dass Unterschiedlichkeits-Scores von Hirnaktivitätssignalen, die von im Wesentlichen unterschiedlichen Vorgängen im Kopf der Person erzeugt werden (z.B. beim Betrachten von Fremdbildern einerseits und von Bekanntbildern andererseits), über diesem Grenzwert liegen.

Unter einer **"Authentifizierungssoftware"** wird hier Software verstanden, die dazu ausgebildet ist, ein Authentifizierungsverfahren durchzuführen, um eine bestimmte Person individuell oder als Mitglied einer Gruppe zu identifizieren. Die Software kann als eigenständiges einzelnes Applikationsprogramm ausgebildet sein oder als eine multimodulare und optional verteilt auf mehreren Rechnern ausgeführte Software (z.B. Authentifizierungssoftware in Form eines Clouddiensts).

Authentifizierung ist die Verifizierung einer behaupteten Eigenschaft (claim) einer Person durch eine prüfende Instanz (z.B. Authentifizierungssoftware), wobei die Person hierfür Daten an die prüfende Instanz bereitstellt, also eine Authentisierung durchführt. Das Wort Authentifizieren bezeichnet hier den gesamten Vorgang der Bereitstellung der Information (Authentisierung) als auch die Echtheitsprüfung dieser Daten. Die Authentisierung einer Person bezüglich der behaupteten Eigenschaft der Authentizität, die beispielsweise Einräumen einer "bestehenden Zugangsberechtigung" oder "Mitgliedschaft in einer Personengruppe, die bestimmte Rechte zum Zugriff auf bestimmte Funktionen hat", sein kann, erlaubt der authentifizierten Person weitere Aktionen. Die Person gilt dann als authentisch bzw. als "erfolgreich authentifiziert".

Unter einer "**Challenge**" wird hier eine Menge aus einem oder mehreren Datenwerten verstanden, welche einer ersten Partei bekannt ist, und welche einer zweiten Partei auf eine bestimmte Weise übermittelt wird um es der zweiten Partei zu ermöglichen, sich durch Nachweis der Kenntnis der Challenge oder durch Nachweis einer vordefinierten Verarbeitung der Challenge bei der ersten Partei zu authentifizieren.

### Kurze Beschreibung der Zeichnung

Nachfolgend werden Ausführungsformen der Erfindung mit Bezug auf die Zeichnung beschrieben. In der Zeichnung zeigt
- Fig. 1: ein exemplarisches Flussdiagramm einer Ausgestaltung des erfindungsgemäßen Verfahrens zur Authentifizierung einer Person;
- Fig. 2: ein Blockdiagramm eines Authentifizierungssystems;
- Fig. 3: ein Authentifizierungssystem auf Basis von Bildern in Ausweisdokumenten;
- Fig. 4: ein Authentifizierungssystem auf Basis von Spiegelbildern;
- Fig. 5: ein Authentifizierungssystem auf Basis von Kamerabildern;
- Fig. 6: eine Sequenz von angezeigten Bekanntbildern und Fremdbildern;
- Fig. 7: eine Sequenz von ersten und zweiten Hirnaktivitätssignalen;
- Fig. 8: ein Schema eines neuronalen Netzes zur Analyse und Klassifizierung der Hirnaktivitätssignale;
- Fig. 9: einen Plot mit zwei Hirnaktivitätssignalen in Form gemessener Ladungsumverteilungen in der Sensorumgebung bei Betrachtung eines Fremdbilds und eines Bekanntbilds;
- Fig. 10: ein Blockdiagramm eines weiteren Authentifizierungssystems; und
- Fig. 11: einen Plot mit Hirnaktivitätssignalen in Form von gemessenen Pupillendurchmesser bei Betrachtung eines Fremdbilds und eines Bekanntbilds.

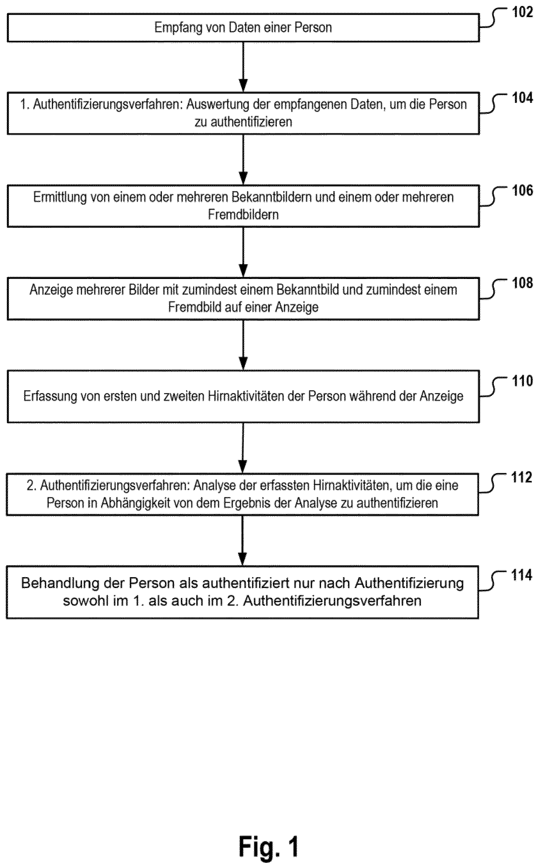

**Figur 1** illustriert ein exemplarisches Flussdiagramm einer Ausgestaltung des erfindungsgemäßen Verfahrens zur Authentifizierung einer Person.

Ausführungsformen der Erfindung können als Erweiterung klassischer Authentifizierungsverfahren, insbesondere automatischen Gesichtserkennungsverfahren, verwendet werden.

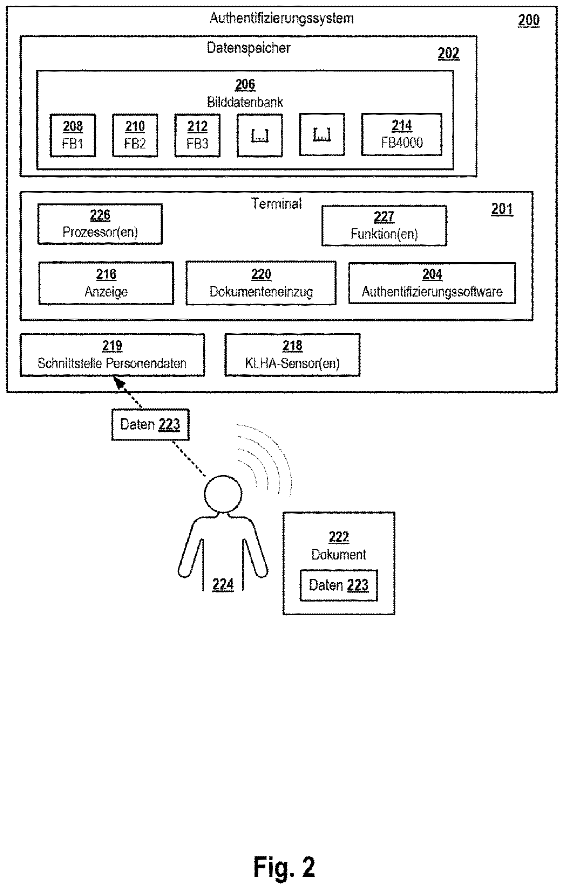

In einem ersten Authentifizierungsverfahren wird eine konventionelle Authentifizierung, z.B. mittels Gesichtserkennung, durchgeführt. Danach wird, zumindest sofern sich die Person im ersten Authentifizierungsverfahren erfolgreich authentifiziert hat, ein zweites Authentifizierungsverfahren auf Basis der kontaktlosen Messung und Analyse von Hirnaktivitätssignalen durchgeführt. Nur wenn die Person sich sowohl im ersten als auch im zweiten Authentifizierungsverfahren erfolgreich authentifiziert hat, wird die Person letztlich vom Authentifizierungssystem als "endgültig" bzw. letztlich "authentifiziert" behandelt. Die Bezeichnung "erstes" und "zweites" Authentifizierungsverfahren sollen hier keine Reihenfolge implizieren. Die in Figur 1 beschriebene Ausführungsform führt zunächst ein klassisches biometrisches Authentifizierungsverfahren durch und danach das auf den kontaktlos erfassten Hirnaktivitätssignalen basierende Authentifizierungsverfahren. Eine umgekehrte Reihenfolge ist jedoch ebenso möglich. Zur Erleichterung des Verständnisses wird teilweise bereits hier auf Elemente des in Figur 2 gezeigten Authentifizierungssystems 200 referenziert, das Verfahren kann jedoch auch mit anderen Ausführungsformen eines Authentifizierungssystems durchgeführt werden.

In einem ersten Schritt 102 empfängt die Authentifizierungssoftware 204 Daten 223 von einer Person 224, die sich authentifizieren möchte.

Beispielsweise kann es sich bei der Person 224 um einen Mitarbeiter einer Firma handeln, welcher ein Tor oder eine Tür zu einem Firmengelände seines Arbeitgebers passieren möchte und sich hierfür bei dem Authentifizierungssystem 200 seines Arbeitgebers authentifizieren muss. Beispielsweise gibt die Person zum Zwecke der Authentifizierung ihren Namen oder Nutzernamen ein oder führt ein papierbasiertes oder elektronisches ID-Dokument 222, das einen Identitätshinweis auf die Person 224 und optional auch personenbezogene Daten 223 enthält, in ein Lesegerät eines Terminals ein. Die von der Authentifizierungssoftware von der Person 224 empfangenen Daten 223 können jegliche Daten der Person sein, die es der Authentifizierungssoftware erlauben, im Zuge des ersten Authentifizierungsverfahren die individuelle Identität oder Gruppenzugehörigkeit der Person (Management/R&D, etc.) zu prüfen und die Person individuell oder als Mitglied einer bestimmten Personengruppe zu identifizieren. Beispielsweise kann es sich bei den Daten um biometrische Daten der Person handeln, also z.B. um ein von einer Kamera aktuell erfasstes Gesichtsbild, um einen von einem Fingerabdrucksensor erfassten Fingerabdruck, oder um ein aktuell von einer Kamera erfasstes Irisbild der Person. Es kann sich aber auch um andere Daten handeln, z.B. um Daten, die elektronisch, optisch oder auf sonstige Weise von einem ID-Dokument 222 der Person (z.B. Mitarbeiterausweis, Reisepass, Personalausweis, Gesundheitskarte etc.) ausgelesen wurden.

In einem weiteren Schritt 104 führt die Authentifizierungssoftware 204 ein erstes Authentifizierungsverfahren durch. Dieses umfasst eine Auswertung der empfangenen Daten 223, um die Person 224 zu authentifizieren. Je nach Art der verwendeten Daten 223 kann es sich bei dem ersten Authentifizierungsverfahren um klassische biometrische oder nicht-biometrische Authentifizierungsverfahren handeln, wie dies im Stand der Technik bekannt sind. Insbesondere kann es sich um ein automatisches Gesichtserkennungsverfahren (face-recognition Verfahren) handeln. Im Zuge dieses Verfahrens wird mindestens ein Gesichtsbild der Person, die sich authentifizieren will, durch einen optischen Sensor (Kamera) erfasst und analysiert.

Gemäß einer bevorzugten Ausführungsform werden ein oder mehrere der nachfolgenden Schritte 106-114 nur dann ausgeführt, wenn sich die Person im ersten Authentifizierungsverfahren erfolgreich gegenüber der Authentifizierungssoftware authentifiziert hat.

In Schritt 106 ermittelt das Authentifizierungssystem für die Person, die sich authentifizieren möchte, mindestens ein Bekanntbild.

Beispielsweise kann dieser Schritt Bestandteil des ersten Authentifizierungsverfahrens sein und aus der Erfassung des Gesichtsbilds dieser Person 224 mit einer Kamera bestehen. Beispielsweise ist das erste Authentifizierungsverfahren ein Gesichtsbilds-Erkennungsverfahren und dass im Zuge dessen erfasste Gesichtsbild der Person 224 wird als das zumindest eine Bekanntbild verwendet. Der Betreiber der Authentifizierungssoftware kann sicher davon ausgehen, dass ein Bild des eigenen Gesichts der Person, die sich authentifizieren möchte, bekannt sein muss, es sei denn - und genau dies gilt es zu erkennen-die Person trägt während der Durchführung des ersten Authentifizierungsverfahrens eine Maske. Die Ermittlung von einem oder mehreren Fremdbildern kann darin bestehen, dass auf eine FremdbildDatenbank zugegriffen wird.

Es ist aber auch möglich, dass die Ermittlung des zumindest einen Bekanntbilds dieser Person 224 in einem separaten Arbeitsschritt implementiert ist.

Beispielsweise kann das Authentifizierungssystem eine Datenbank mit den Nutzerprofilen registrierter Nutzer und eine Bilddatenbank 206 mit mehreren Fremdbildern und Bekanntbildern beinhalten, wobei jedem der registrierten Nutzer über das Nutzerprofil mindestens eines der Bekanntbilder zugewiesen ist. Die Fremdbilder können einzelnen Nutzern spezifisch zugewiesen sein, es ist aber auch möglich dass keine solche Zuweisung vorhanden ist, da das Authentifizierungssystem davon ausgehen kann, dass die in den Fremdbildern gezeigten Bilder keinem der registrierten Nutzer bekannt sind.

Beispielsweise kann die Ermittlung der Bekanntbilder und Fremdbilder für die Person 224 darin bestehen, dass die Authentifizierungssoftware anhand der erfassten Daten 223 und/oder anhand von Identitätsdaten, die manuell eingegeben oder von einem ID-Dokument empfangen wurden, ein Nutzerprofil identifiziert, welches diese Person 224 repräsentiert. In einem nächsten Schritt werden ein oder mehrere Bekanntbilder innerhalb der Bilddatenbank identifiziert, welcher dieser Person spezifisch zugewiesen sind, und werden ein oder mehrere Fremdbilder ermittelt, die dieser Person entweder ebenfalls spezifisch zugewiesen sind oder in einer generischen Fremdbild Datenbank enthalten sind, wobei die Fremdbilder dieser Fremdbilddatenbank für sämtliche registrierten Nutzer Fremdbilder darstellen.

Nachdem also auf Basis des ersten Authentifizierungsverfahrens schon gewisse Informationen über die Person 224 oder der Personengruppe, der diese angehört, bekannt sind, werden ein oder mehrere Bilder in einer Personen-Datenbank ermittelt, die mit der erkannten Person oder Personengruppe verknüpft gespeichert sind und von welchen bekannt ist, dass sie eine Person, einen Gegenstand oder einen Ort abbilden, die der Person bzw. Mitgliedern der Personengruppe bekannt sein müssen.

In einem nächsten Schritt 108 wird vorzugsweise eine zufällige Sequenz ("Challenge") mehrerer Bekanntbilder und Fremdbilder der Person über eine Anzeige 216 angezeigt. Die Sequenz beinhaltet also eine Mischung von Fremdbildern und Bekanntbildern in zufälliger Reihung, wobei die Reihung der Authentifizierungssoftware bekannt ist. Eine zufällige Sequenz von Bekannt- und Fremdbildern ist eine Bildsequenz, bei welcher die chronologische Reihenfolge der Bekanntbilder und Fremdbilder und optional zudem auch die Anzahl der Bekanntbilder und/oder Fremdbilder zufällig gewählt wird. Die Sequenz der Bekanntbilder und Fremdbilder wird von der Authentifizierungssoftware gemäß einer Ausführungsform bei jedem Authentifizierungsvorgang auf Zufallsbasis neu erzeugt.

Bei den mehreren Bekanntbildern kann es sich um Kopien eines einzigen Bekanntbilds handeln. Bei den mehreren Fremdbildern kann es sich um Kopien eines einzigen Fremdbildes handeln. Bei der Anzeige kann es sich z.B. um einen Bildschirm eines Terminals, handeln. Das Terminal kann z.B. ein Terminal zur Prüfung der Identität von Mitarbeitern einer Firma sein. In analoger Weise können entsprechende Terminals und Authentifizierungssysteme bzw. Authentifizierungsverfahren gemäß Ausführungsformen der Erfindung auch für andere Zwecke, z.B. zur Personenkontrolle an Flughäfen, Grenzen und sicherheitskritischen Gebäuden, verwendet werden.

Während der Anzeige der Bildsequenz erfasst in Schritt 110 ein KLHA-Sensor 218 die Hirnaktivitätssignale, die von der Person während des Betrachtens der Sequenz aus Fremdbildern und Bekanntbildern erzeugt werden. Der KLHA-Sensor kann auch mehrere Sensoren umfassen. Sofern in dieser Anmeldung von einem einzigen Sensor gesprochen wird, ist die Möglichkeit der Verwendung mehrerer Sensoren implizit mitgemeint.

Der Authentifizierungssoftware ist bekannt, zu welchen Zeiträumen Bekanntbilder bzw. Fremdbilder angezeigt wurden. Die Authentifizierungssoftware erfasst diejenigen Hirnaktivitätssignale, die jeweils während der Anzeige eines Bekanntbildes von dem KLHA-Sensor erfasst wurden, als sogenannte "erste Hirnaktivitätssignale". Hirnaktivitätssignale, die jeweils während der Anzeige eines Fremdbildes von dem KLHA-Sensor erfasst wurden, werden von der Authentifizierungssoftware als sogenannte "zweite Hirnaktivitätssignale" erfasst.

Der Sensor wird so bereitgestellt, dass die Position, Entfernung und Orientierung des Sensors relativ zu dem Kopf der Person, die sich authentifizieren möchte, geeignet ist, dass im jeweiligen Fall zu erfassende Hirnaktivitätssignal in hinreichender Stärke zu erfassen.

Falls es sich bei dem KLHA-Sensor beispielsweise um einen elektrischen Sensor handelt, kann dieser innerhalb der Kopfstütze einer Sitzgelegenheit, auf weiche sich die zu authentifizierende Person setzen soll, verbaut sein. Der Sensor ist in diesem Fall in der Nähe des Hinterkopfes der Person in einem Abstand von typischerweise weniger als 15 cm, vorzugsweise weniger als 10 cm, vom Kopf der Person entfernt, ohne allerdings den Kopf direkt zu berühren.

Falls es sich bei dem Sensor um einen optischen Sensor handelt, zum Beispiel eine Kamera, so ist diese beispielsweise so an der Decke oder Wand eines Raumes verbaut, dass die Kamera ein Bild des Gesichtes oder zumindest der Augenpartie der Person erfassen kann, wenn die Person sich an einer bestimmten Position innerhalb des Raumes befindet, an welcher die Authentifizierung stattfinden soll. Beispielsweise kann diese Position durch Markierungen auf dem Fußboden angedeutet sein, wie dies beispielsweise schon heute bei Körperscannern für die Sicherheitskontrolle in Flughafen üblich ist.

In Schritt 112 führt die Authentifizierungssoftware ein zweites Authentifizierungsverfahren durch. Im Zuge der zweiten Authentifizierung prüft die Authentifizierungssoftware beispielsweise, ob die chronologische Reihenfolge der ersten und zweiten Hirnaktivitätssignale dem in der Challenge codierten zeitlichen Muster von Bekanntbildern und Fremdbildern entspricht. Im zweiten Authentifizierungsverfahren prüft die Authentifizierungssoftware z.B., ob zu den Zeiten, in welchen Bekanntbilder gezeigt wurden, immer das gleiche oder ein sehr ähnliches erstes Hirnaktivitätssignal empfangen wurde, und ob zu den Zeiten, in welchen Fremdbilder gezeigt wurden, immer das gleiche oder ein sehr ähnliches zweites Hirnaktivitätssignal empfangen wurde, wobei das erste und zweite Hirnaktivitätssignal signifikant unterschiedlich voneinander sind. Nur falls alle diese Kriterien erfüllt sind, wird die Person als im zweiten Authentifizierungsverfahrens erfolgreich authentifiziert behandelt.

Nach manchen Ausführungsformen wird die Sequenz an Bekanntbildern und Fremdbildern so erzeugt, dass sie eine Mindestanzahl an Bekanntbildern und/oder Fremdbildern beinhaltet, z.B. jeweils mindestens 3 oder mindestens 5 Bilder. Im Zuge der Auswertung der ersten und zweiten Hirnaktivitätssignale wird geprüft, ob die ersten Hirnaktivitätssignale zueinander ähnlich sind, ob die zweiten Hirnaktivitätssignale zueinander ähnlich sind, und ob die ersten Hirnaktivitätssignale zu den zweiten Hirnaktivitätssignalen unähnlich sind. Bei manchen Ausführungsformen, bei denen die Challenge eine große Anzahl an Bildern beinhaltet, z.B. mindestens 10 Bilder insgesamt, kann das zweite Authentifizierungsverfahren auch eine gewisse Toleranz für einzelne falsche Ergebnisse haben, beispielsweise maximal ein Hirnaktivitätssignal, welches der anderen Signalkategorie (erstes oder zwietes Hirnaktivitätssignal) ähnlicher sieht als der Kategorie, der es angesichts des gezeigten Bildes angehören sollte. In manchen Ausführungsformen gelten die Anforderungen an die Signalkategorietreue (Reproduzierbarkeit) absolut, d.h., es darf gar keine falsche Abweichung von erwarteten und beobachteten Signalähnlichkeiten geben. In anderen Ausführungsformen, z.B. solchen wo sehr lange Signalsequenzen verwendet und/oder die Sicherheitsanforderungen geringer sind können die Anforderungen an die Reproduzierbarkeit geringer sein und einen oder einige wenige Fehler zulassen.

In Schritt 114 wertet die Authentifizierungssoftware die Ergebnisse des ersten und zweiten Authentifizierungsverfahrens aus und behandelt die Person 224 nur dann als authentifiziert, wenn diese sich sowohl im ersten als auch im zweiten Authentifizierungsverfahren erfolgreich authentifiziert hat. Die ersten und zweiten Authentifizierungsverfahren sind letztlich also nur Teilprozesse eines mehrstufigen Authentifizierungsverfahrens, das in Figur 1 beispielhaft beschrieben ist. Beispielsweise kann die Authentifizierungssoftware dazu konfiguriert sein, eine bestimmte Hardware- oder Softwarefunktion 227, z.B. das automatische Öffnen einer Tür zum Firmengelände, nur dann durchzuführen, wenn die Person sich sowohl im ersten als auch im zweiten Authentifizierungsverfahren erfolgreich authentifiziert hat.

Gemäß einigen Ausführungsformen ist das erste Authentifizierungsverfahren ein kontaktloses Authentifizierungsverfahren, z.B. auf Basis der Erkennung von Gesichtsbildern oder Irisbildern, die mit einem optischen Sensor erfasst wurden.

Da kein Kontakt zwischen der zu authentifizierenden Person und dem Sensor im Zuge des zweiten Authentifizierungsverfahrens erforderlich ist, und in manchen Ausführungsformen auch das erste Authentifizierungsverfahren kontakltos ist, ist das Verfahren besonders geeignet zur schnellen Identitätsprüfung einer großen Zahl von Personen in kurzer Zeit.

Ausführungsformen der Erfindung können viele unterschiedliche Verwendungszwecke haben, zum Beispiel für Identitätskontrollen an Flughäfen, Firmentoren, und/oder Ländergrenzen.

**Figur 2** zeigt ein Blockdiagramm eines Authentifizierungssystems 200.

Das Authentifizierungssystem beinhaltet eine Anzeige 216, zum Beispiel einen elektronischen Monitor, der beispielsweise Bestandteil eines Terminals 201 sein kann. Das Terminal beinhaltet ein oder mehrere Prozessoren 226, die dazu ausgebildet sind, computerinterpretierbare Instruktionen einer Authentifizierungssoftware 204, die auf einem Speicher des Terminals gespeichert ist, auszuführen. In anderen Ausführungsformen kann anstelle eines Terminals aber auch ein anderes datenverarbeitendes System, z.B. ein Standardcomputer, z.B. ein Desktop-Computer oder Server-Computer, verwendet werden, wobei dieses datenverarbeitende System vorzugsweise einige oder alle der hier für das Terminal beschriebenen Komponenten umfasst oder operativ an diese gekoppelt ist.

Die Authentifizierungssoftware 204 umfasst nach Ausführungsformen der Erfindung Funktionen, um das in Figur 1 beschriebenen mehrschrittige Authentifizierungsverfahren durchzuführen.

Das Terminal kann über eine oder mehrere Schnittstellen 219 zum Empfang von Daten 223 der Person 224 beinhalten, wobei die empfangenen Daten zumindest im Zuge des ersten Authentifizierungsverfahrens zur Authentifizierung der Person 224 und optional auch im zweiten Authentifizierungsverfahren zur Ermittlung der Bekanntbilder oder direkt als Bekanntbild der Person 224 verwendet werden. Beispielsweise kann die Schnittstelle 219 als optischer Sensor ausgebildet sein, der Gesichtsbilder der Person 224 aktuell erfasst und im Zuge des ersten Authentifizierungsverfahrens auswertet. In der in Figur 2 gezeigten Ausführungsform beinhaltet das Terminal außerdem einen Dokumenteneinzug 220, über welchen ein ID-Dokument 222 der Person 224, die sich authentifizieren möchte, zum Beispiel ein Personalausweis oder Reisepass, eingezogen werden kann. Beispielsweise kann das erste Authentifizierungsverfahren so ausgebildet sein, dass es wahlweise auf Basis einer Gesichtserkennung oder auf Basis eines ID-Dokuments implementiert ist, oder es kann auch ein Authentifizierungsverfahren sein, das beide Informationsquellen nutzt. Beispielsweise kann die Authentifizierungssoftware den Name oder einen sonstigen Identifikator der Person 224 im Zuge der Ausführung des ersten Authentifizierungsverfahrens vom ID-Dokument lesen und dazu verwenden, die für diese Person hinterlegten Referenz-Gesichtsbilder (für das erste Authentifizierungsverfahren) und/oder die für diese Person gespeicherten Bekanntbilder oder Fremdbilder (für das zweite Authentifizierungsverfahren) zu ermitteln.

Das Authentifizierungssystem 200 umfasst zudem einen KLHA Sensor 218 und einen Datenspeicher 202, in welchem sich eine Bilddatenbank 206 befindet.

Der KLH-Sensor kann der gleiche optische Sensor sein, der zur Erfassung personenbezogener Daten 223 im ersten Authentifizierungsverfahren verwendet wurde, beispielswiese eine Gesichtsbildkamera. Es kann sich aber auch um einen elektrischen Sensor handeln.

Der Datenspeicher kann Bestandteil des Terminals 201 sein, oder mit dem Terminal über ein Netzwerk, zum Beispiel ein Intranet oder das Internet, verbunden sein. Die Bilddatenbank kann eine Vielzahl von Bekanntbildern und/oder Fremdbildern enthalten. Beispielsweise kann es sich bei den Bekanntbildern und/oder Fremdbildern um Gesichtsbilder von Personen handeln, die beim Betreiber des Terminals "registriert" oder auf sonstige Art und Weise bekannt oder verzeichnet sind. Zumindest bei den Fremdbildern kann es sich aber auch um andere Personen handeln, die nicht registriert sind. Falls die Bilddatenbank Bekanntbilder enthält, sind diese jeweils mindestens einem Nutzerprofil eines registrierten Nutzers zugeordnet. Die Zuordnung kann alternativ auch dynamisch erfolgen, falls die Bekanntbilder aus aktuell erfassten Gesichtsbildern bestehen. Die Fremdbilder können, müssen aber keinem Nutzerprofil zugeordnet sein. In anderen Ausführungsformen sind die Motive der Fremdbilder und Bekanntbilder in der Datenbank gar keine Gesichter, sondern sonstige physische Objekte, z.B. Gebäude, Tiere oder Gegenstände.

Die Bilddatenbank beinhaltet beispielsweise ein Fremdbild 208, ein weiteres Fremdbild 210, sowie eine Vielzahl weiterer Fremdbilder 212, 214, die für jede denkbare natürliche Person ein Fremdbild sind, weil die Fremdbilder gemorphte Bilder von Personen sind, die nicht der Organisation angehören, die die Authentifizierungssoftware 204 betreibt. In manchen Ausführungsformen, beinhaltet die Bilddatenbank auch ein oder mehrere Bekanntbilder (hier nicht gezeigt), die jeweils einzelnen Nutzerprofilen registrierter Personen zugewiesen sind. Ob es sich bei einem Bild in der Bilddatenbank um ein Bekanntbild oder Fremdbild handelt, ist keine statische Eigenschaft des jeweiligen Bildes, sondern hängt von der Person ab, die sich zu einem bestimmten Zeitpunkt authentifizieren möchte, jedoch kann zumindest die Erstellung oder Auswahl der Fremdbilder so gestaltet werden, dass sichergestellt ist, dass das darauf gezeigte Motiv allen registrierten Personen unbekannt ist.

Die Authentifizierungssoftware 204 ist dazu ausgebildet, ein erstes und ein zweites Authentifizierungsverfahren durchzuführen, wobei vorzugsweise das zweite nur dann durchgeführt wird, wenn sich die Person im ersten Authentifizierungsverfahren bereits erfolgreich authentifiziert hat. Im Zuge des zweiten Authentifizierungsverfahrens erzeug die Authentifizierungssoftware 204 für jeden Authentifizierungsvorgang eine neue zeitliche Sequenz aus Bekanntbildern und Fremdbildern zeigt die Bildsequenz der Person 224 über die Anzeige 216 an. Die Authentifizierungssoftware ist außerdem dazu ausgebildet, die während der Anzeige der verschiedenen Bilder vom KLHA-Sensor 218 erfassten Hirnaktivitätssignale zu analysieren um festzustellen, ob die ein oder mehreren ersten Hirnaktivitätssignale, die während der Anzeige von Bekanntbildern empfangen wurden, signifikant unähnlich sind zu mehreren zweiten Hirnaktivitätssignalen, die während der Anzeige von Fremdbildern empfangen wurden, wobei Hirnaktivitätssignale, die während der Anzeige von Gesichtsbildern des gleichen Typs erfasst werden, vorzugsweise identisch oder sehr ähnlich sein müssen. Je nach Art der erfassten Hirnaktivitätssignale kann diese Analyse recht unterschiedlich ausfallen. Beispielsweise kann die Analyse eine Bestimmung und einen Vergleich von Amplitude und/oder Amplitudenverlauf über die Zeit ("Signalprofil") eines gemessenen ersten und zweiten Signals umfassen. Bei dem Signal kann es sich um ein elektrisches Signal (gemessen z.B. in Volt) oder ein optisch und durch Bildanalyse erfasstes Signal (gemessen z.B. in mm Pupillendurchmesser bzw. Pupillendurchmesseränderung) handeln. Die Person 224 gilt im zweiten Authentifizierungsverfahren als erfolgreich gegenüber der Authentifizierungssoftware authentifiziert, wenn die Authentifizierungssoftware feststellt, dass die ersten Hirnaktivitätssignale signifikant unterschiedlich sind zu den zweiten Hirnaktivitätssignalen, wobei die Hirnaktivitätssignale, die beim Anzeigen von Bildern des gleichen Typs empfangen wurden, vorzugsweise identisch oder sehr ähnlich sind.

Es ist also nicht in jedem Fall erforderlich, dass zum Beispiel die ersten Hirnaktivitätssignale bei allen Menschen identisch oder nahezu identisch sind. Die Anmelderin hat jedoch festgestellt, dass zumindest eine deutliche Unähnlichkeit der Hirnaktivitätssignale bei Bekanntbildern und Fremdbildern bei praktisch allen Menschen in reproduzierbarer Weise gegeben ist. Somit kann gemäß Ausführungsformen der Erfindung ein Authentifizierungsverfahren bereitgestellt werden, welches die Vorzüge biometrischer Authentifizierungsverfahren mit den Vorzügen von Challenge Response-Authentifizierungsverfahren verbindet.

Das Authentifizierungssystem umfasst eine oder mehrere hardwarebasierte und/oder softwarebasierte Funktion 227. Diese Funktion wird nur dann vom System 200 automatisch ausgeführt oder zur Ausführung an die Person 224 freigegeben, wenn die Person sich im ersten und zweiten Authentifizierungsverfahren erfolgreich authentifiziert. Die Funktion 227 kann z.B. eine Funktion zum Öffnen einer Tür oder eines sonstigen Verriegelungsmechanismus sein.

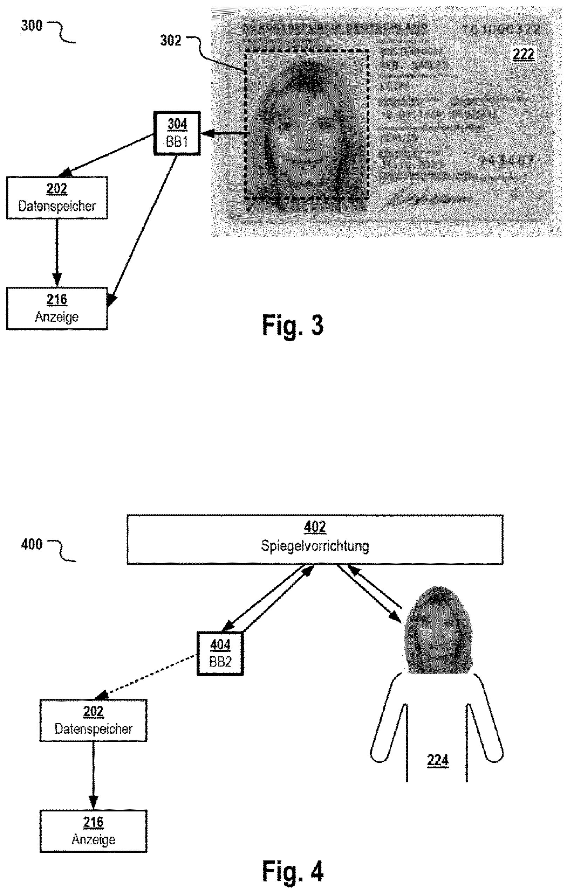

**Figur 3** zeigt ein Authentifizierungssystem 300 auf Basis von Bekanntbildern, die auf Basis von Bildern in Ausweisdokumenten dynamisch während des Authentifizierungsverfahrens erzeugt werden. Beispielsweise kann es sich bei dem Ausweisdokument 222 um einen Personalausweis handeln, der über einen Dokumenteneinzug 220 in ein Terminal eingezogen und dort ausgewertet wird. Das Terminal kann zum Beispiel eine Kamera beinhalten, die das auf dem Dokument 222 abgebildete Gesichtsbild 302 aufnimmt und dieses Bild in einer Kopie oder in mehrfacher Kopie jeweils als Bekanntbild 304 der zu authentifizierenden Person verwendet. Beispielsweise kann das Bild zunächst in einem Datenspeicher 202, zum Beispiel innerhalb der Bilddatenbank 206, gespeichert werden und dann im nächsten Schritt als Bestandteil einer zufällig gewählten Sequenz mehrerer Bekanntbilder und Fremdbilder über die Anzeige 216 angezeigt zu werden. Beispielsweise kann das Bekanntbild 304 ein oder mehrmals dupliziert werden, sodass mehrere Kopien des Bekanntbilds 304 vorhanden sind und eine komplexe Sequenz aus Bekanntbildern und Fremdbildern erzeugt werden kann. Das Authentifizierungssystem 300 verwendet also sowohl eine Bilddatenbank als auch das in einem Ausweisdokument der sich authentifizierenden Person abgedruckten Gesichtsbild, oben für den aktuellen Authentifizierungsprozess eine zufällige Sequenz aus Fremdbildern und Bekanntbildern zu erzeugen, die der Person über die Anzeige angezeigt werden. Falls eine Person unberechtigterweise an den Ausweis einer registrierten, als vertrauenswürdig registrierten Person gelangt ist und eine Maske trägt, die nach dem Bilde des Ausweisgesichtsbildes gefertigt ist, könnte eine solche Person zwar womöglich das erste Authentifizierungsverfahren erfolgreich absolvieren, würde aber am zweiten scheitern.

**Figur 4** zeigt ein Authentifizierungssystem 400 auf Basis von Spiegelbildern. Das System 400 beinhaltet eine Vorrichtung 402 mit einem oder mehreren Spiegeln, die so angeordnet sind, dass die Person, die sich authentifiziert, ihr eigenes Spiegelbild zumindest zeitweise erblickt. Beispielsweise kann die Spiegelvorrichtung 402 einen semi-transparenten Spiegel beinhalten, der dann, wenn das Gesicht der Person 224 von einer bestimmten Lichtquelle beleuchtet wird, dieser Person 224 ihr eigenes Gesichts-Spiegelbild zeigt. Hinter dem semi-transparenten Spiegel ist eine elektronische Digitalanzeige angebracht, die zumindest dann, wenn die Lichtquelle angeschaltet/aktiv ist, ausgeschaltet ist oder nur ein so schwaches Lichtsignal emittiert, dass die Person 224 den angezeigten Inhalt der Digitalanzeige nicht wahrnehmen kann. Die Authentifizierungssoftware ist dazu konfiguriert, die Aktivität der Lichtquelle und die Helligkeit der digitalen Anzeige hinter dem semi-transparenten Spiegels so zu koordinieren, dass dann, wenn auf der Digitalanzeige Fremdbilder angezeigt werden, die Lichtquelle, die das Gesicht der Person 224 bestrahlt, deaktiviert wird und gleichzeitig die digitale Anzeige mit einem angezeigten Fremdbild aktiviert wird. Das Aktivieren der Lichtquelle bei deaktivierter digitaler Anzeige und das Aktivieren der digitalen Anzeige bei deaktivierter Lichtquelle wird von der Authentifizierungssoftware gemäß der für jeden Authentifizierungsvorgang neu erzeugten zufällige Sequenz aus Bekanntbildern und Fremdbildern so orchestriert, dass die Lichtquelle immer dann aktiviert und die digitale Anzeige immer dann deaktiviert wird, wenn der Person ein Bekanntbild (nämlich ihr Spiegelbild) gezeigt werden soll, und dass die Lichtquelle immer dann deaktiviert und die digitale Anzeige immer dann aktiviert wird, wenn der Person über die digitale Anzeige ein Fremdbild angezeigt werden soll.

Das über die Spiegelvorrichtung 402 erzeugte Bekanntbild 404 wird typischerweise direkt über die Spiegelvorrichtung, die als Anzeige 216 für die Bekanntbilder wirkt, angezeigt. Optional kann das Bekanntbild 404 zusätzlich von einer Kamera erfasst und in dem Datenspeicher 202, der die Fremdbilder für die Digitalanzeige 216 umfasst, gespeichert werden.

Unter einer Spiegelvorrichtung 402 wird hierbei ein breites Spektrum von Vorrichtungen zum Spiegeln verstanden, wobei Kameras, digitale Vorrichtungen zum Spiegeln, Displays, Web-Cams und Spiegelfolie ebenfalls zu den Spiegeln gezählt werden.

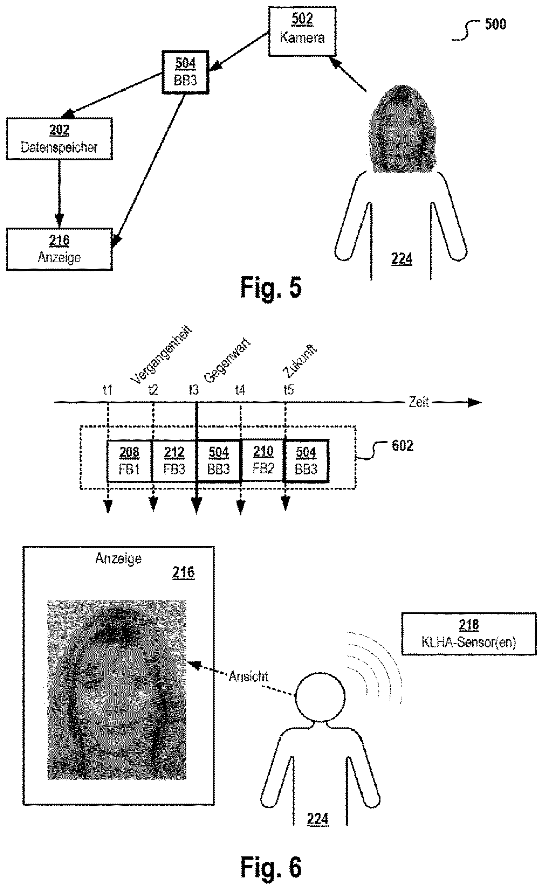

**Figur 5** zeigt ein Authentifizierungssystem 500 auf Basis von Kamerabildern.

Eine Kamera 502 erfasst im Zuge des ersten Authentifizierungsverfahren das Gesichtsbild 504 der einen Person 224, die sich authentifizieren möchte, und zeigt es der Person 224 über die digitale Anzeige 216 an.

Gemäß Ausführungsformen führt die Authentifizierungssoftware nach einer erfolgreichen Authentifizierung der Person 224 im ersten Authentifizierungsverfahren ein zweites Authentifizierungsverfahren durch. Die Authentifizierungssoftware ist dazu konfiguriert, für jede Ausführung des zweiten Authentifizierungsverfahrens eine neue, zufällige Sequenz aus mehreren Bekanntbildern und Fremdbildern zu erzeugen. Beispielsweise kann die Authentifizierungssoftware nach der Erfassung des Bekanntbildes 504 mit der Kamera zunächst eine oder mehrere Kopien des Bekanntbildes erzeugen. Dann liest die Authentifizierungssoftware mehrere Fremdbilder aus einer Bilddatenbank 206 aus und kombiniert die Kopien des Bekanntbildes mit den ausgewählten Fremdbildern zu der neuen Bildsequenz. Die Bilder werden der Person 224 gemäß dieser Sequenz über die Anzeige 216 angezeigt. Währenddessen erfasst der KLHA-Sensor die Hirnaktivitätssignale der Person und übermittelt sie der Authentifizierungssoftware zur weiteren Analyse und Authentifizierung der Person.

Optional kann das erfasste Bekanntbild 504 auch in dem Datenspeicher 202 gespeichert werden, beispielsweise, damit das Bekanntbild 504 als Fremdbild im Zuge der Authentifizierung anderer Personen verwendet werden kann.

**Figur 6** zeigt eine Sequenz 602 von angezeigten Bekanntbildern und Fremdbildern, die von der Authentifizierungssoftware für einen aktuellen Authentifizierungsprozess der Person 224 zufällig erzeugt wurde. Die Sequenz beginnt mit einem ersten Fremdbild 208. Es folgt ein weiteres Fremdbild 212, dann ein Bekanntbild 504, dann ein anderes Fremdbild 210 und schließlich wieder ein Bekanntbild 504.

Die Authentifizierungssoftware ist dazu ausgebildet, gemäß dieser zufällig erzeugten Sequenz die einzelnen Bilder nacheinander über die Anzeige 216 der Person 224 anzuzeigen. Beispielsweise kann die Authentifizierungssoftware dazu konfiguriert sein, jedes der Bilder 208, 212, 504, 210, 504 zwei Sekunden lang anzuzeigen und nach Ablauf der zwei Sekunden ohne Pause sofort das nächste Bild der Sequenz anzuzeigen. Beispielsweise zeigte die Authentifizierungssoftware an dem Zeitpunkt t1 das Fremdbild 208 an, 2 Sekunden später, zum Zeitpunkt t2, ein weiteres Fremdbild einer anderen fremden Person, und wiederum 2 Sekunden später, zum Zeitpunkt t3, ein Bekanntbild 504 der Person 224, die sich aktuell authentifizieren möchte. Die Anzeige 216 zeigt das zum aktuellen Zeitpunkt (zwischen t3 und t4) auf der Anzeige ausgegebene Bekanntbild der Person. Wenn nach dem Zeitpunkt t3 weitere 2 Sekunden verstrichen sein werden, wird das Bekanntbild 504 durch das weitere Fremdbild 210 ersetzt werden, und nach weiteren 2 Sekunden zum Zeitpunkt t5 durch das Bekanntbild 504. Während die Bildsequenz 602 der Person 224 angezeigt wird, wird eine entsprechende Sequenz von ersten und zweiten Hirnaktivitätssignalen vom KLHA-Sensor aufgezeichnet und an die Authentifizierungssoftware zur weiteren Analyse und zur Authentifizierung der Person weitergeleitet.

Die Anzeigedauer der jeweiligen Bilder liegt typischerweise im Bereich zwischen einer und 5 Sekunden, vorzugsweise im Bereich 2-4 Sekunden. Es ist möglich, dass Pausen zwischen dem Anzeigen verschiedener Bilder eingeführt werden, die typischerweise kürzer sind als 5 Sekunden.

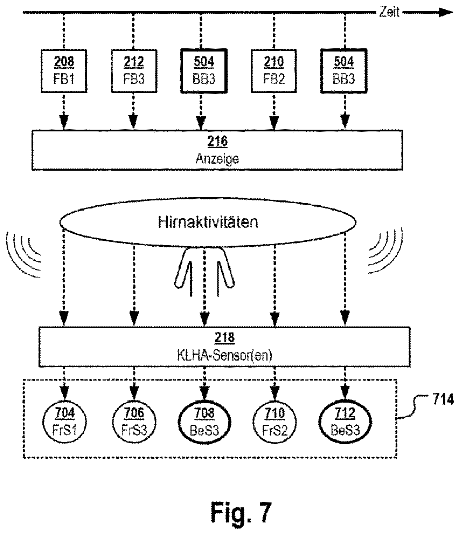

**Figur 7** zeigt eine Sequenz 714 von ersten und zweiten Hirnaktivitätssignalen, die durch Anzeige der Bildsequenz 602 in der Person 224 hervorgerufen werden.

Die Anzeige des Fremdbilds 208 erzeugt ein zweites Hirnaktivitätssignal 704. Die Anzeige des Fremdbilds 212 erzeugt ein zweites Hirnaktivitätssignal 706. Die Anzeige des auf das Bild 212 folgenden Bekanntbildes 504 erzeugt ein erstes Hirnaktivitätssignal 708. Die Anzeige des Fremdbilds 210 erzeugt ein zweites Hirnaktivitätssignal 710. Die Anzeige des auf das Bild 210 folgenden Bekanntbildes 504 erzeugt ein erstes Hirnaktivitätssignal 712. Die gestrichelten Pfeile repräsentieren hier nicht Zeitpunkte, sondern Zeitfenster, zum Beispiel 2 Sekunden lange Zeitfenster, während welcher die entsprechenden Bilder angezeigt und die zu diesen korrespondierenden Hirnaktivitätssignale gemessen werden.

Wenn die Person 224 keine Maske trägt bzw. nicht versucht, sich mit einem Ausweis mit einem gemorphten Bild zu authentifizieren, so ist zu erwarten, dass alle ersten Hirnaktivitätssignale 708, 712 einander sehr ähnlich oder identisch sind, dass alle zweiten Hirnaktivitätssignale 704, 706, 710 einander sehr ähnlich oder identisch sind, und dass die ersten und zweiten Hirnaktivitätssignale signifikant unterschiedlich sind. In diesem Fall behandelt die Authentifizierungssoftware die Person 224 als im zweiten Authentifizierungsverfahren erfolgreich authentifiziert. Andernfalls behandelt die Authentifizierungssoftware die Person als nicht authentifiziert und verweigert den begehrten Zugang bzw. die begehrte Aktion.

Ein praktisches Anwendungsszenario gemäß einer Ausführungsform der Erfindung wird im Folgenden beschrieben: Beispielsweise wird das Authentifizierungsverfahren zur Authentifizierung eines Mitarbeiters beim Betreten des Firmengeländes verwendet. In der Firma arbeiten 500 Mitarbeiter M1-M500. In einer Datenbank sind für jeden Mitarbeiter zehn Gesichtsbilder des jeweiligen Mitarbeiters hinterlegt, also für den Mitarbeiter M1 die Gesichtsbilder B_{M1.1}, B_{M1.2}, ..., BM_{1.10}, für Mitarbeiter M2 die Gesichtsbilder BM_{2.1}-BM_{2.10}. Der erste Mitarbeiter M1 möchte sich authentifizieren und stößt ein Authentifizierungsverfahren gemäß Ausführungsformen der Erfindung an.

Zunächst wird ein erstes Authentifizierungsverfahren durchgeführt, das z.B. auf der Eingabe eines Passworts und Nutzer-ID, auf der Erfassung biometrischer Daten und/oder auf dem Auslesen von Berechtigungsnachweisen aus einem ID-Dokument beruhen kann.

Im Falle einer erfolgreichen Authentifizierung des Mitarbeiters M1 im ersten Verfahren wählt die Authentifizierungssoftware im Zuge eines zweiten Authentifizierungsverahrens aus der Bilddatenbank eine zufällige Sequenz aus zwei Bekanntbildern und vier Fremdbildern aus, z.B. gemäß der Sequenz B_{M44.7}, B_{M102.2}, B_{M1.3}, B_{M15.1}, B_{M1.8}, B_{M16.7.} In dieser Sequenz sind die Bilder B_{M1.3} und B_{M1.8} "Bekanntbilder" bezüglich Mitarbeiter M1. Die Bilder der anderen Mitarbeiter M44, M102, M15 und M16 sind Fremdbilder für Mitarbeiter M1, können jeweils aber als Bekanntbilder dienen, wenn sich diese anderen Mitarbeiter authentifizieren. Sofern es sich bei dem sich gerade authentifizierenden Mitarbeiter tatsächlich um Mitarbeiter M1 handelt, ist also folgende Signalsequenz zu erwarten, wobei die Kürzel "HAS1" für "erstes Hirnaktivitätssignal" und HAS2" für "zweites Hirnaktivitätssignal" verwendet werden: HAS2, HAS2, HAS1, HAS2, HAS1, HAS2. Wenn die Abfolge der erfassten ersten und zweiten Hirnaktivitätssignale den gemäß der Sequenz der gezeigten Gesichtsbilder erwarteten Signalabfolge entspricht, hat sich der Mitarbeiter M1 erfolgreich authentifiziert.

Danach möchte sich Mitarbeiter M55 authentifizieren und stößt ein weiteres Authentifizierungsverfahren an, dessen erster Teil verläuft wie oben für M1 beschrieben, also z.B. auf Basis eines Passworts, ID-Dokuments oder biometrischer Daten. Im Zuge des zweiten Authentifizierungsverfahrens, das M55 angestoßen hat, wählt die Authentifizierungssoftware aus der Bilddatenbank eine zufällige Sequenz aus zwei Bekanntbildern und vier Fremdbildern aus, z.B. gemäß der Sequenz B_{M55.2}, B_{M14.7}, B_{M55.6}, B_{M232.2}, B_{M15.1}, B_{M46.7}. In dieser Sequenz sind die Bilder B_{M55.2} und B_{M55.6} "Bekanntbilder" bezüglich Mitarbeiter M55. Die Bilder der anderen Mitarbeiter M14, M232, M15 und M46 sind Fremdbilder für Mitarbeiter M55, können jeweils aber als Bekanntbilder dienen, wenn sich diese anderen Mitarbeiter authentifizieren. Sofern es sich bei dem sich gerade authentifizierenden Mitarbeiter tatsächlich um Mitarbeiter M55 handelt, ist also folgende Signalsequenz zu erwarten: HAS1, HAS2, HAS1, HAS2, HAS2, HAS2.

**Figur 8** zeigt ein Schema einer Machine-Learning-Software zur Analyse und Klassifizierung der Hirnaktivitätssignale, um festzustellen, ob und wie sehr sich erste und zweite Hirnaktivitätssignale gegenseitig und untereinander ähneln. Es hat sich herausgestellt, dass neuronale Netze, insbesondere rekurrente neuronale Netze 804, besonders geeignet sind, um die Ähnlichkeit von Hirnaktivitätssignalen zu bestimmen.

Beispielsweise kann die Machine-Learning-Software und/oder die Authentifizierungssoftware neben dem neuronalen Netz 804 ein Vorverarbeitung Modul 802 und ein Auswertungsmodul 806 besitzen. Das Vorverarbeitungsmodul 802 ist dazu konfiguriert, das von dem KLHA-Sensor 218 erfasste Signal so zu repräsentieren, dass die in dem Signal enthaltene Information von der Authentifizierungssoftware und/oder dem neuronalen Netz ausgewertet werden kann. Beispielsweise kann das Profil zeitlicher Änderungen von Amplitude (gemessene Ladungsänderungen und/oder von Ladungsumverteilungen in der Nähe eines elektrischen Sensors) und optional auch Frequenz des von einem elektrischen Sensor gemessenen Signals als mehrdimensionaler Vektor dargestellt werden, wobei beispielsweise der Vektor eine Vielzahl von Elementen (z.B. 200 Elemente für einen Vektor, der Signaleigenschaften eines über zwei Sekunden gemessenen Signals mit einer zeitlichen Auflösung von 10 ms spezifiziert) umfassen kann, wobei pro Element ein zu diesem Zeitpunkt gültige Amplitudenwert und eine zu diesem Zeitpunkt gemessene Frequenz in den Vektor enthalten ist. Falls es sich bei dem KLHA-Sensor um einen optischen Sensor handelt, kann das Vorverarbeitungsmodul 802 dazu ausgebildet sein, aus dem von dem optischen Sensor erfassten Gesichtsbild bzw. Augenbereichsbild mehrere Merkmale zu extrahieren wie zum Beispiel Pupillendurchmesser und/oder zeitliches Profil von Pupillendurchmesseränderungen, und diese Merkmale in geeigneter Form zu repräsentieren, zum Beispiel ebenfalls als Vektor mit mehreren numerischen Werten definierte Bedeutung. In einer Trainingsphase hat die Machine-Learning-Software 800 auf Basis eines annotierten Trainingsdatensatzes gelernt, zu erkennen, welche Variabilität der Signaleigenschaften akzeptiert werden kann und muss, ohne dass zwei verglichene Hirnaktivitätssignale als signifikant unähnlich angesehen werden. Biometrische Signale sind niemals 100 % identisch. Es ist aber möglich, durch das Training einer Machine-Learning Software auf einen Datensatz mehrerer Hirnaktivitätssignale, die einmal durch Betrachtung von Bekanntbildern und ein anderes Mal durch Betrachtung von Fremdbildern hervorgerufen wurden, und die entsprechend annotiert sind, zu erreichen, dass die trainierte Machine-Learning Software zuverlässig erkennen kann, ob Abweichungen in Eigenschaften der Hirnaktivitätssignale relevant oder irrelevant im Hinblick auf die Frage sind, ob hier ein Bekanntbild oder ein Fremdbild betrachtet wird.

Das Auswertungsmodul 806 aggregierten die Ausgaben der Neuronen der Ausgabeschicht des neuronalen Netzes und bereitet das Ergebnis (authentifiziert oder nicht) so auf, dass dies vom Empfänger interpretiert werden kann. Beispielsweise kann das Ergebnis als natürlichsprachlicher Text akustisch oder optisch an einen Menschen ausgegeben werden. Alternativ oder zusätzlich kann das Ergebnis der Authentifizierung auch an eine Software oder Hardwarekomponente ausgegeben werden, die in Abhängigkeit von dem Authentifizierungs-Ergebnis beispielsweise eine bestimmte Hardwarefunktion oder Softwarefunktion freigibt oder nicht.

Gemäß einer anderen, hier nicht dargestellten Ausführungsform dient ein Convolutional Neural Network (CNN) zur Klassifikation der Hirnaktivitätssignale in ähnliche und unähnliche Signale. Es kann z.B. eine erste Faltungsschicht mit sechs Feature-Maps, deren Kernelgröße 5 ist, beinhalten. Die erste Faltungsschicht ist eine erste Maxpooling-Schicht mit der Skalengröße 2. Eine zweite Faltungsschicht mit 12 Merkmalspunkten hat die Kernelgröße 3. Außerdem beinhaltet das neuronale Netz eine zweite Maxpooling-Schicht mit der gleichen Skalengröße wie die erste Maxpooling-Schicht. Schließlich gibt es eine voll verbundene Schicht mit 108 Merkmalspunkten, die die Score-Schätzung der Eingabe berechnet. Im Trainingsstadium werden Lernrate, Lossgröße und Lernepoche z.B. auf 1, 100 und 500 gesetzt.

**Figur 9** zeigt einen Plot 900, also ein Diagramm, mit einem ersten Hirnaktivitätssignal 902, das während der Betrachtung eines Bekanntbildes von einem elektrischen Sensor gemessen wurde, und mit einem zweiten Hirnaktivitätssignal 904, dass während der Betrachtung eines Fremdbildes von einem elektrischen Sensor gemessen wurde. Bei den hier gezeigten Hirnaktivitätssignalen handelt es sich um Hirnstromsignale, die kontaktlos in Form von Ladungsänderungen und/oder von Ladungsumverteilungen in der Nähe des elektrischen Sensors erfasst wurden.

Generell unterscheidet man in der Neurologie bei Hirnstrommessungen unterschiedliche Frequenzbänder:
- Das Sub-δ-Band mit einem Frequenzbereich von 0.15 bis 0.5 Hz,
- das δ-Band mit einem Frequenzbereich von 0.5 bis 3.5 Hz,
- das θ -(Theta)-Band mit einem Frequenzbereich von 3.5 bis 8 Hz,
- das α-Band mit einem Frequenzbereich von 8 bis 13 Hz,
- das β-Band mit einem Frequenzbereich von 13 bis 30 Hz, sowie Frequenzanteile oberhalb des β-Bandes (also über 30 Hz).

Gemäß Ausführungsformen werden die Hirnaktivitätssignale in einem oder mehreren dieser Bänder gemessen.

Das α-Band liefert in manchen Ausführungsformen zusätzliche Informationen über den Wachheitszustand einer Person, Frequenzanteile oberhalb dieses α -Bandes (β-Band und darüber) können Informationen über Beeinträchtigung der mentalen Funktionen durch Drogen oder zentral wirksame Medikamente liefern, und das direkt unterhalb des α-Bands angrenzende Theta-Band liefert Informationen anderer Art, die u.a. mit dem Wachheitszustand, eingenommenen Substanzen oder Krankheiten korrelieren können. Spezifische Stimuli, z.B. Gesichtsbilder, lösen oftmals Hirnstromsignale im theta-Band und weiteren Bändern aus. Gemäß Ausführungsformen der Erfindung wird eine "Multi-Band"-Signalanalyse, d.h. eine Analyse der Eigenschaften von Signalen über einen sehr breiten Frequenzbereich, durchgeführt.

Gemäß weiteren Ausführungsformen werden die Hirnaktivitätssignale ohne Differenzierung nach einzelnen Bändern gemessen. Vielmehr wird die Signalamplitude über ein weites Frequenzband, das im Wesentlichen nur durch die Sensitivität des elektrischen Sensors bestimmt ist, erfasst.

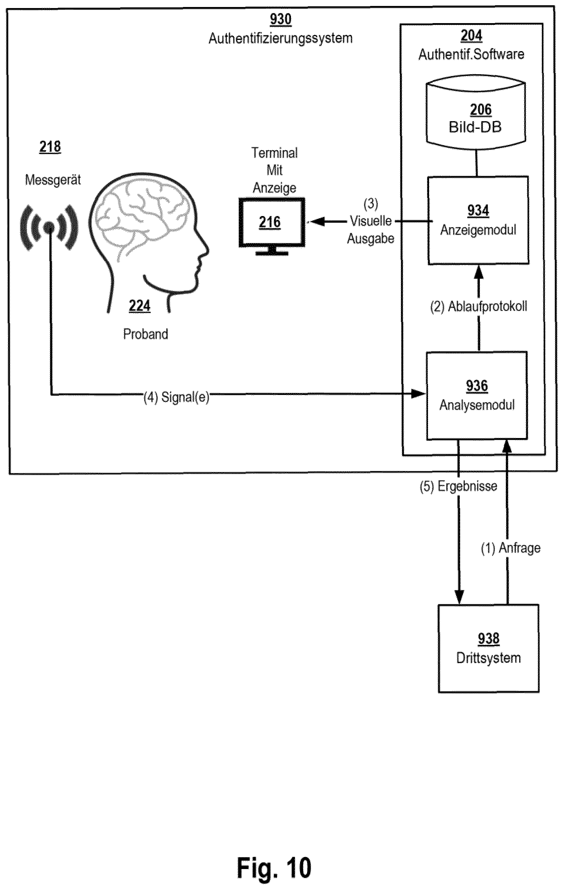

**Figur 10** zeigt ein Blockdiagramm eines weiteren Authentifizierungssystems 930. Der Aufbau des Authentifizierungssystems 930 entspricht im Wesentlichen dem Aufbau des Authentifizierungssystems 200, das in Figur 2 beschrieben ist. Die Person 224 ist nicht Bestandteil des Authentifizierungssystems. Figur 10 zeigt, wie das Authentifizierungssystem von einem Drittsystem 938 verwendet werden kann. Beispielsweise kann es sich bei dem Drittsystem um eine Software zur Kontrolle einer Schließanlage, zum Beispiel eine Tür oder eines Tors, handeln. Der Nutzer 224, hier als Proband bezeichnet, versucht, sich bei dem Drittsystem zu authentifizieren, um die Tür zu öffnen. Beispielsweise gibt der Proband 224 seinen Namen in eine Software des Drittsystems ein oder das Drittsystem liest eine Mitarbeiter-ID aus einem Identitätsdokument, zum Beispiel einem Mitarbeiterausweis, aus. Daraufhin sendet das Drittsystem eine Anfrage an die Authentifizierungssoftware 204. Die Anfrage kann beispielsweise den eingegebenen Namen oder die ausgelesenen Mitarbeiter-ID beinhalten. Die Authentifizierungssoftware kann sodann in einem ersten Authentifizierungsverfahren, das z.B. in dem Analysemodul 936 implementiert sein kann, den Probanden z.B. mittels Gesichtserkennung authentifizieren oder sie fordert den Probanden über eine von dem Analysemodul in Interoperation mit dem Anzeigemodul 934 erzeugte GUI auf, ein Passwort einzugeben und sich dadurch zu authentifizieren.

Im Falle einer erfolgreichen Authentifizierung führt die Authentifizierungssoftware ein zweites Authentifizierungsverfahren durch. Hierbei erzeugt das Analysemodul mithilfe eines Zufallsgenerators eine neue, zufällige Sequenz mehrerer Bekanntbilder und mehrerer Fremdbilder. Hierzu greift es auf eine Bilderdatenbank 206 zu, in welcher mehrere mit dem Profil des Probanden 224 verknüpft gespeicherte Bekanntbilder und mehrere Fremdbilder in Form von Bildern bekannter oder fremder Gesichter hinterlegt sind. Anhand des eingegebenen Namens bzw. der Mitarbeiter-ID kann die Authentifizierungssoftware erkennen, welche der Bilder in der Datenbank 206 als Bekanntbilder und welche als Fremdbilder verwendet werden können. Nachdem die Authentifizierungssoftware eine entsprechende Bildsequenz definiert hat, steuert ein Anzeigemodul 934 der Authentifizierungssoftware die Anzeige der Bilder auf der Anzeige 216 so, dass die Bilder chronologisch gemäß der zufälligen Sequenz dem Nutzer 224 angezeigt werden. Vorzugsweise wird hierbei durch weitere Module der Software 204 in Interoperation mit einer Kamera sichergestellt, dass der Proband 224 den Blick auch wirklich auf die Anzeige 216 richtet, während die Bildsequenz angezeigt wird. Während die Bildsequenz angezeigt wird, erfasst der KLHA-Sensor 218, hier als Messgerät bezeichnet, die von der Person 224 ausgesendeten Hirnaktivitätssignale. Bei diesen Signalen kann es sich um Hirnstromsignale handeln, die die neuronalen Prozesse im Hirn des Probanden direkt wiedergeben, oder um Eigenschaften der Augen, insbesondere der Pupille und der Augenlider, die indirekt die neuronalen Prozesse während des Betrachtens verschiedener Bilder widerspiegeln. Die vom Sensor 218 erfassten Signale werden an ein Analysemodul 936 der Authentifizierungssoftware 204 weitergegeben, wo diese dann aufbereitet und analysiert werden. Beispielsweise kann es sich bei dem Analysemodul 936 um eine Machine-Learning-Software 800 handeln wie diese mit Referenz auf Figur 8 beschrieben ist. In Abhängigkeit davon, ob die Hirnaktivitätssignale, die beim Betrachten von Fremdbildern empfangen wurden, signifikant unterschiedlich sind von den Hirnaktivitätssignalen, die beim Betrachten von Bekanntbildern empfangen wurden, entscheidet das Analysemodul 936, ob der Proband 224 sich erfolgreich gegenüber der Authentifizierungssoftware 204 authentifiziert hat. Das Ergebnis dieser Entscheidung wird an das Drittsystem 938 zurückgegeben. Das Drittsystem führt nun seine Funktion in Abhängigkeit dieses Ergebnisses aus. Beispielsweise öffnet eine Kontrollsoftware einer Schließanlage dem Nutzer 224 die Schließanlage, falls sich der Nutzer erfolgreich authentifiziert hat. Andernfalls unterbleibt die Öffnung.

**Figur 11** zeigt einen Plot 950 mit einem ersten Hirnaktivitätssignal 952 in Form eines Pupillendurchmesserprofils, das während der Betrachtung eines Bekanntbildes (hier: Bild des Gesichts des Betrachters) von einem optischen Sensor gemessen wurde, und mit einem zweiten Hirnaktivitätssignal 904 in Form eines zweiten Pupillendurchmesserprofils, dass während der Betrachtung eines Fremdbildes von dem optischen Sensor gemessen wurde. Die beiden Pupillendurchmesserprofile bzw. die jeweiligen Durchmesser wurden durch automatische Bildanalyse der erfassten Bilder ermittelt. Eine Betrachtung eines Bekanntbildes führt im Gegensatz zu der Betrachtung eines Fremdbildes bei der hier betrachteten Person zu einer deutlichen Pupillendurchmesservergrößerung innerhalb der ersten 1000 ms.

Das erfindungsgemäße Authentisierungsverfahren ist nicht auf die beschriebenen Ausführungsbeispiele beschränkt. Es kann mit weiteren Authentisierungsverfahren kombiniert werden, um eine sichere und bequeme Authentisierung zu ermöglichen. Eine Kombination mehrere biometrischer Verfahren ist vorteilhaft um eine multimodale Authentifizierung zu gewährleisten.

Vorteilhafte Ausführungsformen umfassen beispielsweise die folgenden Merkmale:
1. Computerimplementiertes Verfahren zur Authentifizierung einer Person (224) als Vertrauensperson, umfassend:
   - Empfang (102) von Daten (223) der Person durch eine Authentifizierungssoftware (204);
   - Auswertung (104) der empfangenen Daten durch die Authentifizierungssoftware, um die Person als die Vertrauensperson mittels eines ersten Authentifizierungsverfahrens zu authentifizieren;
   - Ermittlung (104) von einem oder mehreren Bekanntbildern (504) und einem oder mehreren Fremdbildern (208, 212, 210) durch die Authentifizierungssoftware, wobei ein Bekanntbild ein Bild ist mit einem Bildmotiv, das der Person bekannt ist, sofern es sich bei der Person um die Vertrauensperson handelt, wobei ein Fremdbild ein Bild ist mit einem Bildmotiv, das der Person nicht bekannt ist oder signifikant weniger bekannt ist als ein Bekanntbild;
   - Anzeige (108) mehrerer Bilder auf einer Anzeige (216), wobei die angezeigten Bilder zumindest eines der ermittelten Bekanntbilder und zumindest eines der ermittelten Fremdbilder enthalten;
   - während die mehreren Bilder der Person angezeigt werden, Erfassung (110) von ersten (708, 712, 902) und zweiten (704, 706, 710, 904) Hirnaktivitätssignalen, die in der einen Person durch die Betrachtung der Bilder jeweils ausgelöst werden, durch einen kontaktlosen Sensor (218) zur Sensierung von Hirnaktivitäten - im Folgenden als KLHA-Sensor (218) bezeichnet, wobei erste Hirnaktivitätssignale erfasst werden, während der Person ein Bekanntbild (304, 404, 504) angezeigt wird, und wobei zweite Hirnaktivitätssignale erfasst werden, während der Person ein Fremdbild angezeigt wird;
   - Durchführung (112) eines zweiten Authentifizierungsverfahrens durch die Authentifizierungssoftware, um die Person als die Vertrauensperson zu authentifizieren, wobei das zweite Authentifizierungsverfahren umfasst eine Analyse der erfassten ersten und zweiten Hirnaktivitätssignale;
   - Behandlung der Person durch die Authentifizierungssoftware als authentifiziert nur dann, wenn diese sich sowohl im ersten als auch im zweiten Authentifizierungsverfahren erfolgreich authentifiziert hat.
2. Verfahren nach Anspruch 1, wobei das zweite Authentifizierungsverfahren die Person als erfolgreich authentifiziert behandelt, wenn die ersten Hirnaktivitätssignale sich signifikant unterscheiden von den zweiten Hirnaktivitätssignalen.
3. Verfahren nach einem der vorigen Ansprüche, wobei die empfangenen Daten der Person biometrische Daten der Person sind und wobei das erste Authentifizierungsverfahren ein biometrisches Authentifizierungsverfahren ist, insbesondere ein Verfahren der automatischen Gesichtserkennung und/oder ein Verfahren basierend auf Fingerabdruckdaten oder Irisbildern.
4. Verfahren nach Anspruch 3, wobei das erste Authentifizierungsverfahren ein Verfahren der automatischen Gesichtserkennung ist, wobei die Durchführung des ersten Authentifizierungsverfahrens eine Erfassung eines aktuellen Gesichtsbildes der Person durch einen optischen Sensor umfasst, ferner umfassend:
   - Verwendung des erfassten aktuellen Gesichtsbildes der Person als das Bekanntbild oder als eines der Bekanntbilder durch das zweite Authentifizierungsverfahren.
5. Verfahren nach einem der vorigen Ansprüche,
   - wobei der KLHA-Sensor integriert und/oder operativ gekoppelt ist an ein Terminal; und/oder
   - wobei die Authentifizierungssoftware integriert und/oder operativ gekoppelt ist an das Terminal.
6. Verfahren nach einem der vorigen Ansprüche, ferner umfassend:
   - für jeden Authentifizierungsvorgang der Person mit dem zweiten Authentifizierungsverfahren, Erzeugung einer zufälligen Reihung (602) der mehreren für die Person ermittelten Bilder durch die Authentifizierungssoftware und Durchführen des Anzeigens der mehreren Bilder in einer chronologischen Sequenz entsprechend der zufälligen Reihung;
   - wobei die Analyse der erfassten ersten und zweiten Hirnaktivitätssignale erfolgt unter Berücksichtigung der chronologischen Sequenz.
7. Verfahren nach Anspruch 6, wobei die zufällige Reihung als Challenge dient und wobei die Analyse der erfassten Hirnaktivitätssignale eine Prüfung umfasst, ob die zeitliche Reihenfolge der ersten und zweiten Hirnaktivitätssignale die in der Challenge codierte Reihenfolge von Bekanntbildern und Fremdbildern widergibt.
8. Verfahren nach einem der vorigen Ansprüche, wobei die Authentifizierungssoftware die Person im zweiten Authentifizierungsverfahren nur dann als erfolgreich authentifiziert behandelt, wenn neben einer signifikanten Unterschiedlichkeit der ersten und zweiten Hirnaktivitätssignale folgende weitere Kriterien zutreffen:
   - alle ersten Hirnaktivitätssignale, die je während des Anzeigens eines der Bekanntbilder erfasst werden, sind identisch oder hinreichend ähnlich zueinander;
   - alle zweiten Hirnaktivitätssignale, die je während des Anzeigens eines der Fremdbilder erfasst werden, sind identisch oder hinreichend ähnlich zueinander.
9. Verfahren nach einem der vorigen Ansprüche,
   - wobei die Ermittlung der ein oder mehreren Bekanntbilder für die Person beinhaltet eine Erfassung von einem oder mehreren Gesichtsbildern der Person durch eine Kamera; und/oder
   - wobei die Ermittlung der ein oder mehreren Bekanntbilder für die Person beinhaltet eine Erfassung von einem oder mehreren Bildern eines auf einem Identitätsdokument der Person abgebildeten Gesichtsbilds der Person durch eine Kamera; und/oder
   - wobei die Ermittlung der ein oder mehreren Bekanntbilder für die Person beinhaltet eine Identifikation der Bekanntbilder und/oder Fremdbilder in einer Bilddatenbank; und/oder
   - wobei die Ermittlung der ein oder mehreren Fremdbilder für die Person beinhaltet eine Identifikation der Bekanntbilder und/oder Fremdbilder in einer Bilddatenbank.
10. Verfahren nach einem der vorigen Ansprüche, wobei der KLHA -Sensor ein elektrischer Sensor ist.
11. Verfahren nach Anspruch 10,
   - wobei der elektrische Sensor dazu ausgebildet ist, die ersten und/oder zweiten Hirnaktivitätssignale durch Messung von Ladungsänderungen und/oder von Ladungsumverteilungen zu erfassen; und/oder
   - wobei der elektrische Sensor dazu ausgebildet ist, die Amplitude der ersten und/oder zweiten Hirnaktivitätssignale und/oder die Änderung der Amplitude während eines Zeitintervalls zu erfassen.
12. Verfahren nach einem der vorigen Ansprüche,
   - wobei die ersten Hirnaktivitätssignale identisch oder ähnlich sind zu den Hirnaktivitätssignalen gemäß Kurve 902 in Figur 9; und/oder
   - wobei die zweiten Hirnaktivitätssignale identisch oder ähnlich sind zu den Hirnaktivitätssignalen gemäß Kurve 904 in Figur 9.
13. Verfahren nach einem der vorigen Ansprüche 1-9, wobei der KLHA-Sensor ein optischer Sensor, insbesondere eine IR-Kamera oder IR-Videokamera oder eine Kamera oder Videokamera für Licht im sichtbaren Wellenlängenbereich ist.
14. Verfahren nach Anspruch 13, wobei der KLHA-Sensor dazu ausgebildet ist, Bilder oder Bildsequenzen des Gesichts oder von Teilen des Gesichts der einen Person zu erfassen, die die ersten und/oder zweiten Hirnaktivitätssignale enthalten, wobei die Analyse der ersten und zweiten Hirnaktivitätssignale durch die Authentifizierungssoftware eine automatische Bildanalyse der Bilder oder Bildsequenzen umfasst, wobei die Bildanalyse umfasst:
   - Bestimmung eines Pupillendurchmessers der einen Person; und/oder
   - Bestimmung eines Pupillendurchmesseränderungsprofils der einen Person.
15. Verfahren nach einem der vorigen Ansprüche 13-14,
   - wobei die ersten Hirnaktivitätssignale identisch oder ähnlich sind zu den Hirnaktivitätssignalen gemäß Kurve 952 in Figur 11; und/oder
   - wobei die zweiten Hirnaktivitätssignale identisch oder ähnlich sind zu den Hirnaktivitätssignalen gemäß Kurve 954 in Figur 11.
16. Verfahren nach einem der vorigen Ansprüche 13-15, wobei die Analyse (108) der erfassten Hirnaktivitätssignale umfasst:
   - Bildverarbeitung zur Erkennung eines Pupillendurchmessers; und/oder
   - Korrelation von Eigenschaften der ersten und zweiten Hirnaktivitätssignale mit den Zeiten, an welchen Bekanntbilder und Fremdbilder angezeigt wurden.
17. Verfahren nach einem der vorigen Ansprüche, wobei die Analyse (108) ausschließlich auf Basis der ersten und zweiten Hirnaktivitätssignalen und auf Basis der Anzeigesequenz der Bekanntbilder und Fremdbilder und optional zusätzlich auf Basis typisierter (nicht personen-individuell erhobener) Referenzdaten durchgeführt wird.
18. Verfahren nach einem der vorigen Ansprüche, wobei das Verfahren außerdem die Löschung der erfassten Hirnaktivitätssignalen nach dem zweiten Authentifizierungsvorgang umfasst.
19. Authentifizierungssystem (200, 930) zur Authentifizierung einer Person (224) als Vertrauensperson, umfassend:
   - eine Schnittstelle (219) zum Empfang von Daten (223) der Person;
   - eine Authentifizierungssoftware (204, 936);
   - eine Anzeige (216);
   - einen kontaktlosen Sensor (218) zur Sensierung von Hirnaktivitäten - im Folgenden als KLHA-Sensor bezeichnet;
   wobei das Authentifizierungssystem konfiguriert ist zum:
   - Empfang (102) der Daten (233) der Person (224) durch die Authentifizierungssoftware über die Schnittstelle (219);
   - Auswertung (104) der empfangenen Daten durch die Authentifizierungssoftware, um die Person als die Vertrauensperson mittels eines ersten Authentifizierungsverfahrens zu authentifizieren;
   - Ermittlung (106) von einem oder mehreren Bekanntbildern und einem oder mehreren Fremdbildern durch die Authentifizierungssoftware, wobei ein Bekanntbild ein Bild ist mit einem Bildmotiv, das der Person bekannt ist, sofern es sich bei der Person um die Vertrauensperson handelt, wobei ein Fremdbild ein Bild ist mit einem Bildmotiv, das der Person nicht bekannt ist oder signifikant weniger bekannt ist als ein Bekanntbild;
   - Anzeige (108) mehrerer Bilder (208, 212, 504, 210, 404, 304) auf der Anzeige, wobei die angezeigten Bilder zumindest eines der ermittelten Bekanntbilder und zumindest eines der ermittelten Fremdbilder enthalten;
   - während die mehreren Bilder der Person angezeigt werden, Erfassung (110) von ersten (708, 712, 902) und zweiten (704, 706, 710, 904) Hirnaktivitätssignalen, die in der einen Person durch die Betrachtung der Bilder jeweils ausgelöst werden, durch den KLHA-Sensor (218), wobei erste Hirnaktivitätssignale erfasst werden, während der Person ein Bekanntbild (304, 404, 504) angezeigt wird, und wobei zweite Hirnaktivitätssignale erfasst werden, während der Person ein Fremdbild angezeigt wird;
   - Durchführung (112) eines zweiten Authentifizierungsverfahrens durch die Authentifizierungssoftware, wobei das zweite Authentifizierungsverfahren eine Analyse (108) der erfassten ersten und zweiten Hirnaktivitätssignale umfasst;
   - Behandlung (114) der Person durch die Authentifizierungssoftware als authentifiziert nur dann, wenn diese sich sowohl im ersten als auch im zweiten Authentifizierungsverfahren erfolgreich authentifiziert hat.
20. Authentifizierungssystem nach Anspruch19, wobei der KLHA-Sensor ausgewählt ist aus einer Gruppe umfassend:
   - Elektrischer Sensor zur Erfassung eines Hirnsignals ohne direkten Kontakt des Kopfes mit dem elektrischen Sensor;
   - optischer Sensor.
21. Authentifizierungssystem nach Anspruch 19 oder20, wobei die Schnittstelle ausgewählt ist aus einer Gruppe umfassend:
   - Kamera, die positioniert ist zum Erfassen eines Gesichtsbildes oder Irisbildes der Person;
   - Einen Fingerabdrucksensor;
   - Ein Lesegerät zum Lesen von Daten aus einem ID-Dokument der Person.
22. Authentifizierungssystem nach einem der Ansprüche 19-21, ferner umfassend ein Terminal, wobei die Anzeigevorrichtung Bestandteil des Terminals ist und wobei das Terminal insbesondere ein Flughafenterminal, ein Grenzkontrollterminal, ein Terminal zur Kontrolle des Zutritts zu einem geschützten geographischen Bereich ist.

### Bezugszeichenliste

- 102-114: Schritte
- 200: Authentifizierungssystem
- 201: Terminal
- 202: Datenspeicher
- 204: Authentifizierungssoftware
- 206: Bilddatenbank
- 208: Gesichtsbild (Fremdbild)
- 210: Gesichtsbild (Fremdbild)
- 212: Gesichtsbild (Fremdbild)
- 214: Gesichtsbild (Fremdbild)
- 216: Anzeige
- 218: KLHA-Sensor(en)
- 219: Schnittstelle Personendaten
- 220: Dokumenteneinzug
- 222: Dokument
- 223: personenbezogene Daten
- 224: Person
- 226: Prozessor(en)
- 227: Funktion(en)
- 300: Authentifizierungssystem
- 302: Portraitbild auf Dokument
- 304: Gesichtsbild (Bekanntbild)
- 400: Authentifizierungssystem
- 402: Spiegelvorrichtung
- 404: Gesichtsbild (Bekanntbild)
- 500: Authentifizierungssystem
- 502: Bilderfassungseinheit (Kamera)
- 504: Gesichtsbild (Bekanntbild)
- 602: Sequenz Gesichtsbilder
- 702: Sequenz Hirnaktivitätssignale
- 704: zweite Hirnaktivitätssignale
- 706: zweite Hirnaktivitätssignale
- 708: erste Hirnaktivitätssignale
- 710: zweite Hirnaktivitätssignale
- 712: erste Hirnaktivitätssignale
- 800: Authentifizierungssoftware auf Basis eines neuronalen Netzes
- 802: Vorverarbeitung Modul
- 804: Rekurrentes neuronales Netzwerk
- 806: Auswertungsmodul
- 900: Signalplot erfasst von elektrischem Sensor
- 902: erstes Hirnaktivitätssignal (bei Bekanntbildanzeige)
- 904: zweites Hirnaktivitätssignal (bei Fremdbildanzeige)
- 930: Authentifizierungssystem
- 934: Anzeigemodul
- 936: Analysemodul
- 938: Drittsystem
- 950: Signalplot erfasst von optischem Sensor
- 952: erstes Hirnaktivitätssignal (bei Bekanntbildanzeige)
- 954: zweites Hirnaktivitätssignal (bei Fremdbildanzeige)

## Patentansprüche

1. Computerimplementiertes Verfahren zur Authentifizierung einer Person (224) als Vertrauensperson, umfassend:
- Empfang (102) von Daten (223) der Person durch eine Authentifizierungssoftware (204);
- Auswertung (104) der empfangenen Daten durch die Authentifizierungssoftware, um die Person als die Vertrauensperson mittels eines ersten Authentifizierungsverfahrens zu authentifizieren;
- Ermittlung (104) von einem oder mehreren Bekanntbildern (504) und einem oder mehreren Fremdbildern (208, 212, 210) durch die Authentifizierungssoftware, wobei ein Bekanntbild ein Bild ist mit einem Bildmotiv, das der Person bekannt ist, sofern es sich bei der Person um die Vertrauensperson handelt, wobei ein Fremdbild ein Bild ist mit einem Bildmotiv, das der Person nicht bekannt ist oder signifikant weniger bekannt ist als ein Bekanntbild;
- Anzeige (108) mehrerer Bilder auf einer Anzeige (216), wobei die angezeigten Bilder zumindest eines der ermittelten Bekanntbilder und zumindest eines der ermittelten Fremdbilder enthalten;
- während die mehreren Bilder der Person angezeigt werden, Erfassung (110) von ersten (708, 712, 902) und zweiten (704, 706, 710, 904) Hirnaktivitätssignalen, die in der einen Person durch die Betrachtung der Bilder jeweils ausgelöst werden, durch einen kontaktlosen Sensor (218) zur Sensierung von Hirnaktivitäten - im Folgenden als KLHA-Sensor (218) bezeichnet, wobei erste Hirnaktivitätssignale erfasst werden, während der Person ein Bekanntbild (304, 404, 504) angezeigt wird, und wobei zweite Hirnaktivitätssignale erfasst werden, während der Person ein Fremdbild angezeigt wird;
- Durchführung (112) eines zweiten Authentifizierungsverfahrens durch die Authentifizierungssoftware, um die Person als die Vertrauensperson zu authentifizieren, wobei das zweite Authentifizierungsverfahren umfasst eine Analyse der erfassten ersten und zweiten Hirnaktivitätssignale;
- Behandlung der Person durch die Authentifizierungssoftware als authentifiziert nur dann, wenn diese sich sowohl im ersten als auch im zweiten Authentifizierungsverfahren erfolgreich authentifiziert hat.

2. Verfahren nach Anspruch 1, wobei das zweite Authentifizierungsverfahren die Person als erfolgreich authentifiziert behandelt, wenn die ersten Hirnaktivitätssignale sich signifikant unterscheiden von den zweiten Hirnaktivitätssignalen.

3. Verfahren nach einem der vorigen Ansprüche, wobei die empfangenen Daten der Person biometrische Daten der Person sind und wobei das erste Authentifizierungsverfahren ein biometrisches Authentifizierungsverfahren ist, insbesondere ein Verfahren der automatischen Gesichtserkennung und/oder ein Verfahren basierend auf Fingerabdruckdaten oder Irisbildern.

4. Verfahren nach Anspruch 3, wobei das erste Authentifizierungsverfahren ein Verfahren der automatischen Gesichtserkennung ist, wobei die Durchführung des ersten Authentifizierungsverfahrens eine Erfassung eines aktuellen Gesichtsbildes der Person durch einen optischen Sensor umfasst, ferner umfassend:
- Verwendung des erfassten aktuellen Gesichtsbildes der Person als das Bekanntbild oder als eines der Bekanntbilder durch das zweite Authentifizierungsverfahren.

5. Verfahren nach einem der vorigen Ansprüche,
- wobei der KLHA-Sensor integriert und/oder operativ gekoppelt ist an ein Terminal; und/oder
- wobei die Authentifizierungssoftware integriert und/oder operativ gekoppelt ist an das Terminal.

6. Verfahren nach einem der vorigen Ansprüche, ferner umfassend:
- für jeden Authentifizierungsvorgang der Person mit dem zweiten Authentifizierungsverfahren, Erzeugung einer zufälligen Reihung (602) der mehreren für die Person ermittelten Bilder durch die Authentifizierungssoftware und Durchführen des Anzeigens der mehreren Bilder in einer chronologischen Sequenz entsprechend der zufälligen Reihung;
- wobei die Analyse der erfassten ersten und zweiten Hirnaktivitätssignale erfolgt unter Berücksichtigung der chronologischen Sequenz.

7. Verfahren nach Anspruch 6, wobei die zufällige Reihung als Challenge dient und wobei die Analyse der erfassten Hirnaktivitätssignale eine Prüfung umfasst, ob die zeitliche Reihenfolge der ersten und zweiten Hirnaktivitätssignale die in der Challenge codierte Reihenfolge von Bekanntbildern und Fremdbildern widergibt.

8. Verfahren nach einem der vorigen Ansprüche, wobei die Authentifizierungssoftware die Person im zweiten Authentifizierungsverfahren nur dann als erfolgreich authentifiziert behandelt, wenn neben einer signifikanten Unterschiedlichkeit der ersten und zweiten Hirnaktivitätssignale folgende weitere Kriterien zutreffen:
- alle ersten Hirnaktivitätssignale, die je während des Anzeigens eines der Bekanntbilder erfasst werden, sind identisch oder hinreichend ähnlich zueinander;
- alle zweiten Hirnaktivitätssignale, die je während des Anzeigens eines der Fremdbilder erfasst werden, sind identisch oder hinreichend ähnlich zueinander.

9. Verfahren nach einem der vorigen Ansprüche,
- wobei die Ermittlung der ein oder mehreren Bekanntbilder für die Person beinhaltet eine Erfassung von einem oder mehreren Gesichtsbildern der Person durch eine Kamera; und/oder
- wobei die Ermittlung der ein oder mehreren Bekanntbilder für die Person beinhaltet eine Erfassung von einem oder mehreren Bildern eines auf einem Identitätsdokument der Person abgebildeten Gesichtsbilds der Person durch eine Kamera; und/oder
- wobei die Ermittlung der ein oder mehreren Bekanntbilder für die Person beinhaltet eine Identifikation der Bekanntbilder und/oder Fremdbilder in einer Bilddatenbank; und/oder
- wobei die Ermittlung der ein oder mehreren Fremdbilder für die Person beinhaltet eine Identifikation der Bekanntbilder und/oder Fremdbilder in einer Bilddatenbank.

10. Verfahren nach einem der vorigen Ansprüche, wobei der KLHA -Sensor ein elektrischer Sensor ist, und
- wobei der elektrische Sensor dazu ausgebildet ist, die ersten und/oder zweiten Hirnaktivitätssignale durch Messung von Ladungsänderungen und/oder von Ladungsumverteilungen zu erfassen; und/oder
- wobei der elektrische Sensor dazu ausgebildet ist, die Amplitude der ersten und/oder zweiten Hirnaktivitätssignale und/oder die Änderung der Amplitude während eines Zeitintervalls zu erfassen.

11. Verfahren nach einem der vorigen Ansprüche 1-9, wobei der KLHA-Sensor ein optischer Sensor, insbesondere eine IR-Kamera oder IR-Videokamera oder eine Kamera oder Videokamera für Licht im sichtbaren Wellenlängenbereich ist.

12. Verfahren nach Anspruch 11, wobei der KLHA-Sensor dazu ausgebildet ist, Bilder oder Bildsequenzen des Gesichts oder von Teilen des Gesichts der einen Person zu erfassen, die die ersten und/oder zweiten Hirnaktivitätssignale enthalten, wobei die Analyse der ersten und zweiten Hirnaktivitätssignale durch die Authentifizierungssoftware eine automatische Bildanalyse der Bilder oder Bildsequenzen umfasst, wobei die Bildanalyse umfasst:
- Bestimmung eines Pupillendurchmessers der einen Person; und/oder
- Bestimmung eines Pupillendurchmesseränderungsprofils der einen Person.

13. Verfahren nach einem der vorigen Ansprüche,
- wobei die Analyse (108) ausschließlich auf Basis der ersten und zweiten Hirnaktivitätssignalen und auf Basis der Anzeigesequenz der Bekanntbilder und Fremdbilder und optional zusätzlich auf Basis typisierter (nicht personen-individuell erhobener) Referenzdaten durchgeführt wird; und/oder
- wobei das Verfahren außerdem die Löschung der erfassten Hirnaktivitätssignalen nach dem zweiten Authentifizierungsvorgang umfasst.

14. Authentifizierungssystem (200, 930) zur Authentifizierung einer Person (224) als Vertrauensperson, umfassend:
- eine Schnittstelle (219) zum Empfang von Daten (223) der Person;
- eine Authentifizierungssoftware (204, 936);
- eine Anzeige (216);
- einen kontaktlosen Sensor (218) zur Sensierung von Hirnaktivitäten - im Folgenden als KLHA-Sensor bezeichnet;
wobei das Authentifizierungssystem konfiguriert ist zum:
- Empfang (102) der Daten (233) der Person (224) durch die Authentifizierungssoftware über die Schnittstelle (219);
- Auswertung (104) der empfangenen Daten durch die Authentifizierungssoftware, um die Person als die Vertrauensperson mittels eines ersten Authentifizierungsverfahrens zu authentifizieren;
- Ermittlung (106) von einem oder mehreren Bekanntbildern und einem oder mehreren Fremdbildern durch die Authentifizierungssoftware, wobei ein Bekanntbild ein Bild ist mit einem Bildmotiv, das der Person bekannt ist, sofern es sich bei der Person um die Vertrauensperson handelt, wobei ein Fremdbild ein Bild ist mit einem Bildmotiv, das der Person nicht bekannt ist oder signifikant weniger bekannt ist als ein Bekanntbild;
- Anzeige (108) mehrerer Bilder (208, 212, 504, 210, 404, 304) auf der Anzeige, wobei die angezeigten Bilder zumindest eines der ermittelten Bekanntbilder und zumindest eines der ermittelten Fremdbilder enthalten;
- während die mehreren Bilder der Person angezeigt werden, Erfassung (110) von ersten (708, 712, 902) und zweiten (704, 706, 710, 904) Hirnaktivitätssignalen, die in der einen Person durch die Betrachtung der Bilder jeweils ausgelöst werden, durch den KLHA-Sensor (218), wobei erste Hirnaktivitätssignale erfasst werden, während der Person ein Bekanntbild (304, 404, 504) angezeigt wird, und wobei zweite Hirnaktivitätssignale erfasst werden, während der Person ein Fremdbild angezeigt wird;
- Durchführung (112) eines zweiten Authentifizierungsverfahrens durch die Authentifizierungssoftware, wobei das zweite Authentifizierungsverfahren eine Analyse (108) der erfassten ersten und zweiten Hirnaktivitätssignale umfasst;
- Behandlung (114) der Person durch die Authentifizierungssoftware als authentifiziert nur dann, wenn diese sich sowohl im ersten als auch im zweiten Authentifizierungsverfahren erfolgreich authentifiziert hat.

15. Authentifizierungssystem nach Anspruch 14, ferner umfassend ein Terminal, wobei die Anzeigevorrichtung Bestandteil des Terminals ist und wobei das Terminal insbesondere ein Flughafenterminal, ein Grenzkontrollterminal, ein Terminal zur Kontrolle des Zutritts zu einem geschützten geographischen Bereich ist.
